# FASCICULE DE BREVET EUROPEEN

(11) **EP 1 594 438 B1**
(45) Date de publication et mention de la délivrance du brevet: **17.07.2013**
(21) Numéro de dépôt: 04710409.6
(22) Date de dépôt: 12.02.2004
(51) Int. Cl.: A61K 8/36, A61K 8/49, A61K 8/44, A61Q 5/06, A61Q 5/10, A61Q 19/04

(54) **Utilisation d'un inhibiteur de 15-hydroxy prostaglandine déshydrogénase pour favoriser la pigmentation de la peau ou des phanères**
Verwendung eines 15-Hydroxy-Prostaglandin-Dehydrogenase-Inhibitors zur Förderung der Haut- oder Haar-Pigmentierung
Use of an inhibitor of 15-hydroxyprostaglandin dehydrogenase in order to stimulate pigmentation of the skin or hair

(30) Priorité: 12.02.2003 FR 0350023; 24.03.2003 US 456563 P
(43) Date de publication de la demande: 16.11.2005
(73) Titulaire: L'Oréal, 75008 Paris (FR)
(72) Inventeur: MICHELET, Jean-François, F-94000 Créteil (FR); COMMO, Stéphane, F-75015 Paris (FR)
(74) Mandataire: Brohmi, Karim
(86) Numéro de dépôt international: PCT/FR2004/000325
(87) Numéro de publication internationale: WO 2004/073594

(56) Documents cités:
- EP-A- 0 884 045
- EP-A- 1 145 705
- WO-A-01/17479
- WO-A-96/25943
- WO-A-99/51198
- WO-A-03/072033
- US-A- 5 514 374
- US-A- 5 653 983
- US-A- 5 965 157
- US-A- 6 103 765
- US-B1- 6 414 027
- CHO ET AL.: "Thiazolidinediones as a novel class of NAD+-dependent 15-hydroxyprostaglandin dehydrogenase inhibitors" ARCHIVES OF BIOCHEMISTRY AND BIOPHYSICS, vol. 405, 2002, pages 247-251, XP002292688 USA cité dans la demande

## Description

La présente demande décrit à un procédé cosmétique pour favoriser la pigmentation de la peau et/ou des phanères, en particulier des cheveux et/ou des poils et à l'utilisation d'inhibiteurs de 15 hydroxy-prostaglandine déshydrogénase dans des compositions destinées à lutter contre la canitie. Elle décrit notamment l'utilisation de composés tétrazoliques, styryl-pyrazol, phényl-furannes, phényl-thiophène, phényl pyrrazol, pyrazol-carboxamide, 2-thioacétamide ou azoïques comme agent pour favoriser la pigmentation de la peau et/ou des phanères et/ou pour limiter et/ou prévenir la dépigmentation.

La couleur des cheveux et de la peau humaine est fonction de différents facteurs et notamment des saisons de l'année, de la race, du sexe et de l'âge. Elle est principalement déterminée par la concentration de mélanine produite par les mélanocytes. Les mélanocytes sont les cellules spécialisées qui par l'intermédiaire d'organelles particuliers, les mélanosomes, synthétisent la mélanine.

La synthèse de la mélanine ou mélanogénèse est complexe et fait intervenir schématiquement les principales étapes suivantes :
Tyrosine → Dopa → Dopaquinone → Dopachrome → Mélanine

La tyrosinase (monophénol dihydroxyl phénylalanine : oxygen oxydoreductase EC 1.14.18.1) intervient dans cette suite de réactions en catalysant notamment la réaction de transformation de la tyrosine en Dopa (dihydroxyphénylalanine) et la réaction de transformation de la Dopa en Dopaquinone.

La partie supérieure du follicule pileux se présente comme une invagination tubulaire de l'épiderme qui s'enfonce jusqu'aux couches profondes du derme. La partie inférieure, ou bulbe pileux, comporte elle-même une invagination dans laquelle se trouve la papille dermique. On trouve, autour de la papille dermique, dans la partie inférieure du bulbe une zone peuplée de cellules à haut taux de prolifération (cellules de la matrice). Ces cellules sont les précurseurs des cellules kératinisées qui constitueront le cheveu. Les cellules qui résultent de la prolifération de ces précurseurs migrent verticalement dans le bulbe et se kératinisent progressivement dans la partie supérieure du bulbe, cet ensemble de cellules kératinisées formera la tige pilaire. La pigmentation du cheveu et des poils requiert la présence de mélanocytes au niveau du bulbe du follicule pileux. Ces mélanocytes sont dans un état actif, c'est-à-dire qu'ils synthétisent des mélanines. Ces pigments sont transmis aux kératinocytes destinés à former la tige pilaire ce qui conduira à la pousse d'un cheveu ou d'un poil pigmenté. Cette structure est appelée "unité folliculaire de pigmentation".
On sait que dans la plupart des populations la coloration brune de la peau et le maintien d'une coloration constante du cheveu sont des aspirations importantes.
Il est admis que l'apparition de poils et/ou de cheveux gris ou blancs, ou canitie, est associée à une diminution de mélanine dans la tige pilaire. Ce phénomène survient naturellement au cours de la vie d'un individu. Toutefois, l'être humain cherche à avoir un aspect plus jeune et dans un but esthétique, il est souvent tenté de lutter contre ce phénomène, surtout lorsqu'il se produit à un âge relativement précoce.
On a ainsi proposé de nombreuses solutions dans le domaine de la coloration artificielle par apport de colorants exogènes visant à donner aux cheveux une coloration la plus proche possible de ce qu'elle est naturellement. Une autre approche consiste à stimuler la voie naturelle de la pigmentation.
Parmi les solutions proposées, on peut citer des compositions contenant un inhibiteur de phosphodiestérases (WO9517161), des fragments d'ADN (WO9501773), du diacyl glycérol (WO9404122), des prostaglandines (WO9511003), des dérivés de pyrimidine 3-oxyde (EP829260) ou de l'acide oléique (US 5,514,374).

Cependant il existe toujours un besoin de nouvelles solutions efficaces pour favoriser la pigmentation de la peau, des cheveux et/ou des poils et donc prévenir ou diminuer la canitie.

De manière inattendue, la demanderesse a maintenant trouvé qu'il était possible de stimuler la synthèse de mélanine par des mélanocytes en inhibant spécifiquement la dégradation des prostaglandines synthétisés par ces mélanocytes ou celles présentes dans son environnement.

C'est pourquoi la présente demande décrit l'utilisation d'au moins un inhibiteur de la 15 hydroxy-prostaglandine déshydrogénase dans une composition ou pour la préparation d'une composition destinée à favoriser la pigmentation de la peau ou des phanères, en particulier des cheveux et/ou des poils.

La présente invention concerne en particulier l'utilisation non thérapeutique d'au moins un inhibiteur de la 15 hydroxy-prostaglandine déshydrogénase (ou 15-PGDH) dans une composition, l'inhibiteur étant destiné à favoriser la pigmentation de la peau ou des phanères, ledit inhibiteur étant un composé hétérocyclique de formule (I) ou l'un de ses sels: 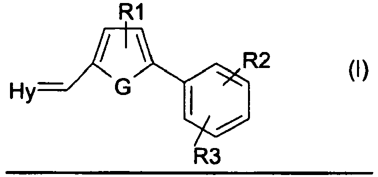 dans laquelle :
- Hy représente un hétérocycle à 4, 5, 6 ou 7 atomes comportant éventuellement au moins une fonction carbonyle et/ou une fonction thiocarbonyle, le dit hétérocycle étant éventuellement substitué par au moins un substituant choisi parmi un halogène, les groupes OR, SR, NRR', COR, CSR, NRCONR'R", C(=NR)R', C(=NR)NR'R", NRC(=NR')NR"R"', OCOR, COSR, SCOR, CSNRR', NRCSR', NRCSNR'R", COOR, CONRR', CF₃, CN. NRCOR', SO₂R', SO₂NRR', NRSO₂R', les radicaux alkyle linéaires ou ramifiés, saturés ou insaturés, en C₁-C₂₀, les cycles de 4 à 7 atomes, saturés ou insaturés, contenant éventuellement au moins un hétéroatome, ces cycles pouvant être séparés ou accolés, les radicaux alkyle et les cycles pouvant, en outre, être substitués, où R, R', R" et R"', identiques ou différents, désignent un hydrogène, un radical alkyle en C₁-C₂₀ linéaire ou ramifié ou un radical aryle, éventuellement substitué :
- G représente O, S, NH :
- R₁, R₂ et R₃ représentent indépendamment l'un de l'autre un hydrogène, un halogène, un groupe OR₀, SR₀, NR₀R₀', COR₀, CSR₀, NR₀CONR₀R₀", C(=NR₀)R_{0'}, C(=NR₀)NR₀'R₀", NR₀C(=NR₀')NR₀"R₀"', OCOR₀, COSR₀, SCOR₀, CSNR₀R₀', NR₀CSR₀, NR₀CSNR₀'R₀", COOR₀, CONR₀R₀', CF₃, NO₂, CN. NR₀COR'₀, SO₂R'₀, SO₂NR₀R'₀, NR₀SO₂R'₀, un radical alkyle linéaire ou ramifié, saturé ou insaturé, en C₁-C₂₀, au moins un cycle de 4 à 7 atomes, saturé ou insaturé, contenant éventuellement au moins un hétéroatome, les cycles pouvant être séparés ou accolés, les radicaux alkyle et les cycles pouvant, en outre, être substitués, où R₀, R₀', R₀" et R₀"', identiques ou différents, désignent un hydrogène, un radical alkyle en C₁-C₂₀ linéaire ou ramifié ou un radical aryle, éventuellement substitué.

On avait précédemment décrit l'implication de certaines prostaglandines dans la pigmentation chez l'homme ou chez l'animal, des poils ou de la peau. (Wand M., 1997, Arch. Ophtalmol, 115; Abdel Malek et al, 1987, cancer Res., 47)

Cependant, les prostaglandines sont des molécules au temps de demi-vie biologique très court et agissant de façon autocrine ou paracrine, ceci traduisant le caractère local et labile du métabolisme des prostaglandines (Narumiya S et al., 1999, Physiol Rev, 79(4), 1193-1226).

De manière surprenante, la demanderesse a maintenant mis en évidence qu'une enzyme spécifiquement impliquée dans la dégradation de ces prostaglandines est exprimée dans les fibroblastes de la papille dermique du cheveu, qui est un compartiment déterminant pour la vie du cheveu. En effet, la demanderesse a maintenant prouvé l'expression de 15 hydroxy prostaglandine déshydrogénase (15-PGDH) à ce niveau.

De plus, il a été montré dans le cadre de l'invention, que cette enzyme est également exprimée dans le mélanocyte du cheveu, ce qui n'avait jamais été mis en évidence jusqu'à présent.

La 15-PGDH est une enzyme clé dans la désactivation des prostaglandines; la 15-PGDH de type 1 répond à la classification EC 1.1.1.141 et est NAD+ dépendante. Cette enzyme catalyse une réaction d'oxydation sur le carbone 15 de l'hydroxyle en cétone. Elle a été isolée de rein de porc; on a notamment observé son inhibition par une hormone thyroïdienne, la tri-iodo thyronine, à des doses très supérieures aux doses physiologiques. La 15-PGDH de type 2 est NADP dépendante.

Cependant on n'avait jamais mis en évidence la présence de 15-PGDH dans la papille dermique et le mélanocyte du cheveu, et il n'avait jamais été proposé d'utiliser un inhibiteur de 15 PGDH pour favoriser la pigmentation de la peau, des poils et/ou des cheveux.

Conformément à l'invention, il est possible de réguler localement le taux de prostaglandines (dont le mode d'action est autocrine et paracrine) notamment celui présent au niveau du mélanocyte, en particulier du chevelu, en agissant sur la dégradation catalysée à la fois par la 15-PGDH du mélanocyte et du fibroblaste de la papille dermique.

La demanderesse a en outre montré de façon surprenante que les mélanocytes de cheveux expriment la prostaglandine H synthase 1 (PGHS-1 ou COX-1 ,E.C : 1.14.99.1).Ceci démontre pour la première fois que les mélanocytes de cheveux possèdent un métabolisme des prostaglandines autonome.

De manière inattendue, on a montré dans le cadre de la présente invention qu'il était possible d'inhiber spécifiquement la 15-PGDH présente au niveau de la papille dermique et/ou du mélanocyte de cheveu. Une telle inhibition permet donc de freiner la désactivation des prostaglandines dans l'environnement du mélanocyte de cheveu. Les prostaglandines peuvent donc continuer par voie autocrine ou paracrine de stimuler les mélanocytes. En effet, l'application de tels inhibiteurs stimule la production par les mélanocytes de mélanine.
Par inhibiteur de la 15-PGDH on entend au sens de l'invention toute substance, composé simple ou complexe, d'origine naturelle ou synthétique, capable d'inhiber ou de diminuer l'activité de l'enzyme 15-PGDH, et/ou capable d'inhiber, diminuer ou ralentir la réaction catalysée par cette enzyme. Les inhibiteurs de 15-PGDH décrits ici sont de préférence des inhibteurs de la 15-PGDH de type 1. Avantageusement, l'inhibiteur est un inhibiteur spécifique de la 15-PGDH de type 1, NAD dépendante.

Par phanères, on entend l'ensemble des annexes tégumentaires et notamment les ongles, les poils et les cheveux. Par poils et cheveux on entend l'ensemble des annexes pileuses et notamment également les cils et les sourcils.

Les compositions selon l'invention pourront être appliquées par toute voie appropriée, notamment orale, parentérale ou topique externe, et leur formulation sera adaptée par l'homme du métier, en particulier pour des compositions cosmétiques ou dermatologiques. Avantageusement, les compositions selon l'invention sont destinées à une administration par voie topique. Elles contiennent un milieu physiologiquement acceptable, en particulier un milieu cosmétologiquement ou pharmaceutiquement, notamment dermatologiquement acceptable.
Dans un mode de réalisation préféré, une composition selon l'invention contient des excipients adaptés à une administration sur le cuir chevelu.

La demande décrit donc notamment l'utilisation d'au moins un inhibiteur de 15-PGDH comme agent pour favoriser et/ou induire et/ou stimuler la pigmentation de la peau et/ou des phanères, et/ou comme agent pour prévenir et/ou limiter la dépigmentation et/ou le blanchiment de la peau et/ou des phanères, notamment comme agent pour prévenir et/ou limiter la canitie; cet agent est plus particulièrement mis en oeuvre chez les mammifères, en particulier chez l'être humain.
La présente demande décrit aussi l'utilisation cosmétique d'au moins un inhibiteur de 15-PGDH dans une composition cosmétique de soin de la peau ou de soin capillaire, pour favoriser et/ou induire et/ou stimuler la pigmentation de la peau et/ou des phanères, et/ou pour prévenir et/ou limiter la dépigmentation et/ou le blanchiment de la peau et/ou des phanères et/ou pour prévenir et/ou limiter la canitie.

La présente invention concerne ainsi l'utilisation non thérapeutique d'une composition cosmétique contenant au moins un inhibiteur de la 15 hydroxy-prostaglandine déshydrogénase, ledit inhibiteur étant un composé hétérocyclique de formule (I) ou l'un de ses sels: 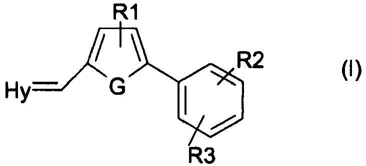 dans laquelle :
- Hy représente un hétérocycle à 4, 5, 6 ou 7 atomes comportant éventuellement au moins une fonction carbonyle et/ou une fonction thiocarbonyle, le dit hétérocycle étant éventuellement substitué par au moins un substituant choisi parmi un halogène, les groupes OR, SR, NRR', COR, CSR, NRCONR'R", C(=NR)R', C(=NR)NR'R", NRC(=NR')NR"R"', OCOR, COSR, SCOR, CSNRR', NRCSR', NRCSNR'R", COOR, CONRR'. CF₃, CN. NRCOR', SO₂R', SO₂NRR', NRSO₂R', les radicaux alkyle linéaires ou ramifiés, saturés ou insaturés, en C₁-C₂₀, les cycles de 4 à 7 atomes, saturés ou insaturés, contenant éventuellement au moins un hétéroatome, ces cycles pouvant être séparés ou accolés, les radicaux alkyle et les cycles pouvant, en outre, être substitués, où R, R', R" et R"', identiques ou différents, désignent un hydrogène, un radical alkyle en C₁-C₂₀ linéaire ou ramifié ou un radical aryle, éventuellement substitué :
- G représente O, S, NH :
- R₁, R₂ et R₃ représentent indépendamment l'un de l'autre un hydrogène, un halogène, un groupe OR₀, SR₀, NR₀R₀', COR₀, CSR₀, NR₀CONR₀'R₀", C(=NR₀)R₀', C(=NR₀)NR₀'R₀", NR₀C(=NR₀')NR₀"R₀", OCOR₀, COSR₀, SCOR₀, CSNR₀R₀', NR₀CSR₀', NR₀CSNR₀'R₀", COOR₀, CONR₀R₀', CF₃, NO₂, CN. NR₀COR'₀, SO₂R'₀, SO₂NR₀R'₀, NR₀SO₂R'₀, un radical alkyle linéaire ou ramifié, saturé ou insaturé, en C₁-C₂₀, au moins un cycle de 4 à 7 atomes, saturé ou insaturé, contenant éventuellement au moins un hétéroatome, les cycles pouvant être séparés ou accolés, les radicaux alkyle et les cycles pouvant, en outre, être substitués, où R₀, R₀', R₀" et R₀"', identiques ou différents, désignent un hydrogène, un radical alkyle en C₁-C₂₀ linéaire ou ramifié ou un radical aryle, éventuellement substitué,
pour le traitement de la canitie.

Le document WO 03/072 033 A2 décrit des thiazolidinediones tombant dans la définition de la formule (I) ci-dessus pour des utilisations en thérapie comme des ligands qui rattachent certain médicaments à leurs récepteurs, par exemple dans le traitement du cancer.

La demande décrit également l'utilisation d'au moins un inhibiteur de 15-PGDH pour la préparation d'une composition destinée à favoriser et/ou induire et/ou stimuler la pigmentation de la peau et/ou des phanères, et/ou destinée à prévenir et/ou limiter la dépigmentation et/ou le blanchiment de la peau et/ou des phanères et/ou destinée à prévenir et/ou limiter la canitie.Les compositions adaptées à la mise en oeuvre de l'invention sont notamment des compositions cosmétiques et/ou dermatologiques, ou des compositions pharmaceutiques.
Par "composition cosmétique", on entend notamment toute substance ou préparation destinée à être mise en contact avec les diverses parties superficielles du corps humain (épiderme, système pileux et capillaire, ongles, lèvres et organes génitaux externes) ou avec les dents et les muqueuses buccales en vue, exclusivement ou principalement, de les nettoyer, de les parfumer, d'en modifier l'aspect et/ou de corriger les odeurs corporelles et/ou de les protéger ou de les maintenir en bon état (directive cosmétique 76/768/CEE modifiée).
Cependant, au sens de la présente invention on entend également par compositions cosmétiques des compositions destinées à être absorbées par toute voie permettant un passage systémique, en particulier par voie orale, en vue de protéger ou maintenir en bon état les parties superficielles du corps, ou d'améliorer l'apparence des individus, en particulier au niveau de la peau et ses annexes.
Le milieu physiologiquement acceptable dans lequel on utilise les inhibiteurs de 15-PGDH selon l'invention peut être anhydre ou aqueux.

La composition peut comprendre un milieu cosmétologiquement acceptable pouvant être constitué d'eau ou d'un mélange d'eau et d'au moins un solvant choisi parmi les solvants organiques hydrophiles, les solvants organiques lipophiles, les solvants organiques amphiphiles et leurs mélanges.
On entend par milieu anhydre, un milieu solvant contenant moins de 1% d'eau. Ce milieu peut être constitué d'un solvant ou d'un mélange de solvants choisi plus particulièrement parmi les alcools inférieurs en C2-C4 comme l'alcool éthylique, les alkylèneglycols comme le propylèneglycol, et les alkyléthers d'alkylèneglycols ou de dialkylèneglycols, dont les radicaux alkyle ou alkylène contiennent de 1 à 4 atomes de carbone. On entend par milieu aqueux, un milieu constitué par de l'eau ou un mélange d'eau et d'un autre solvant physiologiquement acceptable, choisi notamment parmi les solvants organiques cités ci-dessus. Dans ce dernier cas, ces autres solvants, lorsqu'ils sont présents, représentent environ 5 à 95% en poids de la composition.
Il est possible que le milieu physiologiquement acceptable puisse contenir d'autres adjuvants habituellement utilisés dans le domaine cosmétique ou pharmaceutique, tels que des agents tensioactifs, des agents épaississants ou gélifiants, des agents cosmétiques, des agents conservateurs, des agents alcalinisants ou acidifiants bien connus dans l'état de la technique, et en quantités suffisantes pour obtenir la forme de présentation désirée, notamment de lotion plus ou moins épaissie, de gel, d'émulsion, ou de crème. L'utilisation peut éventuellement se faire sous une forme pressurisée en aérosol ou vaporisée à partir d'un flacon pompe.
Pour une application topique, la composition utilisable selon l'invention peut être notamment sous la forme de solutions ou de suspensions aqueuse, alcoolique, hydroalcoolique ou huileuse, ou de dispersion du type lotion ou sérum, d'émulsions de consistance liquide ou semi-liquide ou pâteuses, obtenues par dispersion d'une phase grasse dans une phase aqueuse (H/E) ou inversement (E/H) ou multiples, , d'une poudre libre ou compactée à utiliser telle quelle ou à incorporer dans un milieu physiologiquement acceptable, ou encore de microcapsules ou microparticules, ou de dispersions vésiculaires de type ionique et/ou non ionique. Elle peut ainsi se présenter sous forme d'onguent, de teinture, de laits, de crème, de pommade, de poudre, de timbre, de tampon imbibé, de solution, d'émulsion ou de dispersion vésiculaire, de lotion, de gels aqueux ou anhydres, de spray, de suspension, de shampooing, d'aérosol ou de mousse. Elle peut être anhydre ou aqueuse. Elle peut également consister en des préparations solides constituant des savons ou des pains de nettoyage.
Ces compositions sont préparées selon les méthodes usuelles.

La composition utilisable selon l'invention peut en particulier consister en une composition pour soins capillaires, et notamment un shampooing, une lotion de mise en plis, une lotion traitante, une crème ou un gel coiffant, des lotions restructurantes pour les cheveux, un masque, etc.
La composition cosmétique selon l'invention sera préférentiellement une crème, une lotion capillaire, un shampoing ou un après-shampoing. Elle peut être utilisée notamment dans les traitements mettant en oeuvre une application qui est suivie ou non suivie d'un rinçage, ou encore sous forme de shampooing.
On peut également envisager une composition sous forme de mousse ou encore sous forme de spray ou d'aérosol comprenant alors un agent propulseur sous pression.

Elle peut ainsi se présenter sous forme d'une lotion, sérum, lait, crème H/E ou E/H, gel, d'onguent, pommade, poudre, baume, patch, tampon imbibé, pain, mousse.

En particulier la composition à application sur le cuir chevelu ou les cheveux peut se présenter sous forme d'une lotion de soin capillaire, par exemple d'application journalière ou bi-hebdomadaire, d'un shampooing ou d'un après-shampooing capillaire, en particulier d'application bi-hebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.

Par ailleurs, pour une application sur les cils ou les poils, la composition à laquelle s'applique l'invention peut se présenter sous forme d'un mascara, pigmenté ou non, à appliquer à la brosse sur les cils ou encore sur les poils de barbe ou de moustache. Pour une composition à usage par injection, la composition peut se présenter sous forme de lotion aqueuse ou de suspension huileuse. Pour un usage par voie orale, la composition peut se présenter sous forme de capsules, de granulés, de sirops buvables ou de comprimés.

Selon un mode de réalisation particulier, la composition selon l'invention se présente sous forme de crème ou lotion capillaire, de shampooing ou d'après-shampooing capillaire, de mascara capillaire ou des cils.

Les quantités des différents constituants du milieu physiologique de la composition selon l'invention sont ceux généralement utilisées dans les domaines considérés Elle peut également se présenter sous forme de teinture ou de mascara à appliquer au pinceau ou au peigne, en particulier sur les cils, les sourcils ou les cheveux.
Elle peut également se présenter sous forme de vernis destiné à être appliqué à la surface de l'ongle.

Les quantités des différents constituants des compositions utilisables selon l'invention sont celles classiquement utilisées dans les domaines considérés.

Lorsque la composition utilisable selon l'invention est une émulsion, la proportion de la phase grasse peut aller de 5% à 80% en poids, et de préférence de 5% à 50% en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3% à 30% en poids, et de préférence de 0,5 à 20% , voire de 1 à 8% en poids par rapport au poids total de la composition. L'émulsion peut, en outre, contenir des vésicules ou sphérules lipidiques.
Lorsque la composition utilisable selon l'invention est une solution ou un gel huileux, la phase grasse peut représenter plus de 90% du poids total de la composition. Avantageusement, la composition est une solution ou suspension aqueuse, alcoolique ou hydro-alcoolique et mieux une solution ou suspension eau/éthanol. La fraction alcoolique peut représenter de 5% à 99,9% et mieux de 8% à 80%.

Pour une application mascara en particulier des cils, la composition de l'invention est une dispersion de cire-dans-eau ou de cire-dans-huile, une huile gélifiée, un gel aqueux, ce mascara étant, en outre, pigmenté ou non.

Avantageusement, la composition comprendra des microsphères, des nanosphères, des liposomes, des oléosomes ou des nanocapsules, dans lesquels au moins un agent inhibiteur de la 15-PGDH sera encapsulé. Des exemples de telles formulations sont décrits notamment dans les brevets EP199636, EP 375520, EP447318, EP557489, WO 97/12602, EP1151741 ou US 5 914126.

A titre d'exemple, les microsphères pourront être préparées selon la méthode décrite dans la demande de brevet EP 0 375 520.

Les nanosphères pourront se présenter sous forme de suspension aqueuse et être préparées selon les méthodes décrites dans les demandes de brevet FR 0015686 et FR 0101438.
Les oléosomes consistent en une émulsion huile dans eau formée par des globules huileux pourvus d'un enrobage cristal liquide lamellaire dispersé dans une phase aqueuse (voir les demandes de brevet EP 0 641 557 et EP 0 705 593).

L'inhibiteur de 15PGDH pourra aussi être encapsulé dans des nanocapsules consistant en un enrobage lamellaire obtenu à partir d'un tensio-actif siliconé tel que décrit dans la demande de brevet EP 0 780 115; les nanocapsules pourront également être préparées à base de polyesters sulfonique hydrodispersibles selon par exemple la technique décrite dans la demande de brevet FR 0113337.
De façon connue, la composition selon l'invention peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01% à 20%, notamment inférieur ou égal à 10% du poids total de la composition. Ces adjuvants, selon leur nature, peuvent être introduits dans la phase grasse, dans la phase aqueuse et/ou dans les sphérules lipidiques.
La phase grasse peut contenir des composés gras ou huileux, liquides à température ambiante (25°C) et pression atmosphérique (760 mm de Hg), généralement appelés huiles. Ces huiles peuvent être compatibles ou non entre elles et former une phase grasse liquide macroscopiquement homogène ou un système bi- ou triphasique.

La phase grasse peut, en plus des huiles, contenir des cires, des gommes, des polymères lipophiles, des produits "pâteux" ou visqueux contenant des parties solides et des parties liquides.

La phase aqueuse contient de l'eau et éventuellement un ingrédient miscible en toute proportion à l'eau comme les alcools inférieurs en C₁ à C₈ tel que l'éthanol, l'isopropanol, les polyols comme le propylène glycol, le glycérol, le sorbitol ou encore l'acétone ou l'éther.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).
Comme émulsionnants utilisables dans l'invention, on peut citer par exemple le stéarate de glycérol, ou laurate de glycérol, les stéarates ou oléates de sorbitol, les alkyl diméthiconecopolyol (avec alkyle ≥ 8) et leurs mélanges pour une émulsion E/H. On peut aussi utiliser le monostéarate ou monolaurate de polyéthylène glycol, le stéarate ou oléate de sorbitol polyoxyéthyléné, les diméthiconecopolyols et leurs mélanges, notamment le polysorbate 60 et le mélange de PEG-6/PEG-32/Glycol Stéarate vendu sous la dénomination de Tefose^{®} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les Bentones®, les sels métalliques d'acides gras comme les stéarates d'aluminium et la silice hydrophobe, l'éthylcellulose. Lorsque la composition est épaissie ou gélifiée à l'aide d'un agent épaississant, ce dernier est généralement présent dans des concentrations comprises entre environ 0,1 et 6% par rapport au poids total de la composition.

De préférence, le ou les agents inhibiteurs sont présents à une concentration supérieure ou égale à 10⁻³ %, notamment de 0,001% à 5% p/v par rapport à la composition, de manière encore préférée de 0,01 à 2%. Toutefois ces quantités seront adaptées par l'homme du métier selon le composé utilisé, pour obtenir une activité d'inhibition enzymatique équivalente à une inhibition pratiquement totale de l'enzyme 15-PGDH dans les conditions d'application de la composition, la concentration utilisée étant généralement supérieure ou égale à celle pour laquelle une inhibition de 100% de la 15-PGDH est observée in vitro. La concentration en agent inhibiteur de 15-PGDH sera notamment 50 à 500 fois supérieure à la concentration pour laquelle une inhibition de 100% de la 15-PGDH a été observée in vitro, par exemple on utilisera de concentrations d'environ 100 fois celle correspondant à une inhibition totale in vitro.

La quantité efficace d'inhibiteur de 15-PGDH correspond à la quantité nécessaire pour obtenir le résultat désiré (à savoir favoriser la pigmentation de la peau et/ou des phanères, et/ou limiter leur blanchiment) et sera évaluée aisément par l'homme du métier en fonction de la nature de l'inhibiteur utilisé, de la personne à laquelle on l'applique et du temps de cette application.

Des inhibiteurs de la 15-PGDH adaptés pourront être déterminés par l'homme du métier.

De préférence, les inhibiteurs de 15PGDH NAD dépendante selon l'invention ne sont pas inhibiteurs des prostaglandines H synthase ou cycloxygénases, aussi désignées par l'abréviation COX, qui sont des enzymes impliquées dans la synthèse des prostaglandines.
En particulier, si le ou les inhibiteurs de 15 PGDH sont introduits dans les compositions selon l'invention sous forme d'extraits végétaux, ces extraits sont essentiellement exempts de flavonoïdes et/ou n'ont pas d'activité inhibitrice des COX (PGHS) aux doses utilisées.
Selon un mode de réalisation particulièrement avantageux de l'invention, la composition comprendra au moins un inhibiteur spécifique de la 15 PGDH; par inhibiteur spécifique on entend un actif qui soit peu ou pas inhibiteur de la synthèse des prostaglandines, en particulier de la synthèse de PGF2α ou de PGE2. De préférence, l'inhibiteur de la 15PGDH ne sera peu ou pas inhibiteur de la prostaglandine synthase (PGF synthase).

En effet, dans le cadre de ses recherches, la demanderesse a maintenant trouvé, de façon inattendue, que la PGF synthase est également exprimée dans la papille dermique. Le maintien d'une quantité efficace de prostaglandines au site d'action résulte donc d'un équilibre biologique complexe entre la synthèse et la dégradation de ces molécules. L'apport exogène de composés inhibant le catabolisme sera donc moins efficace si cette activité est combinée à une inhibition de la synthèse.

En complément ou remplacement de cet apport exogène, la demanderesse a maintenant mis en évidence qu'il est possible de favoriser le maintien d'un pool endogène de prostaglandines, et donc le maintien voire l'augmentation de la pigmentation de la peau et des phanères, en particulier des cheveux.

En application de la présente invention, il est maintenant possible de cibler des composés particulièrement actifs, pour lesquels l'activité inhibitrice de la 15-PGDH est significativement supérieure à l'activité d'inhibition de la PGF synthase. Le rapport entre les activités inhibitrices respectivement de la 15PGDH et de la PGF synthase pour la dose administrée, déterminées notamment par les concentrations inhibitrices de 50% de l'activité enzymatique, sera au moins supérieur à 1, de préférence d'au moins 3:1, avantageusement supérieur ou égal à 5:1. Des agents particulièrement adaptés à la mise en oeuvre de l'invention présentent un ratio entre les activités inhibitrices de la 15PGDH et de la PGF synthase supérieur ou égal à 10:1, en particulier supérieur ou égal à 15, de préférence supérieur ou égal à 25 :1.
à cet égard, des composés particulièrement adaptés décrits dans la demande sont les composés répondant aux formules suivantes :

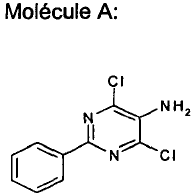

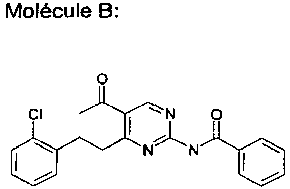

Le choix d'autres agents actifs sur l'inhibition de la 15-PGDH selon la présente invention pourra être effectué par l'homme du métier en mettant en oeuvre un test simple à partir de candidats potentiels. Ce test consistera à comparer la cinétique d'une réaction catalysée par cette enzyme, dans un milieu réactionnel comprenant un substrat de l'enzyme et d'éventuels co-substrats, en présence ou non du composé dont on veut évaluer le rôle d'inhibiteur de la 15-PGDH; les conditions de la réaction (pH, température, temps de réaction, etc,) sont celles adaptées à la réaction et sont les mêmes pour la mesure en présence ou en absence du composé ou de la substance à tester.
Pour cela, par exemple, on met en présence l'enzyme 15-PGDH à une concentration finale de 7. 10⁻³ mg/ml, son co-substrat (β-NAD) et un substrat (PGE2) aux concentrations correspondant aux conditions classiquement mises en oeuvre pour ce test tel que décrit par exemple par Cho et Taï, (Inhibition of NAD-dependent 15-hydroxyprostaglandin dehydrogenase (15-PGDH) by cyclooxygenase inhibitors and chemopreventive agents. Prostaglandins, Leukotrienes and Essential Fatty Acids, 2002 , 67(6) : 461-465) , par exemple 1,5 mM de β-NAD et 50µM de prostaglandine E2. On mesure la vitesse de réaction à 37°C pendant 1 minute. On réalise la même réaction, mais en ajoutant dans le milieu en début de réaction le composé à tester. On compare la vitesse maximale de réaction enzymatique par unité de temps (Vmax) mesurée en présence du composé à celle du témoin sans composé, et l'on détermine le pourcentage d'inhibition [100-(Vmax essai X 100) / Vmax témoin].
Les composés notés inhibiteurs de la 15PGDH sont alors testés pour leur capacité à inhiber la PGF synthase. Pour cela, par exemple, on met en présence l'enzyme PGFS à une concentration finale de 25. 10⁻³ mg/ml, son co-substrat (le β-NADPH2) et un substrat (par exemple la phénanthrène quinone) aux concentrations classiquement mises en oeuvre pour ce test tel que décrit par exemple par Suzuki et al. ( cDNA cloning, expression and mutagenesis study of liver-type prostaglandin F synthase. J Biol Chem, 1999, 274(1) : 241-248), c'est à dire 100 µM de β-NADPH2 et 20 µM de phénanthrène quinone. On mesure la vitesse maximale de réaction par unité de temps à 37°C. On réalise la même réaction, mais en ajoutant dans le milieu en début de réaction le composé à tester. On compare la vitesse maximale de réaction enzymatique avec le composé à celle du témoin sans composé, et l'on détermine le pourcentage d'inhibition [100-(Vmax essai X 100) / Vmax témoin].

On compare ensuite le pourcentage d'inhibition de la réaction catalysée par la 15-PGDH et celui de la réaction catalysée par la PGFS. De façon plus précise, on établit le ratio des IC50 d'un composé vis-à-vis de la PGFS et de la 15-PGDH (IC50 PGFS /IC50 15-PGDH). L'IC50 est la concentration du composé pour laquelle la Vmax est réduite de 50%.

L'activité de composés montrant une inhibition sélective de la 15-PGDH au sens de la présente invention pourra également être mise en évidence en mesurant le taux de prostaglandines dans un modèle cellulaire mimant l'environnement enzymatique de la papille du cheveu. Cela permet d'évaluer l'efficacité d'un inhibiteur sélectif de 15-PGDH sur la protection des prostaglandines dans un système biologique complexe produisant les différents types d'enzymes impliquées dans le métabolisme de ces molécules. Par exemple, on utilise une culture de promonocytes, qui sont des précurseurs des macrophages dans certaines conditions, modèle très répandu pour étudier le métabolisme des prostaglandines.
En effet, les esters de phorbol (PMA 10 nM) provoquent en 24h00 la différenciation de la lignée de promonocytes U937 en macrophages; cette différenciation s'accompagne par l'induction de la 15-PGDH. (Tong et Tai, Biochim Biophys Acta, 2000; 1497 : 61-68).
Par ailleurs, une stimulation de ces macrophages par du LPS (lipopolysaccharide extrait de paroi bactérienne) induit (à 100 ng/ml) en 6h00 la PGHS-2 (ou COX-2), enzyme responsable (au même titre que COX-1) de la synthèse de PGH2, précurseur (entre autres) via la PGFS ,de PGF2α. (Arias-Negrete et al., 1995. Biochem Biophys Res Commun, 208(2), 582-589).
Dans une 1^{ère} étape, on cultive des précurseurs de macrophages dans un milieu adapté, en présence d'un composé stimulant leur différenciation et l'induction de la 15-PGDH, puis on stimule la production de prostaglandines par ces cellules, par exemple par des LPS sous forme d'extrait ou purifiés; cette 2^{nde} étape est effectuée en présence ou non du composé à tester. On compare les concentrations de prostaglandines, en particulier de PGF2α, obtenues en présence du composé inhibiteur de 15-PGDH à tester, à celle du témoin ne contenant que l'inducteur de 15-PGDH, cette mesure pouvant s'effectuer par toute méthode connue de l'homme du métier, notamment par dosage immunoenzymatique. Au moment du dosage la quantité de PGF2α mesurée est donc la résultante des deux activités enzymatiques pour lesquelles les composés testés sont plus ou moins actifs : celle de la PGFS qui conduit à la synthèse de PGF2α et celle de la 15-PGDH qui conduit à la dégradation. En présence d'un inhibiteur non sélectif de la 15-PGDH (inhibiteur aussi de PGFS) on assistera à une baisse de PGF2α (correspondant à une baisse de la synthèse par action du produit sur la PGFS). En présence d'un inhibiteur sélectif de la 15-PGDH on assistera à une augmentation de PGF2α qui correspond à la baisse de la dégradation. Les composés pour lesquels le taux de PGF2α observé sont supérieurs d'au moins 5%, de préférence d'au moins 10% à celle du témoin (inducteur de la synthèse de prostaglandine seul) ont donc un rôle protecteur de la prostaglandine F2α. Avantageusement, pour les composés adaptés à la mise en oeuvre de l'invention la concentration de prostaglandines avec le composé à tester est supérieure ou égale d'au moins 20%, voire même de 30% à celle du témoin.

De tels composés sont notamment ceux décrits dans la demande WO 03/090699:

### COMPOSES ACETYL-TETRAZOLIQUES:

De tels composés sont notamment certains acétyl-tétrazoles, salifiés ou non, inhibiteurs de la 15-hydroxy prostaglandine déshydrogénase.

Selon l'un des aspects décrits dans la demande, l'inhibiteur de 15-PGDH comprend au moins une quantité efficace d'un composé tétrazolique de formule (I) ou (II) ou d'un de ses sels : 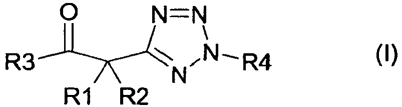 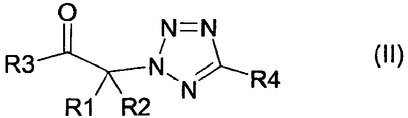 dans lesquelles :
- R₁ et R₂ sont indépendamment choisis parmi l'hydrogène, les halogènes, OR₅, SR₅, NR₅R'₅, COOR₅, COR₅, CONR₅R'₅, CF₃, CN, NR₅COR'₅, SO₂R₅, SO₂NR₅R'₅, NR₅SO₂R'₅, CSR₅, OCOR₅, COSR₅, SCOR₅, CSNR₅R'₅, NR₅CONR'₅R"₅, NR₅C(=NR'₅)NR"₅R"'₅, NR₅CSR'₅, NR₅CSNR'₅R"₅, les radicaux alkyle en C₁-C₂₀, linéaires ou ramifiés, et les cycles de 4 à 7 atomes, contenant éventuellement au moins un hétéroatome, ces cycles pouvant être séparés ou accolés, les radicaux alkyle et les cycles étant, en outre, saturés ou non, et éventuellement substitués par au moins un substituant A₁, où R₅, R'₅, R"₅ et R"₅ désignent indépendamment l'hydrogène, un radical alkyle en C₁-C₂₀, linéaire ou ramifié, ou un cycle hydrocarboné de 4 à 7 atomes, le cycle hydrocarboné ou le radical alkyle étant saturé ou non et éventuellement substitué par au moins un substituant A₂ ;
- R₃ est choisi parmi l'hydrogène, OR₆, SR₆, NR₆R'₆, CF₃, NR₆COR'₆, NR₆SO₂R'₆, NR₆CONR'₆R"₆, NR₆CSR'₆, NR₆CSNR'₆R"₆, les radicaux alkyle en C₁-C₂₀, linéaires ou ramifiés, et les cycles hydrocarbonés de 4 à 7 atomes, séparés ou accolés, les radicaux alkyle et les cycles hydrocarbonés étant, en outre, saturés ou non et éventuellement substitués par au moins un substituant A₃, avec R₆, R'₆ et R"₆ désignant indépendamment l'hydrogène, un radical alkyle en C₁-C₂₀, linéaire ou ramifié, ou un cycle hydrocarboné de 4 à 7 atomes, le radical alkyle ou le cycle hydrocarboné étant saturé ou non et éventuellement substitué par au moins un substituant A₄ ;
- R₄ est choisi parmi l'hydrogène, COOR₇, CONR₇R'₇, SO₂R₇, SO₂NR₇R'₇, COR₇, CSR₇, COSR₇, CSNR₇R'₇, les radicaux alkyle en C₁-C₂₀, linéaires ou ramifiés, et les cycles hydrocarbonés de 4 à 7 atomes, séparés ou accolés, les radicaux alkyle et les cycles hydrocarbonés étant, en outre, saturés ou non et éventuellement substitués par au moins un substituant A₅ ; R₄ peut, en outre, représenter, dans le cas de la formule (II), un halogène, OR₇, SR₇, NR₇R'₇, CF₃, CN, NR₇COR'₇, NR₇SO₂R'₇, OCOR₇, SCOR₇, NR₇CONR'₇R"₇, NR₇C(=NR'₇)NR"₇R"'₇, NR₇CSR'₇ ou NR₇CSNR'₇R"₇, avec R₇, R'₇, R"₇ et R"'₇ désignant indépendamment l'hydrogène, un radical alkyle en C₁-C₂₀, linéaire ou ramifié, ou un cycle hydrocarboné de 4 à 7 atomes, le radical alkyle ou le cycle hydrocarboné étant saturé ou non et éventuellement substitué par au moins un substituant A₆ ;
- A₁ et A₂ sont indépendamment choisis parmi les halogènes, les hétérocycle comportant de 4 à 7 atomes et au moins un hétéroatome, OR₈, SR₈, NR₈R'₈, COOR₈, CONR₈R'₈, CF₃, CN, NR₈COR'₈, SO₂R₈, SO₂NR₈R'₈, NR₈SO₂R'₈, COR₈, CSR₈, OCOR₈, COSR₈, SCOR₈, CSNR₈R'₈, NR₈CONR'₈R"₈, NR₈C(=NR'₈)NR"₈R"'₈, NR₈CSR'₈, NR₈CSNR'₈R"₈ ;
- A₃ et A₄ sont indépendamment choisis parmi les halogènes, R₉, OR₉, SR₉, NR₉R'₉, COOR₉, CONR₉R'₉, CF₃, CN, NR₉COR'₉, SO₂R₉, SO₂NR₉R'₉, NR₉SO₂R'₉, CSR₉, OCOR₉, COSR₉, SCOR₉, CSNR₉R'₉, NR₉CONR'₉R"₉, NR⁹C(=NR'₉)NR"₉R"'₉, NR₉CSR'₉, NR₉CSNR'₉R"₉ ;
- A₅ et A₆ sont indépendamment choisis parmi les halogènes, R₁₀, OR₁₀, SR₁₀, NR₁₀R'₁₀, CF₃, CN, NR₁₀COR'₁₀, SO₂R₁₀, SO₂NR₁₀R'₁₀, NR₁₀SO₂R'₁₀, CSR₁₀, OCOR₁₀, SCOR₁₀, CSNR₁₀R'₁₀, NR₁₀CONR'₁₀R"₁₀, NR₁₀C(=NR'₁₀)NR"₁₀R'"₁₀, NR₁₀CSR'₁₀, NR₁₀CSNR'₁₀R"₁₀ ;
- R₈, R'₈, R"₈, R'"₈, R₉, R'₉, R"₉, R"'₉, R₁₀, R'₁₀, R"₁₀ et R"'₁₀ désignant indépendamment l'hydrogène, un radical alkyle en C₁-C₂₀, linéaire ou ramifié, saturé ou non, un cycle hydrocarboné de 4 à 7 atomes, saturé ou non, ou un radical benzyle.

La demande décrit encore à l'utilisation d'au moins un composé tétrazolique de formule (I) ou (II) ou forme tautomère ou de l'un de ses sels, tel que défini précédemment, comme agent pour induire et/ou stimuler la pigmentation des cheveux des êtres humains et/ou de la peau.

Avantageusement, les composés de formule (I) ou (II), sous forme salifiée ou non, présentent une activité inhibitrice de la 15-PGDH qui est supérieure à l'activité d'inhibition de la PGF synthase.
Dans la suite du texte, et sauf mention exprès, l'emploi du terme composé de formule (I) ou (II) doit être compris comme signifiant aussi bien le composé de formule (I) ou de formule (II), sous forme acide ou basique que sous forme de sel.

"Au moins un" selon l'invention signifie un ou plusieurs (2, 3 ou plus). En particulier, la composition peut contenir un ou plusieurs composés de formule (I), un ou plusieurs composés de formule (II) ou un mélange de composés de formule (I) et de formule (II). Ce ou ces composés peuvent être des isomères cis ou trans ou un mélange d'isomères cis/trans. Ils peuvent aussi être sous forme tautomère. Ils peuvent être également des énantiomères et/ou des diastéréoisomères ou un mélange de ces isomères, en particulier un mélange racémique.

Par cycle "hydrocarboné", on entend ici un cycle ne contenant que des liaisons carbone-carbone pour former le cycle.
Tels que décrits ici, les cycles employés pour R₁ à R₄ dans les formules (I) et (II) comportent indépendamment de 4 à 7 atomes et mieux de 5 à 6 atomes. Ils peuvent être saturés ou insaturés. Ils peuvent, de plus, être seuls ou accolés à un autre cycle de même structure chimique ou non. En outre, R₁ et R₂ comportent éventuellement un ou plusieurs hétéroatomes tels que S, N, O ou leurs associations.

Tels que décrits ici, les cycles employés pour R₅, R'₅, R"₅, R"'₅, R₆, R'₆, R"₆, R"'₆, R₇, R'₇, R"₇, R"'₇, R₈, R'₈, R"₈, R"'₈, R₉, R'₉, R"₉, R"'₉, R₁₀, R'₁₀, R"₁₀ et R"'₁₀ dans les formules (I) et (II) comportent indépendamment de 4 à 7 atomes de carbone et mieux de 5 à 6 atomes de carbone. Ils peuvent être saturés ou mieux insaturés.

Par ailleurs, les hétérocycles employés pour A₁ et A₂ dans les formules (I) et (II) comportent un ou plusieurs hétéroatomes tels que S, N, O ou leurs associations. Ils comportent, en outre, indépendamment de 4 à 7 atomes et mieux de 5 à 6 atomes. De plus, ils peuvent être saturés ou insaturés.

Comme cycles hydrocarbonés saturés utilisables dans les formules (I) ou (II) on peut citer le radical cyclopentyle ou cyclohexyle. Comme hétérocycle, on peut citer les cycles pyridine, pipéridine, morpholine, pyrrole, furanne, thiazole. Comme cycles hydrocarbonés insaturés, on peut citer le radical phényle, naphtyle. En outre, ces cycles peuvent être substitués par un ou plusieurs substituants ayant la définition indiquée ci-dessus pour A₁, A₂, A₃, A₄, A₅ et A₆, selon qu'il s'agit de R₁, R₂, R₃, R₄, R₅, R'₅, R"₅, R"'₅, R₆, R'₆, R"₆, R₇, R'₇, R"₇ ou de R'"₇.

Par radical alkyle linéaire ou ramifié en C1-C20 on entend ici les radicaux acycliques provenant de l'enlèvement d'un atome d'hydrogène dans la molécule d'un hydrocarbure, linéaire ou ramifié ayant de 1 à 20 atomes de carbone et en particulier les radicaux méthyle, éthyle, propyle, isopropyle, butyle, isobutyle, tertiobutyle, pentyle, hexyle, heptyle, octyle ainsi que leurs isomères de position correspondant. Comme exemple de radical alkyle (saturé) utilisable décrit ici, on peut citer les radicaux méthyle, éthyle, n-butyle, iso-propyle, n-hexyle.

Comme atome d'halogène, on peut utiliser les atomes de chlore, de fluor, d'iode ou de brome, et plus spécialement de chlore.

Tels que décrits ici, les composés de formule (I) ou (II) sont sous forme isolée, c'est-à-dire non polymérique. En outre, les substituants A₁, A₂, A₃, A₄, A₅ et A₆ peuvent être situés en toute position du cycle le portant et en particulier en position adjacente au groupement portant le cycle tétrazole.

Selon un mode de réalisation, l'un au moins des R₁ et R₂ représentent un atome d'hydrogène, un atome d'halogène et notamment un atome de fluor ou de chlore. En particulier, R₁ et R₂ représentent l'hydrogène.

Avantageusement, R₃ représente NR₆R'₆ ou un radical aryle, et dans un mode de réalisation particulier un radical naphtyle ou phényle, éventuellement substitué par le substituant A₃. En particulier, A₃ représente OR₉.

Selon un mode de réalisation, R₆ représente l'hydrogène et R'₆ un radical aryle, en particulier phényle, éventuellement substitué par le groupe OR₉.

En particulier, R₉ représente un radical alkyle en C₁-C₂₀ et mieux en C₁-C₁₀, linéaire ou ramifié, saturé et par exemple le radical méthyle.

Selon un mode de réalisation de l'invention, R₄ représente un radical aryle et notamment un radical naphtyle ou phényle.

Par sels de composé de formule (I) ou (II), on entend ici, les sels organiques ou inorganiques d'un composé de formule (I) ou (II).

Comme sels inorganiques utilisables décrits ici on peut citer les sels de sodium ou de potassium ainsi que les sels de zinc (Zn²⁺), de calcium (Ca²⁺), de cuivre (Cu²⁺), de fer (Fe²⁺), de strontium (Sr²⁺), de magnésium (Mg²⁺) et de manganèse (Mn²⁺) ; les hydroxydes et les carbonates.

Les sels organiques utilisables décrits ici sont par exemple les sels de tri-éthanolamine, mono-éthanolamine, di-éthanolamine, hexadécylamine, N,N,N',N'-tétrakis-(hydroxy-propyl-2) éthylène diamine, tris-hydroxyméthyl aminométhane.

Des composés adaptés décrits dans la présente demande sont en particulier les composés inhibiteurs de 15-PGDH de formule (I) ou (II) telles que définies ci-dessus, dans lesquelles:
- R₁ et R₂ sont indépendamment choisis parmi l'hydrogène, les halogènes, OR₅, SR₅, NR₅R'₅, COOR₅, CF₃, CN, les radicaux alkyle en C₁-C₂₀, linéaires ou ramifiés, et les cycles de 4 à 7 atomes, contenant éventuellement au moins un hétéroatome, ces cycles pouvant être séparés ou accolés, les radicaux alkyle et les cycles étant, en outre, saturés ou non, où R₅ et R'₅ désignent indépendamment l'hydrogène, un radical alkyle en C₁-C₂₀, linéaire ou ramifié, ou un cycle hydrocarboné de 4 à 7 atomes, le cycle hydrocarboné ou le radical alkyle étant saturé ou non ;
- R₃ est choisi parmi l'hydrogène, OR₆, SR₆, NR₆R'₆, CF₃, les radicaux alkyle en C₁-C₂₀, linéaires ou ramifiés, et les cycles hydrocarbonés de 4 à 7 atomes, séparés ou accolés, les radicaux alkyle et les cycles hydrocarbonés étant, en outre, saturés ou non et éventuellement substitués par au moins un substituant A₃, avec R₆ et R'₆ désignant indépendamment l'hydrogène, un radical alkyle en C₁-C₂₀, linéaire ou ramifié, ou un cycle hydrocarboné de 4 à 7 atomes, le radical alkyle ou le cycle hydrocarboné étant saturé ou non et éventuellement substitué par au moins un substituant A₄ ;
- R₄ est choisi parmi l'hydrogène, COOR₇, CSR₇, les radicaux alkyle en C₁-C₂₀, linéaires ou ramifiés, et les cycles hydrocarbonés de 4 à 7 atomes, séparés ou accolés, les radicaux alkyle et les cycles hydrocarbonés étant, en outre, saturés ou non ; R₄ peut, en outre, représenter, dans le cas de la formule (II), un halogène, OR₇, SR₇, NR₇R'₇, CF₃, CN , avec R₇ et R'₇, désignant indépendamment l'hydrogène, un radical alkyle en C₁-C₂₀, linéaire ou ramifié, ou un cycle hydrocarboné de 4 à 7 atomes, le radical alkyle ou le cycle hydrocarboné étant saturé ou non, éventuellement substitué par au moins un substituant choisi parmi OR₁₀, SR₁₀, NR₁₀R'₁₀, CF₃, avec R10 et R'10 désignant un radical alkyle en C₁-C₂₀ linéaire ou ramifié, saturé ou non;
- A₃ et A₄ sont indépendamment choisis parmi les halogènes, R₉, OR₉, SR₉, NR₉R'₉, COOR₉, CF₃, avec R₉ et R'₉, désignant indépendamment l'hydrogène, un radical alkyle en C₁-C₂₀, linéaire ou ramifié, saturé ou non, un cycle hydrocarboné de 4 à 7 atomes, saturé ou non, ou un radical benzyle.

Selon un autre mode de réalisation décrit ici, les composés inhibiteurs de la 15-PGDH sont tels que dans la formule (I) ou (II) définie précédemment,
- R₃ est choisi parmi l'hydrogène, OR₆, SR₆, NR₆R'₆, CF₃, les radicaux alkyle en C₁-C₂₀, linéaires ou ramifiés, et les cycles hydrocarbonés de 4 à 7 atomes, séparés ou accolés, les radicaux alkyle et les cycles hydrocarbonés étant, en outre, saturés ou non et éventuellement substitués par au moins un substituant A₃, avec R₆ et R'₆ désignant indépendamment l'hydrogène, un radical alkyle en C₁-C₂₀, linéaire ou ramifié, ou un cycle hydrocarboné de 4 à 7 atomes, le radical alkyle ou le cycle hydrocarboné étant saturé ou non et éventuellement substitué par au moins un substituant A₄ ;
- R₄ est choisi parmi l'hydrogène, les radicaux alkyle en C₁-C₂₀, linéaires ou ramifiés, et les cycles hydrocarbonés de 4 à 7 atomes, séparés ou accolés, les radicaux alkyle et les cycles hydrocarbonés étant, en outre, saturés ou non éventuellement substitué par au moins un substituant choisi parmi OR₁₀, SR₁₀, NR₁₀R'₁₀, CF₃, avec R10 et R'10 désignant un radical alkyle en C₁-C₂₀ linéaire ou ramifié, saturé ou non;
- A₃ et A₄ sont indépendamment choisis parmi R₉, OR₉, SR₉, NR₉R'₉, COOR₉, avec R₉ et R'₉, désignant indépendamment l'hydrogène, un radical alkyle en C₁-C₂₀, linéaire ou ramifié, saturé ou non, un cycle hydrocarboné de 4 à 7 atomes, saturé ou non, ou un radical benzyle.

Comme exemples de composés tétrazoliques de formule (I) décrits dans la présente demande, on peut citer les composés suivants :
Composé 1 : 1-phenyl-2-(2-phenyl-2H-tetrazol-5-yl)ethanone 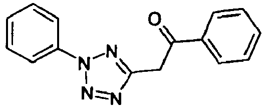
Composé 2 : 1-(2-methoxyphenyl)-2-(2-phenyl-2H-tetrazol-5-yl)ethanone 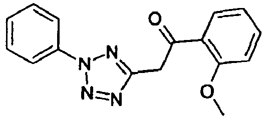
ethyl-2-(2-phenyl-2H-tetrazol-5-yl)acetate 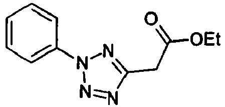
acide 2-(2-phenyl-2H-tetrazol-5-yl)acétique 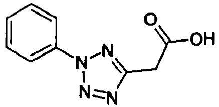
1-(3,4,5-trimethoxyphenyl)-2-(2-phenyl-2H-tetrazol-5-yl)ethanone 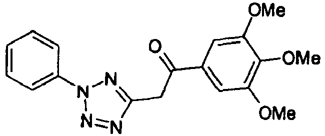
4-[2-(2-Phenyl-2H-tetrazol-5-yl)-acetyl]-benzoic acid 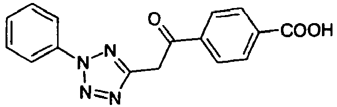
1-(4-Benzyloxy-phenyl)-2-(2-phenyl-2H-tetrazol-5-yl)-ethanone 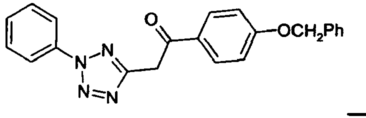
3-Methoxy-1-phenyl-2-(2-phenyl-2H-tetrazol-5-yl)-propan-1-one

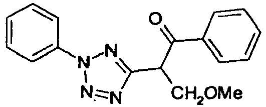

Comme exemples de composés tétrazoliques de formule (II) décrits dans la présente demande, on peut citer les composés suivants :
Composé 3 : N-(2-phenyl)-2-(5-phenyl-2H-tetrazol-2-yl)acetamide 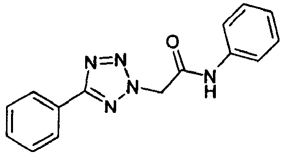
Composé 4 : N-(2-methoxyphenyl)-2-(5-phenyl-2H-tetrazol-2-yl)acetamide 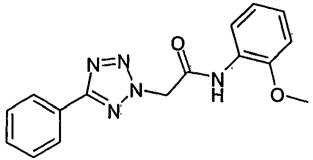
N-(4-methylphenyl)-2-{5-[3-(trifluoromethyl)phenyl]-2H-tetrazol-2-yl}acetamide: 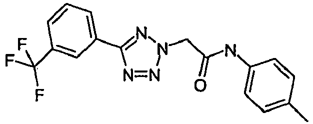
N-(2-methoxyphenyl)-2-(2H-tetrazol-2-yl)acetamide 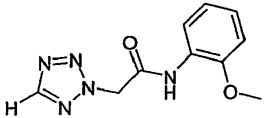
2-(5-phenyl-2H-tetrazol-2-yl)acetamide 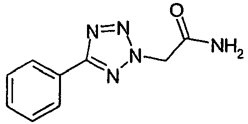
N-(2-methoxyphenyl)-2-[5-(2-naphtyl)-2H-tetrazol-2-yl]acetamide 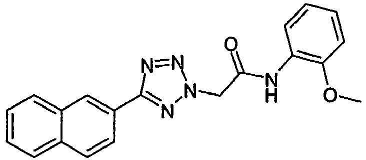
N-(2-methoxyphenyl)-2-[5-(4-methoxyphenyl)-2H-tetrazol-2-yl)acetamide 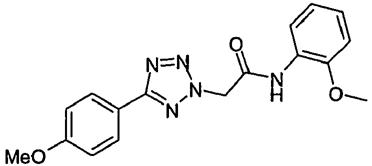
N-(3-hydroxypropyl)-2-(5-phenyl-2H-tetrazol-2-yl)acetamide 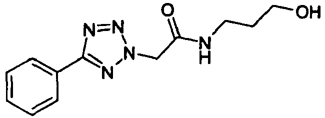
3-Hydroxy-N-(2-methoxy-phenyl)-2-(5-phenyl-tetrazol-2-yl)-propionamide 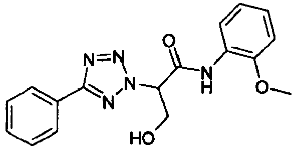
3-Methoxy-N-(2-methoxy-phenyl)-2-(5-phenyl-tetrazol-2-yl)-propionamide

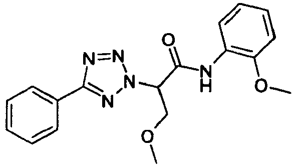

Les composés de formule (I) ou (II), salifiés ou non peuvent être fabriqués de façon connue.

### 1) Préparation de 5 - acétyl tétrazo/es (formule I)

Les composés (I) décrit ici peuvent être préparés par une méthode décrite dans la littérature : D. Moderhack et al., J. Chem. Soc. Perkin Trans. 1, 2001, 720-728. Le schéma réactionnel est le suivant:

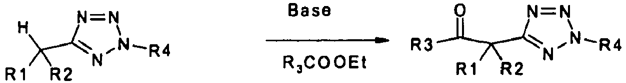

### 2)Préparation de 2 - acétyl tétrazoles (formule II)

Les composés de formule (II) décrits ici peuvent être préparés par l'alkylation par des réactifs α-chlorocarbonylés de tétrazoles substitués en position 5. Cette réaction est particulièrement adaptée dans le cas de la synthèse de 5-phényl-tétrazoles (correspondant à R₄ = phényle). Ce genre de préparation est connu de l'homme de l'art et notamment du document F. Eindberg, J. Org. Chem., 1970, 35, 11, 3978-3980.
Le schéma réactionnel peut être le suivant:

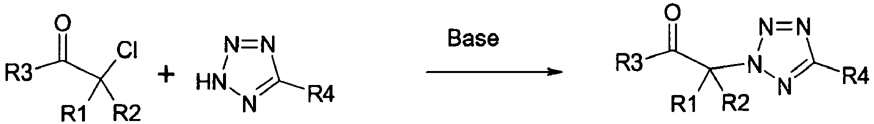

La quantité efficace d'un composé de formule (I) ou (II) ou de l'un de ses sels correspond à la quantité nécessaire pour obtenir le résultat désiré (à savoir augmenter la pigmentation des cheveux et/ou des poils et/ou de la peau). L'homme du métier est donc en mesure d'évaluer cette quantité efficace qui dépend de la nature du composé utilisé, de la personne à laquelle on l'applique, et du temps de cette application.

Dans la suite du texte, et sauf indication contraire, les quantités des différents ingrédients de la composition sont données en pourcentage en poids par rapport au poids total de la composition.

Pour donner un ordre de grandeur, tel que décrit ici, le composé de formule (I) ou de l'un de ses sels peut être utilisé en une quantité représentant de 10⁻³ % à 5% du poids total de la composition et préférentiellement en une quantité représentant de 10⁻²% à 2% du poids total de la composition, par exemple de 0,5 à 2 %.

### COMPOSES HETEROCYCLIQUES PHENYL-FURANNES, PHENYL-THIOPHENES et PHENYL-PYRROLES

Selon un autre mode de réalisation, les inhibiteurs de 15 PGDH utiles selon l'invention sont certains composés hétérocycliques et notamment certains phényl-furannes, phényl-thiophènes, phényl-pyrroles, salifiés ou non.
On peut donc utiliser selon l'invention au moins un composé hétérocyclique de formule (I) ou l'un de ses sels, 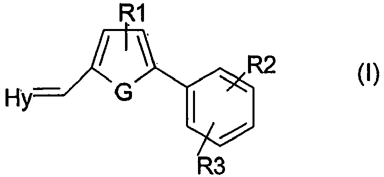 dans laquelle :
- Hy représente un hétérocycle à 4, 5, 6 ou 7 atomes comportant éventuellement au moins une fonction carbonyle et/ou une fonction thiocarbonyle, le dit hétérocycle étant éventuellement substitué par au moins un substituant choisi parmi un halogène, les groupes OR, SR, NRR', COR, CSR, NRCONR'R", C(=NR)R', C(=NR)NR'R", NRC(=NR')NR"R"', OCOR, COSR, SCOR, CSNRR', NRCSR', NRCSNR'R", COOR, CONRR', CF₃, CN, NRCOR', SO₂R', SO₂NRR', NRSO₂R', les radicaux alkyle linéaires ou ramifiés, saturés ou insaturés, en C₁-C₂₀, les cycles de 4 à 7 atomes, saturés ou insaturés, contenant éventuellement au moins un hétéroatome, ces cycles pouvant être séparés ou accolés, les radicaux alkyle et les cycles pouvant, en outre, être substitués, où R, R', R" et R"', identiques ou différents, désignent un hydrogène, un radical alkyle en C₁-C₂₀ linéaire ou ramifié ou un radical aryle, éventuellement substitué ;
- G représente O, S, NH ;
- R₁, R₂ et R₃ représentent indépendamment l'un de l'autre un hydrogène, un halogène, un groupe OR₀, SR₀, NR₀R₀', COR₀, CSR₀, NR₀CONR₀'R₀", C(=NR₀)R₀', C(=NR₀)NR₀'R₀", NR₀C(=NR₀')NR₀"R₀'", OCOR₀, COSR₀, SCOR₀, CSNR₀R₀', NR₀CSR₀', NR₀CSNR₀'R₀", COOR₀, CONR₀R₀', CF₃, NO₂, CN, NR₀COR'₀, SO₂R'₀, SO₂NR₀R'₀, NR₀SO₂R'₀, un radical alkyle linéaire ou ramifié, saturé ou insaturé, en C₁-C₂₀, au moins un cycle de 4 à 7 atomes, saturé ou insaturé, contenant éventuellement au moins un hétéroatome, les cycles pouvant être séparés ou accolés, les radicaux alkyle et les cycles pouvant, en outre, être substitués, où R₀, R₀', R₀" et R₀"', identiques ou différents, désignent un hydrogène, un radical alkyle en C₁-C₂₀ linéaire ou ramifié ou un radical aryle, éventuellement substitué.
les composés et les compositions les contenant seront utiles pour induire et/ou stimuler la pigmentation des fibres kératiniques notamment humaines comme les cils et les cheveux des êtres humains et/ou de la peau.

L'invention s'applique aussi aux fibres kératiniques des mammifères de l'espèce animale (chien, cheval ou chat par exemple).

L'invention se rapporte encore à l'utilisation cosmétique d'au moins un hétérocycle de formule (I) ou d'un de ses sels dans une composition cosmétique de soin et/ou de maquillage des fibres kératiniques humaines pour induire et/ou stimuler leur pigmenation ainsi qu'à l'utilisation d'au moins un composé de formule (I) ou de l'un de ses sels pour la préparation d'une composition de soin ou de traitement des fibres kératiniques humaines, destinée à induire et/ou stimuler la pigmenation des fibres.
Les fibres kératiniques humaines auxquelles s'applique l'invention sont notamment les cheveux, les sourcils, les cils, les poils de barbe, de moustache et les poils pubiens. Plus spécialement, l'invention s'applique aux cheveux et/ou aux cils humains.
La demande décrit également un procédé de traitement cosmétique des fibres kératiniques (cheveux ou cils notamment) et/ou de la peau, y compris du cuir chevelu et des paupières, en particulier destiné à stimuler la pigmentation des fibres kératiniques et/ou de la peau d'être humain, caractérisé par le fait qu'il consiste à appliquer sur les fibres kératiniques et/ou la peau, une composition cosmétique comprenant une quantité efficace d'au moins un composé de formule (I) ou d'un de ses sels, à laisser celle-ci en contact avec les fibres kératiniques et/ou la peau, et éventuellement à rincer les fibres et/ou ladite peau.

"Au moins un" selon l'invention signifie un ou plusieurs (2, 3 ou plus). En particulier, la composition peut contenir un ou plusieurs composés de formule (I). Ce ou ces composés peuvent être des isomères cis ou trans ou Z ou E ou un mélange d'isomères cis/trans ou Z/E. Ils peuvent aussi être sous forme tautomère. En particulier, l'hétérocycle Hy peut être en position cis ou trans ou Z ou E et mieux en position Z de la double liaison adjacente. Ce ou ces composés peuvent être des énantiomères et/ou des diastéréoisomères ou un mélange de ces isomères, en particulier un mélange racémique.

Par "radical alkyle" on entend au sens de l'invention un radical hydrocarboné qui peut être linéaire ou ramifié et saturé ou insaturé. De préférence, le radical alkyle comporte de 1 à 10 atomes de carbone. Comme exemple de radical alkyle utilisable dans l'invention, on peut citer les radicaux méthyle, éthyle, isopropyle, n-butyle, *ter*-butyle, n-hexyle, éthyle-2-hexyle, éthylène, propylène. Ce radical peut éventuellement être substitué en particulier par OR₀, avec R₀ valant H ou un radical alkyle, linéaire ou ramifié, saturé en C₁-C₂₀ et mieux en C₁-C₁₀ et par exemple en C₁-C₅.

Selon l'invention, le ou les hétéroatomes de Hy peuvent être O, N, S, P, Si, Se et en particulier O, N, S. L'hétérocycle Hy peut être saturé ou insaturé. En outre, il peut comporter 4, 5, 6 ou 7 atomes et une ou plusieurs fonctions carbonyle ou thio-carbonyle ou les deux, le carbone de ces fonctions faisant partie de l'hétérocycle.

Dans un mode particulier de réalisation de l'invention, Hy représente un cycle aromatique à 5 atomes comportant comme hétéroatome le soufre, l'azote et leurs associations. En outre, cet hétérocycle Hy comporte un ou deux groupements carbonyle, groupements dont le carbone fait partie de l'hétérocycle. A titre d'exemple, cet hétérocycle présente la formule (II) suivante : 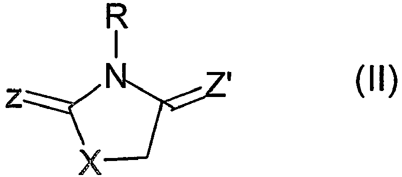 où Z, Z' et X représentent indépendamment S ou O et R représente H ou un radical alkyle linéaire ou ramifié, saturé en C₁-C₁₀. X peut aussi représenter NH. Avantageusement, Z et Z' représentent l'oxygène, ce qui correspond à un cycle 1,3-thiazolidine-2,4-dione.

Selon l'invention, les cycles employés comme substituant (S₁) comportent de 4 à 7 atomes et mieux de 5 à 6 atomes. Ils peuvent être saturés ou insaturés et comporter éventuellement un ou plusieurs hétéroatomes tels que S, N, O ou leurs associations. De plus, ces cycles peuvent être seuls ou accolés à un autre cycle de même structure chimique ou non. Lorsqu'ils sont accolés, ils forment des cycles condensés.

Comme cycles hydrocarbonés saturés utilisables on peut citer le radical cyclopentyle ou cyclohexyle et comme cycles hydrocarbonés insaturés, on peut citer le cycle cyclohexényle ou phényle. Comme cycles hydrocarbonés accolés, on peut citer le radical naphtyle. Comme hétérocycle, on peut citer les cycles pyridine, pipéridine, morpholine, pyrrole, furanne, thiazole. En outre, ces cycles peuvent être substitués par un ou plusieurs substituants ayant la définition indiquée ci-dessus pour R ou R₀.

Selon l'invention, les composés de formule (I) sont sous forme isolée, c'est-à-dire non polymérique. Ce sont des phényl-furannes, phényl-thiophènes ou phényl-pyrroles. En outre, R₁ peut être situé en position 3 ou 4, en considérant G en position 1 de l'hétérocycle à 5 atomes. Par ailleurs, R₂ et R₃ peuvent être situés en toute position du cycle phényle les portant et en particulier en position para ou méta de la partie (A) suivante :

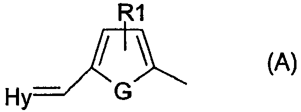

De préférence, R₁ représente un atome d'hydrogène.

Avantageusement, l'un au moins des R₂ et R₃ représentent CF₃, OR₀ ou COOR₀ avec R₀ valant H ou un radical alkyle, linéaire ou ramifié, saturé ou non en C₁-C₂₀ et mieux en C₁-C₁₀. Comme exemple de radical alkyle utilisable, on peut citer méthyle, éthyle, tertio-butyle, iso-propyle, n-butyle, n-hexyle. En particulier COOR₀ représente COOH ou COOCH₂-CH₃. En outre, OR₀ représente notamment OH ou OCH₃ .En particulier, R₂ représente COOH ou OH et R₃ représente H ; R₂ représente COOCH₂-CH₃ et R₃ représente H ; ou R₂ et R₃ représentent CF₃ ou OCH₃.

Par sels de composé de formule (I) on entend selon l'invention les sels organiques ou inorganiques, simples ou doubles, d'un composé de formule (I).

Comme sels inorganiques utilisables selon l'invention on peut citer : les sels simples ou doubles de sodium ou de potassium ainsi que les sels de zinc (Zn²⁺), de calcium (Ca²⁺), de cuivre (Cu²⁺), de fer (Fe²⁺), de strontium (Sr²⁺), de magnésium (Mg²⁺), d'ammonium et de manganèse (Mn²⁺) ; les hydroxydes, les carbonates, les halogénures (comme les chlorures), les sulfates, les nitrates, les phosphates. De préférence, le sel est un sel de sodium.

Les sels organiques utilisables selon l'invention sont par exemple les sels de tri-éthanolamine, mono-éthanolamine, di-éthanolamine, hexadécylamine, N,N,N',N'-tétrakis-(hydroxy-propyl-2) éthylène diamine, tris-hydroxyméthyl aminométhane.

Selon un mode particulier de réalisation de l'invention, les composés hétérocycliques auxquels s'applique l'invention, présentent la formule (III) et mieux la formule (IIIa) suivantes ou la forme sel (mono ou disel) correspondante : 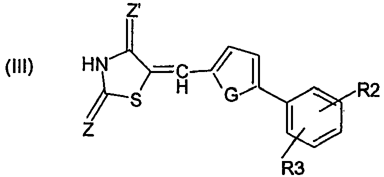 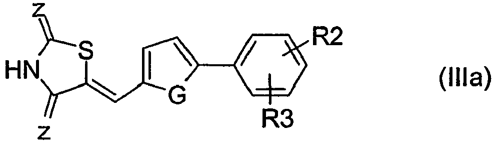 dans laquelle Z, Z' et G représentent indépendamment O ou S ; l'un au moins des R₂ et R₃ représentent CF₃, OR₀ ou COOR₀ avec R₀ valant H ou un radical alkyle, linéaire ou ramifié, saturé ou non en C₁-C₂₀ et mieux en C₁-C₁₀.

L'invention a encore pour objet de nouveau composé hétérocyclique de formule (IV) suivante ou sous forme d'un des ses sels, présentant notamment la propriété d'inhiber la 15-PGDH et/ou de préserver la quantité et/ou l'activité des prostaglandines notamment au niveau du follicule pileux des êtres humains : 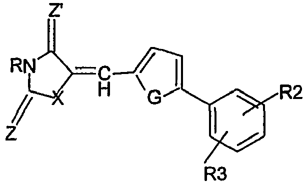 dans laquelle Z, Z' et G représentent indépendamment O ou S ; X représente O, NH ou S ; R représente l'hydrogène ou un radical alkyle linéaire ou ramifié, saturé en C₁-C₁₀ ; l'un au moins des R₂ et R₃ représentent un hydrogène, CN, NO₂, CF₃, un radical phényle, OR₀ ou COOR₀, un radical alkyle linéaire ou ramifié, saturé en C₁-C₂₀ et mieux en C₁-C₁₀, éventuellement substitué par OR₀, avec R₀ valant H ou un radical alkyle, linéaire ou ramifié, saturé en C₁-C₂₀ et mieux en C₁-C₁₀, à condition que lorsque X = S et Z = Z' = G ou Z ≠ Z', alors R₂ et R₃ soient différents de COOH.

Selon un mode de réalisation particulier, le composé hétérocyclique présente la formule (V) suivante ou un sel correspondant : 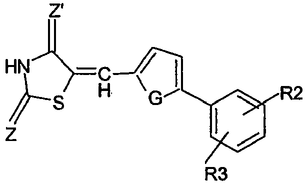 dans laquelle Z, Z' et G représentent indépendamment O ou S ; l'un au moins des R₂ et R₃ représentent phényle, NO₂, CF₃, OR₀, OR₀, COOR₀ ou un radical alkyle linéaire ou ramifié, saturé en C₁-C₂₀ et mieux en C₁-C₁₀, éventuellement substitué par OR₀, avec R₀ valant H ou un radical alkyle, linéaire ou ramifié, saturé en C₁-C₂₀ et mieux en C₁- C₁₀, à condition que lorsque Z = Z' = G ou Z ≠ Z', alors R₂ et R₃ soient différents de COOH.

Avantageusement, lorsque Z = Z' = G, l'un au moins des R₂ et R₃ représentent CF₃, OR₀ ou COOR₀ avec R₀ valant un radical alkyle, linéaire ou ramifié, saturé en C₁-C₁₀ et mieux en C₁-C₅. Selon un autre mode préféré de réalisation de l'invention, lorsque Z = Z' et sont différents de G, l'un au moins des R₂ et R₃ représente CF₃ ou COOR₀, avec R₀ valant H.

Selon un autre de mode de réalisation de l'invention, le composé hétérocyclique présente la formule (VI) suivante ou une forme sel correspondante : 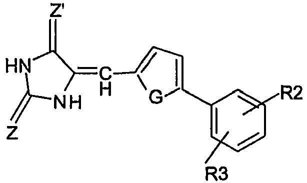 dans laquelle Z, Z' et G représentent indépendamment O ou S ; l'un au moins des R₂ et R₃ représentent un hydrogène, CN, CF₃, NO₂, OR₀, COOR₀ ou un radical alkyle linéaire ou ramifié, saturé en C₁-C₂₀ et mieux en C₁-C₁₀, éventuellement substitué par OR₀, avec R₀ valant H ou un radical alkyle, linéaire ou ramifié, saturé en C₁-C₂₀ et mieux en C₁-C₁₀.

Selon un autre de mode de réalisation de l'invention, le composé hétérocyclique présente la formule (VII) suivante ou la forme sel correspondante : 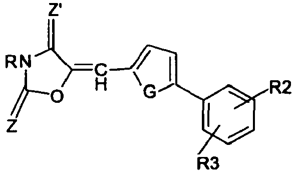 dans laquelle Z, Z' et G représentent indépendamment O ou S ; R représente un radical alkyle linéaire ou ramifié, saturé en C₁-C₁₀ ; l'un au moins des R₂ et R₃ représentent un radical alkyle linéaire ou ramifié, saturé en C₁-C₂₀ et mieux en C₁-C₁₀, NO₂, OR₀ avec R₀ valant H ou un radical alkyle, linéaire ou ramifié, saturé en C₁-C₂₀ et mieux en C₁-C₁₀.

De préférence, le composé hétérocyclique de l'invention est sous forme Z.

Les composés de formule (I) ou leurs sels peuvent être fabriqués de façon connue comme décrite dans le document WO01/066541. Les composés de formule (I) sont solides à température ambiante.

### COMPOSES STYRYL-PYRAZOLE:

La présente demande décrit aussi des inhibiteurs de 15-PGDH utiles qui comprennent au moins un composé styryl pyrazole ou l'un de ses sels physiologiquement acceptables.
De tels composés répondent à la formule (I): 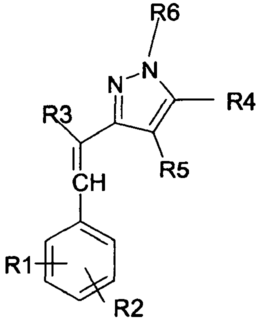 dans laquelle :
- R₁, R₂, R₄ et R₅ identiques ou différents sont choisis parmi l'hydrogène, un halogène, les groupes OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C(=NR'₇)NR"₇R"'₇, NR₇CSR'₇, NR₇CSNR'₇R"₇, les radicaux alkyle, saturés ou insaturés, linéaires ou ramifiés, en C₁-C₂₀, les cycles de 4 à 7 atomes, saturés ou insaturés, contenant éventuellement au moins un hétéroatome, ces cycles pouvant être séparés ou accolés, les radicaux alkyle et les cycles pouvant, en outre, être substitués par au moins un substituant A₁, avec R₇, R'₇, R"₇ et R"'₇, désignant indépendamment l'hydrogène, un radical alkyle en C₁-C₂₀, linéaire ou ramifié ou un cycle de 4 à 7 atomes, contenant éventuellement au moins un hétéroatome, isolé ou accolé à un autre cycle, le radical alkyle ou lesdits cycles étant saturés ou non et éventuellement substitués par au moins un substituant A₂ ;
- R₃ est choisi parmi CN, COOR₈, CONR₈R'₈, COR₈, SO₂R₈, SO₂NR₈R'₈, avec R₈ et R'₈ désignant indépendamment l'hydrogène, un radical alkyle en C₁-C₂₀, linéaire ou ramifié ou un cycle de 4 à 7 atomes, isolé ou accolé à un autre cycle et contenant éventuellement au moins un hétéroatome, le radical alkyle ou lesdits cycles étant saturés ou non et éventuellement substitués par un au moins un substituant A₃ ;
- R₆ est choisi parmi l'hydrogène, les groupes COOR₉, COR₉, CSR₉, COSR₉, CONR₉R'₉, SO₂R₉, SO₂NR₉R'₉, les radicaux alkyle en C₁-C₂₀, linéaires ou ramifiés, saturés ou non et les cycles de 4 à 7 atomes, saturés ou insaturés, contenant éventuellement au moins un hétéroatome, ces cycles pouvant être séparés ou accolés, les radicaux alkyle et les cycles pouvant, en outre, être, substitués par au moins un substituant A₄, avec R₉ et R'₉ identiques ou différents désignant l'hydrogène, un radical alkyle en C₁-C₂₀, linéaire ou ramifié ou un cycle de 4 à 7 atomes, contenant éventuellement au moins un hétéroatome, isolé ou accolé à un autre cycle, le radical alkyle ou lesdits cycles étant saturés ou non et éventuellement substitués par au moins un substituant A₅ ;
- A₁, A₂, A₃, A₄ et A₅ étant indépendamment choisis parmi les halogènes, les groupes OR₁₀, SR₁₀, NR₁₀R'₁₀, COOR₁₀, CH₂COOR₁₀, CONR₁₀R'₁₀, CF₃, CN, NR₁₀₇COR'₁₀, SO₂R₁₀, SO₂NR₁₀R'₁₀, NR₁₀SO₂R'₁₀, COR₁₀, CSR₁₀, OCOR₁₀, COSR₁₀, SCOR₁₀, CSNR₁₀R'₁₀, NR₁₀CONR'₁₀R"₁₀, NR₁₀C(=NR'₁₀)NR"₁₀R"'₁₀, NR₁₀CSNR'₁₀R"₁₀, NR₁₀CSR'₁₀, avec R₁₀, R'₁₀, R"₁₀ et R"'₁₀ identiques ou différents désignant l'hydrogène, un radical alkyle en C₁-C₂₀, linéaire ou ramifié ou un cycle de 4 à 7 atomes, contenant éventuellement au moins un hétéroatome, isolé ou accolé à un autre cycle, le radical alkyle ou lesdits cycles étant saturés ou non.

La demande décrit encore l'utilisation notamment cosmétique d'au moins un composé pyrazolique de formule (I) ou de l'un de ses sels, tel que défini précédemment, comme agent pour induire et/ou stimuler la pigmentation des fibres kératiniques notamment humaines comme les cils et les cheveux des êtres humains et/ou de la peau.

L'invention s'applique aussi aux fibres kératiniques des mammifères de l'espèce animale (chien, cheval ou chat par exemple).

La demande décrit encore l'utilisation cosmétique d'au moins un composé pyrazolique de formule (I) ou d'un de ses sels dans une composition cosmétique de soin et/ou de maquillage des fibres kératiniques humaines pour induire et/ou stimuler leur pigmentation et/ou celle de la peau ainsi qu'à l'utilisation d'au moins un composé de formule (I) ou de l'un de ses sels pour la préparation d'une composition de soin ou de traitement des fibres kératiniques humaines, destinée à induire et/ou stimuler la pigmentation des fibres et/ou de la peau.

Les fibres kératiniques humaines auxquelles s'applique l'invention sont notamment les cheveux, les sourcils, les cils, les poils de barbe, de moustache et les poils pubiens. Plus spécialement, l'invention s'applique aux cheveux et/ou aux cils humains.
La demande décrit encore l'utilisation cosmétique d'au moins un composé pyrazolique de formule (I) ou de l'un de ses sels dans une composition cosmétique de soin capillaire d'être humain pour favoriser la pigmentation des cheveux et/ou de la peau.
La demande décrit encore l'utilisation cosmétique d'au moins un composé pyrazolique de formule (I) ou d'un de ses sels, dans une composition cosmétique de soin et/ou de maquillage des cils d'être humain, pour induire et/ou stimuler la pigmentation des cils et/ou augmenter leur densité ainsi que l'utilisation d'au moins un composé de formule (I) ou d'un de ses sels, pour la préparation d'une composition de soin et/ou de traitement des cils d'être humain, destinée à induire et/ou stimuler la pigmentation des cils. Cette composition permet ainsi de maintenir en bon état les cils et/ou améliorer leur état et/ou leur aspect.
"Au moins un" selon l'invention signifie un ou plusieurs (2, 3 ou plus). En particulier, la composition peut contenir un ou plusieurs composés de formule (I). Ce ou ces composés peuvent être des isomères cis ou trans ou Z ou E ou un mélange d'isomères cis/trans. ou Z/E En particulier, le cycle aromatique peut être en position cis ou trans ou Z ou E et mieux en position Z par rapport au cycle pyrazole. Ce ou ces composés peuvent aussi être sous forme tautomère. Ils peuvent être également des énantiomères et/ou des diastéréoisomères ou un mélange de ces isomères, en particulier un mélange racémique.

Tel que décrit ici, les cycles employés pour R₁ à R₁₀, R'₇, R"₇, R"'₇, R'₈, R'₉, R'₁₀, R"₁₀ et R"'₁₀ comportent de 4 à 7 atomes et mieux de 5 à 6 atomes. Ils peuvent être saturés ou insaturés et comporter éventuellement un ou plusieurs hétéroatomes tels que S, N, O ou leurs associations. Ils peuvent être seuls ou accolés à un autre cycle qui peut être identique ou différent. Comme cycles carbonés saturés utilisables on peut citer le radical cyclopentyle ou cyclohexyle. Comme hétérocycle, on peut citer les cycles pyridine, pipéridine, morpholine, pyrrole, furanne, thiazole. Comme cycles carbonés insaturés, on peut citer le radical phényle, naphtyle. En outre, ces cycles peuvent être substitués par un substituant ayant la définition indiquée ci-dessus pour A₁. Avantageusement, lorsque R₆ comporte un ou plusieurs hétéroatomes, la liaison avec l'azote du cycle pyrazole se fait sous forme d'une liaison N-C.

Selon un mode de réalisation décrit ici, le ou les substituants portés par les radicaux alkyle ou aryle, à savoir A₁ à A₅ sont les atomes d'halogène et notamment les atomes de chlore, brome, iode ou fluor, de préférence les atomes de chlore ou les radicaux alkyle en C₁-C₂₀, linéaires ou ramifiés ou encore les radicaux alkyle perfluorés. Comme exemple de radicaux alkyle perfluorés utilisables, on peut citer CF₃.

Par "radical alkyle" on entend au sens de l'invention un radical hydrocarboné qui peut être linéaire ou ramifié et saturé ou insaturé. De préférence, le radical alkyle comporte de 1 à 10 atomes de carbone.

Comme exemple de radical alkyle utilisable selon l'invention, on peut citer méthyle, éthyle, iso-propyle, n-butyle, n-hexyle, éthyl-2-héxyle, éthylène, propylène.

Tel que décrit ici, les composés de formule (I) (ou leur(s) sel(s)) sont sous forme isolée, c'est-à-dire non polymérique. En outre, R₁ et R₂ peuvent être situés en toute position du cycle phénylique et en particulier en position ortho du branchement de la partie pyrazole.

Avantageusement, R₁ et R₂ ne représentent pas simultanément OR₇.

De préférence, l'un au moins des R₁ et R₂ représentent un atome d'hydrogène OR₇ CF₃, un atome d'halogène et notamment un atome de chlore, R₇ représentant un radical alkyle en C₁-C₁₀ et par exemple méthyle. En particulier, R₁ et/ou R₂ représentent un atome d'halogène et notamment de chlore.

Avantageusement, R₃ représente CN, COOR₈, CONR₈R'₈, COR₈ et par exemple CN.

Selon un mode de réalisation décrit ici, R₄, R₅, R₆ représentent indépendamment l'un de l'autre un radical alkyle en C₁-C₁₀ éventuellement substitué par OR₁₀ comme CH₂CH₂OR₁₀, NH₂, H, ou CN, ou un cycle hydrocarboné, saturé ou non, comme un cycle phényle, R₁₀ représentant par exemple H. Avantageusement, R₆ représente CH₂CH₂OR₁₀ et en particulier CH₂CH₂OH ou le radical phényle. De préférence, R₄ représente NH₂ ou H. Selon un mode avantageux, R₅ représente CN ou H.

Par sels de composé de formule (I) on entend ici, les sels organiques ou inorganiques d'un composé de formule (I).

Comme sels inorganiques utilisables décrits ici, on peut citer les sels de sodium ou de potassium ainsi que les sels de zinc (Zn²⁺), de calcium (Ca²⁺), de cuivre (Cu²⁺), de fer (Fe²⁺), de strontium (Sr²⁺), de magnésium (Mg²⁺), d'ammonium et de manganèse (Mn²⁺) ; les hydroxydes, et les carbonates, les halogénures (chlorures), les sulfates, les nitrates, les phosphates.

Les sels organiques utilisables décrits ici sont par exemple les sels de tri-éthanolamine, mono-éthanolamine, di-éthanolamine, hexadécylamine, N,N,N',N'-tétrakis-(hydroxy-propyl-2) éthylène diamine, tris-hydroxyméthyl aminométhane.

Selon un mode particulier de réalisation décrit ici, les composé pyrazoliques utilisés présentent la formule (II) ou un de ses sels : 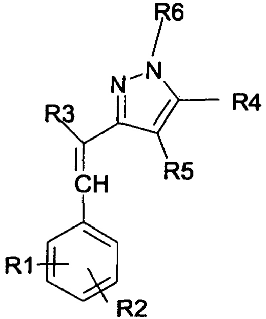 dans laquelle :
- R₁, R₂, R₄ et R₅ représentent indépendamment H, un halogène, OR₇, SR₇, NR₇R'₇, COOR₇, CONR₇R'₇, CF₃, CN, un radical alkyle, saturé ou non, en C₁-C₁₀, un cycle saturé ou non, séparé ou accolé à un autre cycle, contenant éventuellement au moins un hétéroatome, les radicaux alkyle et les cycles pouvant, en outre, être substitués par au moins un substituant A₁, avec R₇, R₇' désignant indépendamment H, un radical alkyle en C₁-C₁₀ ou un cycle isolé ou accolé à un autre cycle ;
- R₃ représente CN, COOR₈, CONR₈R'₈, COR₈, avec R₈ et R'₈ désignant indépendamment H, un radical alkyle en C₁-C₁₀ ou un cycle isolé ou accolé à un autre cycle et contenant éventuellement au moins un hétéroatome, lesdits cycles étant saturés ou non et éventuellement substitués par au moins un substituant A₁ ;
- R₆ représente l'hydrogène, COOR₉, COR₉, un radical alkyle en C₁-C₁₀ saturé ou non ou un cycle saturé ou non, séparé ou accolé à un autre cycle, contenant éventuellement au moins un hétéroatome, les radicaux alkyle et les cycles pouvant, en outre, être, substitués par au moins un substituant A₁, avec R₉ et R'₉ désignant indépendamment H, un radical alkyle en C₁-C₂₀ ou un cycle isolé ou accolé à un autre cycle ;
- les cycles ayant de 5 à 6 atomes ;
- les hétéroatomes étant O, N, S ou leur association.

Avantageusement, le composé de formule (I) ou (II) est sous forme Z.

Selon un autre mode de réalisation décrit ici, les composés pyrazoliques présentent la formule (III) suivante ou un de ses sels: 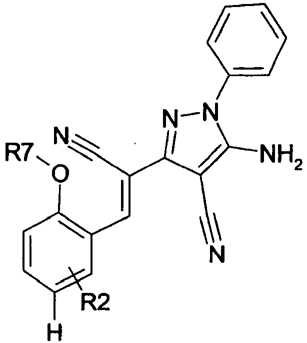
R₇ représente
   a) un radical alkyle en C₁-C₁₀, linéaire ou ramifié, saturé ou non, éventuellement substitué par au moins un substituant A₁ ; ou
   b) un cycle C¹ de 4 à 7 atomes, saturé ou insaturé, contenant éventuellement au moins un hétéroatome et/ou étant éventuellement substitué par au moins un substituant A₁ et/ou éventuellement accolé à au moins un cycle C² de 4 à 7 atomes, saturé ou insaturé, contenant éventuellement au moins un hétéroatome ;
R₂ représente
   - OR₇, SR₇, NR₇R'₇, COOR₇, CONR₇R'₇, CF₃, CN, NR₇COR'₇, SO₂R₇, SO₂NR₇R'₇, NR₇SO₂R'₇, COR₇, CSR₇, OCOR₇, COSR₇, SCOR₇, CSNR₇R'₇, NR₇CONR'₇R"₇, NR₇C(=NR'₇)NR"₇R"'₇, NR₇CSR'₇, NR₇CSNR'₇R"₇, un radical alkyle, saturé ou non, en C₁-C₁₀, un cycle C³ saturé ou non, séparé ou accolé à un autre cycle C⁴, contenant éventuellement au moins un hétéroatome, les radicaux alkyle et les cycles pouvant, en outre, être substitués par au moins un substituant A₁ où R₇ et R'₇ identiques ou différents désignent :
      l'atome d'hydrogène ou un radical alkyle en C₁-C₁₀, linéaire ou ramifié, saturé ou non,
   - un cycle aromatique C₅ incluant éventuellement au moins un hétéroatome, éventuellement substitué par au moins un substituant A₂ ; et
où les hétéroatomes sont choisis parmi N, O, S et leur association.

Les composés de formule (III) ou leur sel étant nouveaux, la demande décrit encore un composé styryl-pyrazole de formule (III) ou un de ses sels. Avantageusement, R₂ représente OR₇ et R₇ représente un radical alkyle saturé en C₁-C₁₀ tel que le méthyle.

Comme exemples de composés pyrazoliques de formule (I) utilisables décrits ici on peut citer les composés suivants :
Composé 1 : 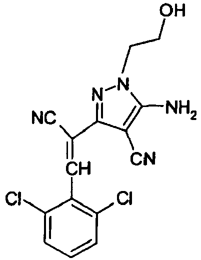
Composé 2 : 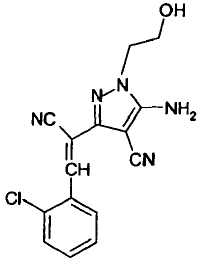
Composé 3 : 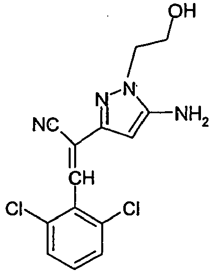
Composé 4 : 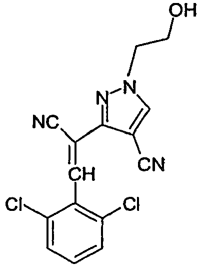
Composé 5:

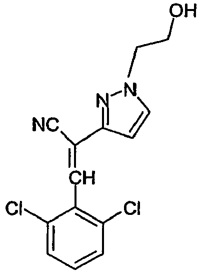

Les composés de formule (I) ou leurs sels dont certains sont connus en tant que tels, peuvent être fabriqués par la condensation d'un benzaldéhyde, éventuellement substitué avec un pyrazole substitué par un méthylène activé, par l'une des fonctions nitrile, acide, ester, amide ou cétone. L'élimination d'eau est réalisée simultanément par distillation azéotropique et la mise en place d'un appareil type "Dean-Stark". Ce genre de préparation est connu par l'homme de l'art du document EP 0 245 825. Ces composés se présentent sous forme de solide et en particulier sous forme pulvérulente ou bien sous forme liquide.

Dans la suite du texte, et sauf indication contraire, les quantités des différents ingrédients de la composition sont données en pourcentage en poids par rapport au poids total de la composition.

Pour donner un ordre de grandeur, que décrit ici, le composé de formule (I) ou de l'un de ses sels ou mélange de composés de formule (I) et/ou de leur sel peut être utilisé en une quantité représentant de 10⁻³ % à 105% du poids total de la composition et préférentiellement en une quantité représentant de 10⁻³% à 5% et mieux de 10⁻²% à 2% du poids total de la composition, par exemple de 0,5 à 2 %.

### COMPOSES PYRAZOL-CARBOXAMIDE

Selon un autre mode de réalisation, l'inhibiteur de 15-PGDH utile décrit ici comprend au moins un composé pyrazolique de formule (I) ou l'un de ses sels: 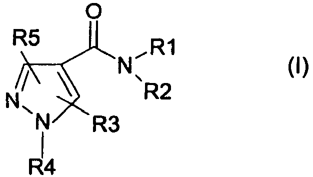 dans laquelle :
- R₁ et R₂, sont choisis indépendamment parmi :
   - l'hydrogène,
   - les radicaux alkyle en C₁-C₂₀, saturés ou insaturés, linéaires ou ramifiés éventuellement substitués par au moins un substituant T₁,
   - les cycles saturés ou insaturés, contenant au moins un hétéroatome choisi parmi O, N, S et les cycles saturés hydrocarbonés, ces cycles contenant de 4 à 7 atomes et pouvant éventuellement être accolés, comporter une fonction carbonyle ou thiocarbonyle et/ou être substitués par au moins un substituant T₂ choisi parmi A et R, R₁ et R₂ pouvant en outre former un hétérocycle de 4 à 7 atomes avec l'azote auquel ils sont liés ;
- R₃ et R₅ sont choisis indépendamment parmi :
   - l'hydrogène,
   - A,
   - les halogènes,
   - les groupes OR₆, SR₆, NR₆R'₆, CN, CF₃, COR₆, CSR₆, COOR₆, COSR₆, CSOR₆, CSSR₆, NR₆COR'₆, NR₆CSR'₆, OCOR₆, SCOR₆, CSNR₆R'₆, SO₂R₆, SO₂NR₆R'₆, NR₆SO₂R'₆, NR₆C(=NR'₆)NR"₆R"'₆, SiR₆R'₆R"₆,
   - les cycles saturés ou insaturés, de 4 à 7 atomes, contenant éventuellement au moins un hétéroatome parmi O, N, S, ces cycles pouvant éventuellement être accolés, comporter une fonction carbonyle ou thiocarbonyle et/ou être substitués par au moins un substituant T₃ choisi parmi A et R ;
- R₄ est choisi parmi :
   - l'hydrogène,
   - A,
   - les groupes COR₆, CSR₆, COOR₆, CONR₆R'₆, CSNR₆R'₆, SO₂R₆, SO₂NR₆R'₆,
   - les cycles hydrocarbonés saturés ou insaturés, de 4 à 7 atomes, les hétérocycles à 5 atomes contenant de un à quatre hétéroatomes, les hétérocycles à 6 atomes contenant de un à trois hétéroatomes non-adjacents, les hétérocycles à 4 ou 7 atomes contenant de un à trois hétéroatomes, les hétéroatomes étant choisis parmi O, N, S, ces hétérocycles étant saturés ou insaturés, lesdits cycles et lesdits hétérocycles pouvant éventuellement être accolés, comporter une fonction carbonyle ou thiocarbonyle et/ou être substitués par au moins un substituant T₄ choisi parmi A et R ;
- R₆, R'₆, R"₆ et R"'₆ sont choisis parmi :
   - l'hydrogène,
   - les radicaux alkyle en C₁-C₂₀, saturés ou insaturés, linéaires ou ramifiés éventuellement substitués par au moins un substituant R',
   - les cycles saturés ou insaturés, de 4 à 7 atomes, contenant éventuellement au moins un hétéroatome parmi O, N, S, ces cycles pouvant éventuellement être accolés, comporter une fonction carbonyle ou thiocarbonyle et/ou être substitués par au moins un substituant R ;
- R est choisi parmi :
   - les radicaux alkyle en C₁-C₂₀, saturés ou insaturés, linéaires ou ramifiés,
   - les halogènes,
   - les groupes OR₇, SR₇, NR₇R'₇, CN, CF₃, COR₇, CSR₇, COOR₇, COSR₇, CSOR₇, CSSR₇, NR₇COR'₇, NR₇CSR'₇, OCOR₇, SCOR₇, CSNR₇R'₇, SO₂R₇, SO₂NR₇R'₇, NR₇SO₂R'₇, NR₇C(=NR'₇)NR"₇R"'₇ et SiR₇R'₇R"₇ ;
- R' est choisi parmi :
   - les radicaux alkyle en C₁-C₂₀, saturés ou insaturés, linéaires ou ramifiés,
   - les halogènes,
   - les groupes OR₇, SR₇, NR₇R'₇, CN, CF₃, COR₇, CSR₇, COOR₇, COSR₇, CSOR₇, CSSR₇, NR₇COR'₇, NR₇CSR'₇, OCOR₇, SCOR₇, CSNR₇R'₇, SO₂R₇, SO₂NR₇R'₇, NR₇SO₂R'₇, NR₇C(=NR'₇)NR"₇R"'₇ et SiR₇R'₇R"₇,
   - les cycles saturés ou insaturés, de 4 à 7 atomes, contenant éventuellement au moins un hétéroatome parmi O, N, S, ces cycles pouvant éventuellement être accolés et/ou comporter une fonction carbonyle ou thiocarbonyle ;
- R₇, R'₇, R"₇ et R"'₇ représentent indépendamment l'hydrogène ou un alkyle en C₁-C₂₀ saturé ou insaturé, linéaire ou ramifié ;
- A représente un radical alkyle en C₁-C₂₀, saturé ou insaturé, linéaire ou ramifié, éventuellement substitué par au moins un substituant T₅ choisi parmi : R' et les cycles saturés ou insaturés de 4 à 7 atomes contenant éventuellement au moins un hétéroatome parmi O, N, S, ces cycles pouvant éventuellement être accolés, comporter une fonction carbonyle ou thiocarbonyle et/ou être substitués par au moins un substituant R ;
- T₁ est choisi parmi OR₆, SR₆, NR₆R'₆, CN, CF₃, COR₆, CSR₆, COOR₆, COSR₆, CSOR₆, CSSR₆, NR₆COR'₆, NR₆CSR'₆, OCOR₆, SCOR₆, CSNR₆R'₆, SO₂R₆, SO₂NR₆R'₆, NR₆SO₂R'₆, NR₆C(=NR'₆)NR"₆R"'₆, SiR₆R'₆R"₆, les halogènes, les cycles saturés ou insaturés de 4 à 7 atomes contenant éventuellement au moins un hétéroatome parmi O, N, S, ces cycles pouvant éventuellement être accolés, comporter une fonction carbonyle ou thiocarbonyle et être substitués par au moins un substituant R.

La demande décrit encore l'utilisation d'au moins un composé pyrazolique de formule (I) ou de l'un de ses sels, tel que défini précédemment, comme agent pour induire et/ou stimuler la pigmentation des fibres kératiniques notamment humaines comme les cheveux et les cils des êtres humains et/ou de la peau.

L'invention s'applique aussi aux fibres kératiniques des mammifères de l'espèce animale (chien, cheval ou chat par exemple).

La demande décrit encore l'utilisation cosmétique d'au moins un composé pyrazolique de formule (I) ou d'un de ses sels dans une composition cosmétique de soin et/ou de maquillage des fibres kératiniques humaines pour induire et/ou stimuler leur pigmentation, ainsi que l'utilisation d'au moins un composé de formule (I) ou de l'un de ses sels pour la préparation d'une composition de soin ou de traitement des fibres kératiniques humaines, destinée à induire et/ou stimuler la pigmentation des fibres.
Les fibres kératiniques humaines auxquelles s'applique l'invention sont notamment les cheveux, les sourcils, les cils, les poils de barbe, de moustache et les poils pubiens. Plus spécialement, l'invention s'applique aux cheveux et/ou aux cils humains.

La demande décrit encore l'utilisation cosmétique d'au moins un composé pyrazolique de formule (I) ou de l'un de ses sels dans une composition cosmétique de soin capillaire d'être humain pour réduire la canite et/ou augmenter la pigmentation. Elle décrit encore l'utilisation d'au moins un composé pyrazolique de formule (I) ou de l'un de ses sels pour la préparation d'une composition capillaire pour être humain, destinée à induire et/ou stimuler la pigmentation des cheveux et/ou freiner leur blanchiment.
La demande décrit notamment l'utilisation de nouveaux composés pyrazole-carboxamide de formule III ou l'un de ses sels : 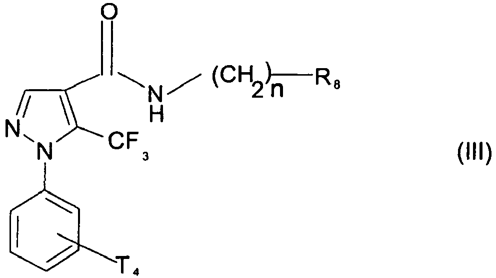 où R₈ représente OH ou -S-(CH₂)ₘ-R_{9'}, avec R₉ représentant H ou Hy ; T4 représente H ou 4-COOH ; n représente un entier allant de 1 à 10 et m représente un entier allant de 1 à 10 ; Hy représente un hétérocycle de 4 à 7 atomes.

Dans la suite du texte, et sauf mention exprès, l'emploi du terme composé de formule (I) doit être compris comme signifiant aussi bien le composé de formule (I) sous forme acide ou basique, que l'un de ses sels.

"Au moins un" selon l'invention signifie un ou plusieurs (2, 3 ou plus). En particulier, la composition peut contenir un ou plusieurs composés de formule (I). Ce ou ces composés peuvent être des isomères cis ou trans ou Z ou E ou un mélange d'isomères cis/trans ou Z/E. Ils peuvent aussi être sous forme tautomère. Ce ou ces composés peuvent aussi être des énantiomères et/ou des diastéréoisomères ou un mélange de ces isomères, en particulier un mélange racémique.

Tel que décrit ici, les cycles employés pour R₁ R₂, R₃, R₄, R₅, R₆, R'₆, R"₆, R"'₆, R', T₁ et T₅ comportent de 4 à 7 atomes et mieux de 5 à 6 atomes. Ils peuvent être saturés ou insaturés et comporter éventuellement un ou plusieurs hétéroatomes tels que S, N, O ou leurs associations. Comme cycles carbonés saturés utilisables on peut citer le radical cyclopentyle ou cyclohexyle. Comme hétérocycle, on peut citer les cycles pyridine, pipéridine, morpholine, pyrrole, furanne, thiophène, imidazole, oxazole, thiazole, pyrazole, pyrimidine, pyrazine, pyridazine. Comme cycles carbonés insaturés, on peut citer le radical phényle. En outre, ces cycles peuvent être substitués en particulier par un substituant tel que A ou R. De plus R₁ et R₂ peuvent former un hétérocycle avec l'azote auquel ils sont liés, comportant de 4 à 7 atomes et mieux de 5 à 6 atomes et contenant de 1 à 3 hétéroatomes choisis parmi O, N, S.

Pour R₄, on peut utiliser comme hétérocycle, le cycle pyridine, pipéridine, morpholine, pyrrole, furanne, thiophène, imidazole, oxazole, thiazole, pyrazole, pyrimidine ou pyrazine.

De plus, ces cycles (ou hétérocycles) peuvent être seuls ou accolés à un autre cycle de même structure chimique ou non, et former ainsi des cycles condensés. Comme cycles condensés on peut citer le radical naphtyle, benzofuranne, benzothiophène, indole.

Par "radical alkyle", on entend au sens de l'invention un radical hydrocarboné qui peut être linéaire ou ramifié, saturé ou insaturé. En particulier, le radical alkyle comporte de 1 à 10 atomes de carbone.

Comme exemple de radical alkyle utilisable dans l'invention, on peut citer les radicaux méthyle, éthyle, isopropyle, n-butyle, terbutyle, n-hexyle, éthyl-2-hexyle, éthylène, propylène.

Comme atome d'halogène, on peut utiliser les atomes de chlore, de fluor ou de brome, et mieux les atomes de fluor et de chlore.

Tel que décrit ici, les composés de formule (I) sont sous forme isolée, c'est-à-dire non polymérique.

Selon un mode particulier de réalisation décrit ici, le composé pyrazol-carboxamide présente la formule (II) suivante ou un des ses sels : 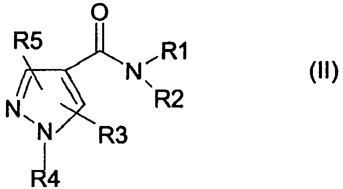 dans laquelle :
- R₁ et R₂, sont choisis indépendamment parmi :
   - l'hydrogène,
   - les radicaux alkyle en C₁-C₂₀, saturés ou insaturés, linéaires ou ramifiés éventuellement substitués par au moins un substituant T₁, R₁ et R₂ pouvant en outre former un hétérocycle de 4 à 7 atomes avec l'azote auquel ils sont liés ,;
- R₃ et R₅ sont choisis indépendamment parmi :
   - l'hydrogène,
   - A,
   - les halogènes,
   - les groupes OR₆, SR₆, NR₆R'₆, CN, CF₃, COOR₆,
   - les cycles saturés ou insaturés, de 4 à 7 atomes, contenant éventuellement au moins un hétéroatome parmi O, N, S, ces cycles pouvant éventuellement être accolés et/ou être substitués par au moins un substituant T₃ choisi parmi A et R ;
- R₄ est choisi parmi :
   - l'hydrogène,
   - A,
   - les groupes COR₆, COOR₆,
   - les cycles hydrocarbonés saturés ou insaturés, de 4 à 7 atomes, ces cycles pouvant éventuellement être substitués par au moins un substituant T₄ choisi parmi A et R ;
- R₆ et R'₆ sont choisis parmi :
   - l'hydrogène,
   - les radicaux alkyle en C₁-C₂₀, saturés ou insaturés, linéaires ou ramifiés éventuellement substitués par au moins un substituant R',
   - les cycles saturés ou insaturés, de 4 à 7 atomes, contenant éventuellement au moins un hétéroatome parmi O, N, S, ces cycles pouvant éventuellement être accolés et/ou être substitués par au moins un substituant R ;
- R est choisi parmi :
   - les radicaux alkyle en C₁-C₂₀, saturés ou insaturés, linéaires ou ramifiés,
   - les halogènes,
   - les groupes OR₇, SR₇, NR₇R'₇, CN, CF₃, COOR₇ ;
- R' est choisi parmi :
   - les radicaux alkyle en C₁-C₂₀, saturés ou insaturés, linéaires ou ramifiés,
   - les halogènes,
   - les groupes OR₇, SR₇, NR₇R'₇, CN, CF₃, COOR₇,
   - les cycles saturés ou insaturés, de 4 à 7 atomes, contenant éventuellement au moins un hétéroatome parmi O, N, S, ces cycles pouvant éventuellement être accolés ;
- R₇ et R'₇, représentent indépendamment l'hydrogène ou un alkyle en C₁-C₂₀ saturé ou insaturé, linéaire ou ramifié ;
- A représente un radical alkyle en C₁-C₂₀, saturé ou insaturé, linéaire ou ramifié éventuellement substitué par au moins un substituant T₅ choisi parmi les halogènes, les groupes OR₇, SR₇, NR₇R'₇, CN, CF₃, COOR₇ et les cycles saturés ou insaturés de 4 à 7 atomes contenant éventuellement au moins un hétéroatome parmi O, N, S, ces cycles pouvant éventuellement être accolés et/ou être substitués par au moins un substituant R ;
- T₁ est choisi parmi OR₆, SR₆, NR₆R'₆, CN, CF₃, , COOR₆, les halogènes, les cycles saturés ou insaturés de 4 à 7 atomes contenant éventuellement au moins un hétéroatome parmi O, N, S, ces cycles pouvant éventuellement être accolés et être substitués par au moins un substituant R.

Selon un mode de réalisation décrit ici, l'un au moins des R₁ et R₂ représente un groupe radical alkyle saturé, en C₁-C₂₀ et mieux en C₁-C₁₀ substitué, par SR₆ ou OH. En particulier, R₆ représente un radical alkyle en C₁-C₂₀ et mieux en C₁-C₁₀ éventuellement substitué par un hétérocycle Hy de 4 à 7 atomes. Par exemple l'un au moins des R₁ et R₂ représentent un groupe représentent un groupe (CH₂)ₙSₙR₈ avec R₈ représentant OH ou -S-(CH₂)ₘR₉, avec R₉ représentant H ou Hy(CH₂)ₘHy, où n et m représentent chacun un entier allant de 1 à 20 et mieux de 1 à 10. En particulier, R₁ représente l'hydrogène et R₂ représente (CH₂)ₙS(CH₂)ₘR₉Hy, avec n valant 2 et m valant 1. Par exemple, Hy représente un hétérocycle à 5 atomes comportant par exemple comme hétéroatome l'oxygène, comme le furanne.

Avantageusement, l'un au moins des R₃ et R₅ représente CF₃. En particulier R₃ représente CF₃ et R₅ représente H.

Selon un mode de réalisation particulier, R₄ représente un cycle hydrocarboné comportant 5 à 6 atomes, en particulier insaturé et notamment un radical phényle éventuellement substitué par T₄ et par exemple par 4-COOH.

Selon un mode particulier de réalisation décrit ici, le composé pyrazol-carboxamide présente la formule (III) suivante ou l'un de ses sels : 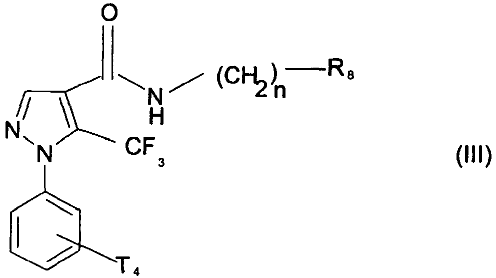 où R₈ représente OH ou -S-(CH₂)ₘ-R₉, avec R₉ représentant H ou Hy ; T4 représente H ou 4-COOH ; n et m représentent indépendamment un entier allant de 1 à 10 et mieux de 1 à 5 ; Hy représentant un hétérocycle notamment de 5 à 6 atomes.

Par sels de composé de formule (I), on entend ici, les sels organiques ou inorganiques d'un composé de formule (I).

Comme sels inorganiques utilisables décrits ici, on peut citer : les sels de sodium ou de potassium ainsi que les sels de zinc (Zn²⁺), de calcium (Ca²⁺), de cuivre (Cu²⁺), de fer (Fe²⁺), de strontium (Sr²⁺), de magnésium (Mg²⁺), de manganèse (Mn²⁺), d'ammonium ; les hydroxydes, les carbonates, les halogénures, les chlorures, les sulfates, les phosphates, les nitrates.

Les sels organiques utilisables décrits ici sont par exemple les sels de tri-éthanolamine, mono-éthanolamine, di-éthanolamine, hexadécylamine et N,N,N',N'-tétrakis-(hydroxy-propyl-2) éthylène diamine, tris-hydroxyméthylaminométhane.

Les composés de formule (I), salifiés ou non dont certains sont connus en tant que tels. Ils peuvent être fabriqués de façon connue et notamment comme décrit dans le document T.W. Waldrep et al., J. Agr. Food Chem., 1990, 38, 541-544. Ils se présentent sous forme de solide notamment pulvérulente.

Pour donner un ordre de grandeur, que décrit ici, le composé de formule (I) ou de l'un de ses sels ou mélange de composés de formule (I) et/ou de leur sel peut être utilisé en une quantité représentant de 10⁻³ % à 10 % du poids total de la composition et préférentiellement en une quantité représentant de 10⁻³ à 5 % et mieux de 10⁻²% à 2% du poids total de la composition, par exemple de 0,5 à 2 %.

La composition décrite dans la demande peut être à usage cosmétique ou pharmaceutique. Préférentiellement, la composition décrite dans la demande est à usage cosmétique. Aussi, la composition doit contenir un milieu physiologiquement acceptable non toxique et susceptible d'être appliqué sur la peau, y compris le cuir chevelu et les paupières, ou sur les fibres kératiniques d'êtres humains. Par "cosmétique", on entend ici une composition d'aspect, d'odeur et de toucher agréables.

Le composé de formule (I), salifié ou non, peut être utilisé dans une composition qui doit être ingérée, injectée ou appliquée sur la peau ou sur les fibres kératiniques (sur toute zone cutanée ou des fibres à traiter).

Tel que décrit ici, le composé de formule (I) peut être utilisé par la voie orale en une quantité de 0,1 à 300 mg par jour, 5 à 10mg/j.

Une composition préférée décrite dans la demande est une composition à usage cosmétique et en particulier d'application topique sur la peau et les fibres kératiniques, et plus spécialement sur le cuir chevelu, les cheveux et les cils.

Cette composition peut se présenter sous toutes formes galéniques connues adaptées au mode d'utilisation.

### COMPOSES 2-ALKYLIDENE AMINOOXYACETAMIDE:

Selon encore un autre aspect, des inhibiteurs de 15-PGDH utiles décrits dans la demande comprennent certains composés 2-alkylidèneaminooxy-acétamides et notamment certains thiophène ou furanne -aminooxy-acétamides, salifiés ou non. La demande décrit donc également l'utilisation d'au moins un composé 2-alkylidène aminooxy-acétamide de formule (I) ou d'un de ses sels : 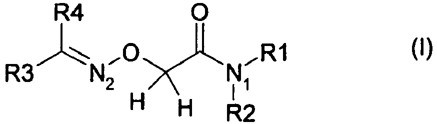 dans laquelle :
a) R₁ et R₂ sont choisis indépendamment parmi :
   *1)* les radicaux alkyle en C₁-C₂₀ linéaires ou ramifiés, saturés ou insaturés éventuellement substitués par au moins un substituant T₁ choisi parmi :
      ■ les halogènes,
      ■ les groupes CF₃, CN, OR, SR, NRR', NRC(=NR')NR"R"', COR, CSR, COOR, CONRR', NRCOR', NRCONR'R", SO₂NRR', NRSO₂R', SO₂R, SiRR'R",
      ■ les cycles saturés ou insaturés, de 4 à 7 atomes, contenant éventuellement au moins un hétéroatome pour former un hétérocycle Hy, ces cycles pouvant éventuellement être accolés, comporter une fonction carbonyle ou thiocarbonyle et/ou être substitués par au moins un substituant T₂,
   2) les cycles saturés ou insaturés, de 4 à 7 atomes, contenant éventuellement au moins un hétéroatome pour former un hétérocycle Hy, ces cycles pouvant éventuellement être accolés, comporter une fonction carbonyle ou thiocarbonyle et/ou être substitués par au moins un substituant T₃ choisi parmi :
      ■ les halogènes,
      ■ les groupes CF₃, CN, OR, SR, NRR', NRC(=NR')NR"R"', COR, CSR, COOR, CONRR', NRCOR', NRCONR'R", SO₂NRR', NRSO₂R', SO₂R, SiRR'R",
      ■ les radicaux alkyle en C₁-C₂₀ linéaires ou ramifiés, saturés ou insaturés éventuellement substitués par un au moins substituant T₁,
      ■ les cycles saturés ou insaturés, de 4 à 7 atomes, contenant éventuellement au moins un hétéroatome pour former un hétérocycle Hy, ces cycles pouvant éventuellement être accolés, comporter une fonction carbonyle ou thiocarbonyle et/ou être substitués par au moins un substituant T₂ ;
      à condition que dans le cas où R₁ et R₂ sont tous les deux des hétérocycles Hy, au moins un hétérocycle est relié à l'azote N₁ de la formule (I) par un carbone ;
   3) les groupes CN, C(=NR)R', C(=NR)NR'R", COR, CSR, COOR, CONRR' ;
   4) R₁ peut aussi être un atome d'hydrogène ou un groupe SO₂R ou SO₂NRR' ;
   5) R₁ et R₂ peuvent aussi former un hétérocycle Hy, éventuellement substitué par au moins un substituant T₂ et éventuellement accolé à un radical aryle ou à un cycle carboné, saturé ou insaturé de 4 à 7 atomes et pouvant contenir une fonction carbonyle ou thiocarbonyle, ou accolé à un autre hétérocycle Hy ;
b) R₃ et R₄ sont choisis indépendamment parmi :
   *1)* les radicaux alkyle en C₁-C₂₀ linéaires ou ramifiés ou les cycles carbonés de 4 à 7 atomes, pouvant contenir une fonction carbonyle ou thiocarbonyle, ces groupes alkyle ou ces cycles étant saturés ou insaturés et éventuellement substitués par au moins un substituant T₄ choisi parmi :
      ■ les halogènes,
      ■ les groupes CF₃, CN, OR, SR, NRR', NRC(=NR')NR"R"', COR, CSR, COOR, CONRR', NRCOR', NRCONR'R", SO₂NRR', NRSO₂R', SO₂R, SiRR'R",
      ■ les radicaux aryle ou les hétérocycles Hy, ces radicaux et hétérocycles étant éventuellement substitués par un au moins un substituant T₂ et éventuellement accolés à un radical aryle ou à un cycle carboné, saturé ou insaturé de 4 à 7 atomes et pouvant contenir une fonction carbonyle ou thiocarbonyle, ou accolés à un autre hétérocycle Hy ;
   2) les radicaux aryle ou les hétérocycles Hy, ces radicaux aryle et ces hétérocycles étant éventuellement accolés à un radical aryle ou à un cycle carboné, saturé ou insaturé de 4 à 7 atomes et pouvant contenir une fonction carbonyle ou thiocarbonyle, ou accolés à un hétérocycle Hy, ces radicaux aryle, ce cycle carboné ou ces hétérocycles Hy étant éventuellement substitués par au moins un substituant T₃ ;
   3) les groupes CF₃, CN, OR, SR, NRR', NRC(=NR')NR"R'", COR, CSR, COOR, CH₂COOR, CONRR', NRCOR', NRCONR'R", SO₂NRR', NRSO₂R', SO₂R, SiRR'R" ;
   *4)* R₃ et R₄ peuvent aussi être un atome d'hydrogène ;
c) R, R', R" et R"' identiques ou différents désignent un des groupes suivants :
   - un hydrogène,
   - un radical alkyle en C₁-C₂₀ linéaire ou ramifié, saturé ou insaturé, éventuellement substitué par au moins un substituant T₂,
   - les cycles saturés ou insaturés, de 4 à 7 atomes, contenant éventuellement au moins un hétéroatome pour former un hétérocycle Hy, ces cycles pouvant éventuellement être accolés, comporter une fonction carbonyle ou thiocarbonyle et/ou être substitués par au moins un substituant T₂ ;
d) T₂ représente :
   - un radical alkyle en C₁-C₂₀ linéaire ou ramifié, saturé ou insaturé,
   - un halogène,
   - un groupe choisi parmi CN, CF₃, OR₅, SR₅, NR₅R₆, NR₅C(=NR₆)NR₇R₆, COR₅, CSR₅, COOR₅, CH₂COOR₅, CONR₅R₆, NR₅COR₆, NR₅CONR₆R₇, SO₂NR₅R₆, NRSO₂R₅, SO₂R₅, SiR₅R₆R₇ dans lesquels R₅, R₆, R₇ et R₈ identiques ou différents désignent l'hydrogène ou un radical alkyle en C₁-C₂₀ linéaire ou ramifié, saturé ou insaturé,
   - un radical aryle ou un hétérocycle Hy éventuellement accolé à un radical aryle ou à un cycle carboné, saturé ou insaturé de 4 à 7 atomes, ou accolé à un autre hétérocycle Hy ;
e) Hy représente un hétérocycle saturé ou insaturé de 4 à 7 atomes pouvant contenir de 1 à 4 hétéroatomes choisis parmi N, O et S, et/ou comporter une fonction carbonyle ou thiocarbonyle.

L'invention s'applique aussi aux fibres kératiniques des mammifères de l'espèce animale (chien, cheval, mouton ou chat par exemple).

La demande décrit encore l'utilisation cosmétique d'au moins un oxy-acétamide de formule (I) ou d'un de ses sels dans une composition cosmétique de soin et/ou de maquillage des fibres kératiniques humaines pour induire et/ou stimuler leur pigmentation, freiner leur blanchiment ainsi que l'utilisation d'au moins un composé de formule (I) ou de l'un de ses sels pour la préparation d'une composition de soin ou de traitement des fibres kératiniques humaines, destinée à induire et/ou stimuler la pigmentation des fibres et/ou freiner leur blanchiment.

Les fibres kératiniques humaines auxquelles s'applique l'invention sont notamment les cheveux, les sourcils, les cils, les poils de barbe, de moustache et les poils pubiens. Plus spécialement, l'invention s'applique aux cheveux et/ou aux cils humains.

Aussi, la demande décrit encore une composition de soin ou de maquillage des fibres kératiniques, notamment une composition de soin capillaire ou de mascara, à application topique contenant un milieu physiologiquement acceptable et une quantité efficace d'au moins un composé de formule (I) ou de l'un de ces sels, tel que décrit précédemment.

La demande décrit encore l'utilisation notamment cosmétique d'au moins un composé 2-alkylidèneaminooxy-acétamide de formule (I) ou de l'un de ses sels, tel que défini précédemment, comme agent pour induire et/ou stimuler la pigmentation des fibres kératiniques notamment des êtres humains et/ou de la peau.
Dans ce qui suit, et sauf mention exprès, l'emploi du terme composé de formule (I) doit être compris comme signifiant aussi bien le composé de formule (I) sous forme, neutre, acide ou basique que sous forme de sels.
"Au moins un" selon l'invention signifie un ou plusieurs (2, 3 ou plus). En particulier, la composition peut contenir un ou plusieurs composés de formule (I). Ce ou ces composés peuvent être des isomères cis ou trans ou Z ou E ou un mélange d'isomères cis/trans ou Z/E. Ils peuvent aussi être sous forme tautomère. Ce ou ces composés peuvent être des énantiomères et/ou des diastéréoisomères ou un mélange de ces isomères, en particulier un mélange racémique.

Par "radical alkyle" on entend ici un radical hydrocarboné qui peut être linéaire ou ramifié et saturé ou insaturé. De préférence, le radical alkyle comporte de 1 à 10 atomes de carbone.

Tel que décrit ici, les cycles employés pour R₁, R₂, R₃, R₅, R₆, R₇, R₈, R, R', R", R"', T₁, T₂ et T₃ comportent de 4 à 7 atomes et mieux de 5 à 6 atomes. Ils peuvent être saturés ou insaturés et comporter éventuellement un ou plusieurs hétéroatomes tels que S, N, O ou leurs associations. Les cycles peuvent, en outre, comporter une ou plusieurs fonctions carbonyle ou thiocarbonyle ou les deux, le carbone de ces fonctions faisant partie de l'hétérocycle. Comme cycles carbonés saturés utilisables on peut citer le radical cyclobutyle, cyclopentyle, cyclohexyle ou cycloheptyle. Comme hétérocycle Hy, on peut citer les cycles pyridine, pipéridine, morpholine, pyrrole, furanne, thiophène, imidazole, oxazole, thiazole, pyrazole, pyrimidine, pipérazine, pyrazine, pyridazine, triazine, pyrrolidine, thiazolidine. Comme cycles carbonés insaturés, on peut citer le cycle cyclohexényle ou phényle et comme radicaux aryle, on peut citer le radical phényle ou naphtyle. En outre, ces cycles peuvent être substitués en particulier par un substituant T₂.

De plus, ces cycles peuvent être seuls ou accolés à un autre cycle de même structure chimique ou non et former ainsi des cycles condensés.

Pour l'ensemble des définitions de R₁ à R₈ et de R, R', R" et R"', certains carbones des cycles carbonés ou des hétérocycles, saturés ou insaturés, à 4, 5, 6 ou 7 atomes peuvent également faire partie d'une fonction carbonyle ou thiocarbonyle comme par exemple :

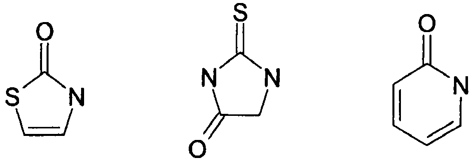

Lorsque R₁ et R₂ forment un hétérocycle, cet hétérocycle peut être, par exemple, le cycle pyrrolidine, pyrrole, imidazole, triazole, pipéridine, morpholine, pipérazine ou tétrazole.

Comme exemple de radical alkyle utilisable décrit ici, on peut citer les radicaux méthyle, éthyle, isopropyle, n-butyle, *ter*-butyle, n-hexyle, éthyle-2-hexyle ou encore éthylène ou propylène. Ce radical peut être éventuellement substitué par un ou par plusieurs substituants T₄ par exemple choisi parmi OR, COOR.

Comme atome d'halogène, on peut utiliser les atomes de chlore, de fluor ou de brome, et mieux les atomes de fluor et de chlore.

Tels que décrits ici, les composés de formule (I) sont sous forme isolée, c'est-à-dire non polymérique.

Selon un mode de réalisation, l'un au moins des R₃ et R₄ représente un groupe radical alkyle saturé, en C₁-C₂₀ et mieux en C₁-C₁₀. comme par exemple le radical méthyle ou éthyle ou un hétérocycle Hy. Par exemple l'un au moins des R₃ et R₄ représente un radical alkyle saturé en C₁-C₂₀ et l'autre un hétérocycle Hy. En particulier, R₃ représente le radical méthyle ou éthyle et R₄ représente un hétérocycle à 5 atomes. Par exemple, Hy représente un hétérocycle à 5 atomes comportant par exemple comme hétéroatome le soufre ou l'oxygène, comme le thiophène ou le furanne.

Avantageusement, l'un au moins des R₁ et R₂ représente un atome d'hydrogène, un cycle carboné saturé ou non et notamment un radical aryle. En particulier R₂ représente H et R₁ représente un radical phényle éventuellement substitué par un alkyle ou un alkoxy comme le groupe méthoxy.

Par sels de composé de formule (I), on entend ici, les sels organiques ou inorganiques, simples ou doubles, d'un composé de formule (I).

Comme sels inorganiques utilisables décrits ici on peut citer : les sels de sodium ou de potassium ainsi que les sels de zinc (Zn²⁺), de calcium (Ca²⁺), de cuivre (Cu²⁺), de fer (Fe²⁺), de strontium (Sr²⁺), de magnésium (Mg²⁺), d'ammonium et de manganèse (Mn²⁺) ; les hydroxydes, les carbonates, les halogénures, les sulfates, les nitrates, les phosphates.

Les sels organiques utilisables décrits ici sont par exemple les sels de tri-éthanolamine, mono-éthanolamine, di-éthanolamine, hexadécylamine et N,N,N',N'-tétrakis-(hydroxy-propyl-2) éthylène diamine, tris-hydroxyméthylaminométhane.

Les composés de formule (I), salifiés ou non sont connus en tant que tels et peuvent être fabriqués de façon connue. Par exemple, la synthèse peut être réalisée en trois étapes. La condensation d'une cétone sur le chlorhydrate d'hydroxylamine donne une oxime. Celle-ci peut être alkylée par le sel de sodium de l'acide 2-chloroacétique. L'acide formé est alors transformé en amide via la formation d'un chlorure d'acide et réaction de ce dernier avec une amine. Une telle préparation est décrite par A. Buzas et al., Chimie Thérapeutique, 1972, 2, 140-142.

Pour donner un ordre de grandeur, tel que décrit ici, le composé de formule (I) ou de l'un de ses sels ou un mélange de composés de formule (I) et/ou de leurs sels peut être utilisé en une quantité représentant de 10⁻³% à 10% du poids total de la composition et préférentiellement en une quantité représentant de 10⁻³ à 5 % et mieux de 10⁻²% à 2 % du poids total de la composition, par exemple de 0,5 à 2 %.

Tel que décrit ici, le composé de formule (I) peut être utilisé par la voie orale en une quantité de 0,1 à 300 mg par jour, 5 à 10mg/j.

### COMPOSES 2-THIOACETAMIDE:

Selon un autre mode de réalisation, l'inhibiteur de 15-PGDH utile décrits dans la demande comprend au moins un composé 2-thioacétamide de formule (I) ou l'un de ses sels : 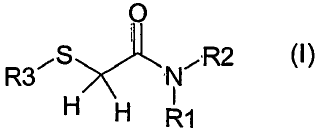 dans laquelle :
a) R₁ et R₂ représentent indépendamment :
   1) un atome d'hydrogène,
   2) un radical alkyle en C₁-C₂₀ éventuellement substitué par au moins un substituant A₁,
   3) un cycle hydrocarboné C₁, éventuellement accolé à un cycle C₂ contenant éventuellement au moins un hétéroatome pour former un hétérocycle Hy₁, ces cycles C₁ et C₂ pouvant comporter au moins une fonction carbonyle ou thiocarbonyle et être substitués par au moins un substituant A₃,
   4) un hétérocycle Hy₂ éventuellement accolé à un cycle C₂ contenant éventuellement au moins un hétéroatome pour former un hétérocycle Hy₃, ces cycles Hy₂ et C₂ pouvant comporter une fonction carbonyle ou thiocarbonyle et être substitués par au moins un substituant A₃, ou
   5) un groupe C(=NR)R', C(=NR)NR'R", COR, CSR, COOR, CONRR', SO₂R ou SO₂NRR' ;
b) R₃ représente :
   1) un radical alkyle en C₁-C₂₀ éventuellement substitué par au moins un substituant A₂,
   2) un cycle hydrocarboné C₃, éventuellement accolé à un cycle C₄ contenant éventuellement au moins un hétéroatome pour former un hétérocycle Hy₃, ces cycles C₃ et C₄ pouvant comporter au moins une fonction carbonyle ou thiocarbonyle et être substitués par au moins un substituant A₃,
   3) un hétérocycle Hy₄ représentant un cycle pyrrole, furanne, thiophène ou pyrazole, éventuellement accolé à un cycle C₅ représentant un cycle phényle, pyridine ou pyrimidine, ces deux cycles Hy₄ et C₅ pouvant être substitués par au moins un substituant A₃,
   4) un cycle pyridine éventuellement accolé à un cycle C₂ contenant éventuellement au moins un hétéroatome pour former un hétérocycle Hy₁, ces cycles pyridine et C₂ pouvant comporter au moins une fonction carbonyle ou thiocarbonyle et être substitués par au moins un substituant A₄,
   5) un hétérocycle Hy₅ différent de Hy₄ et du cycle pyridine, éventuellement accolé à un cycle C₂ contenant éventuellement au moins un hétéroatome pour former un hétérocycle Hy₁, ces cycles Hy₅ et C₂ pouvant comporter au moins une fonction carbonyle ou thiocarbonyle et être substitués par au moins un substituant A₃, ou
   6) un groupe C(=NR)R', C(=NR)NR'R", COR, CSR, COOR ou CONRR' ;
c) A₁ représente :
   1) un halogène,
   2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
   3) un cycle hydrocarboné C₆, éventuellement accolé à un cycle C₇ contenant éventuellement au moins un hétéroatome pour former un hétérocycle Hy₆, ces cycles C₆ et C₇ pouvant comporter au moins une fonction carbonyle ou thiocarbonyle et être substitués par au moins un substituant A₅,
   4) un hétérocycle Hy₇ éventuellement accolé à un cycle C₇ contenant éventuellement au moins un hétéroatome pour former un hétérocycle Hy₆, ces cycles Hy₇ et C₇ pouvant comporter au moins une fonction carbonyle ou thiocarbonyle et être substitués par au moins un substituant A₅ ;
d) A₂ représente :
   1) un halogène,
   2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R", ou
   3) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
e) A₃ représente :
   1) A₂ , ou
   2) un groupe alkyle en C₁-C₂₀ éventuellement substitué par au moins un substituant A₅;
f) A₄ représente :
   1) un halogène,
   2) un groupe CF₃, CN, OR, SR, NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
   3) un groupe alkyle en C₁-C₂₀ éventuellement substitué par au moins un substituant A₅,
   4) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
g) A₅ représente :
   1) un halogène,
   2) un groupe CF₃, CN, OR₅, SR₅, NR₅R₅', NR₅C(=NR₅')NR₅"R₅"', COR₅, CSR₅, COOR₆, CONR₅R₅', CSNR₅R₅', NR₅CSR₅', NR₅CSNR₅'R₅", NR₆COR₅', NR₅CONR₅'R₅", SO₂NR₅R₅', NR₅SO₂R₅', SO₂R₅ ou SiR₅R₅'R₅", R₅, R₅', R₅" étant un atome d'hydrogène ou un groupe alkyle en C₁-C₂₀,
   3) un groupe alkyle en C₁-C₂₀, ou
   4) un cycle C₁₀ éventuellement accolé à un autre cycle C₁₁, ces cycles contenant éventuellement au moins un hétéroatome pour former un hétérocycle Hy₉ et/ou comportant au moins une fonction carbonyle ou thiocarbonyle ;
h) R, R', R" et R"', identiques ou différents, représentent :
   1) un atome d'hydrogène,
   2) un groupe alkyle en C₁-C₂₀ éventuellement substitué par au moins un substituant A₅, ou
   3) un cycle C₁₂ éventuellement accolé à un autre cycle C₁₃, ces cycles contenant éventuellement au moins un hétéroatome pour former un hétérocycle Hy₁₀ et pouvant être substitués par au moins un substituant A₅ ;
i) Hy₁ à Hy₁₀ représentent indépendamment un hétérocycle pouvant contenir de 1 à 4 hétéroatomes choisis parmi N, O et S.

La demande décrit encore l'utilisation notamment cosmétique d'au moins un composé 2-thioacétamide de formule (I) ou d'un de ses sels, tel que défini précédemment, comme agent pour induire et/ou stimuler la pigmentation des fibres kératiniques humaines notamment les cils et les cheveux et/ou de la peau.

Dans la suite du texte, et sauf mention exprès, l'emploi du terme composé de formule (I) doit être compris comme signifiant aussi bien le composé de formule (I) sous forme acide ou basique, que l'un de ses sels. Il peut aussi être sous forme tautomère.

"Au moins un" selon l'invention signifie un ou plusieurs (2, 3 ou plus). En particulier, la composition peut contenir un ou plusieurs composés de formule (I). Ce ou ces composés peuvent être des isomères cis ou trans ou un mélange d'isomères cis/trans. Ils peuvent aussi être sous forme tautomère. Ce ou ces composés peuvent être des énantiomères et/ou des diastéréoisomères ou un mélange de ces isomères, en particulier un mélange racémique.

Par "hydrocarboné", on entend ici un groupement d'atomes d'hydrogène et de carbone.

Par "radical alkyle" on entend ici un radical hydrocarboné qui peut être linéaire ou ramifié et saturé ou insaturé. En particulier, le radical alkyle comporte de 1 à 20 atomes de carbone, de préférence de 1 à 10. Comme exemple de radical alkyle utilisable dans l'invention, on peut citer les radicaux méthyle, éthyle, isopropyle, n-butyle, *ter*-butyle, n-hexyle, éthyle-2-hexyle, éthylène, propylène.

Tels que décrits ici, les cycles C₁ à C₁₃, Hy₂, Hy₄ et Hy₅ de la formule (I) comportent de 3 à 7 atomes et mieux de 5 à 6 atomes et peuvent être saturés ou insaturés. En outre, les cycles C₂, C₄, C₇, C₈, C₉, C₁₀, C₁₁, C₁₂ et C₁₃ peuvent comporter éventuellement un ou plusieurs hétéroatomes tels que S, N, O ou leurs associations et former ainsi des hétérocycles. Hy₇ représente un hétérocycle pouvant comporter de 1 à 4 hétéroatomes choisis parmi N, S, O et leur association et comportant de 3 à 15 atomes et mieux de 5 à 6 atomes. Selon un mode particulier décrit ici, l'hétérocycle Hy₇ peut comporter jusqu'à 15 atomes comme un éther-couronne possédant 5 motifs - CH₂CH₂O-. Ces cycles peuvent en outre être accolés à un autre cycle de nature chimique identique ou différente. En outre, ces cycles peuvent être substitués en particulier par un substituant A₃ ou A₅.
Comme cycles hydrocarbonés saturés utilisables décrits ici, on peut citer le radical cyclopropyle, cyclobutyle, cyclopentyle, cyclohexyle ou cycloheptyle. Comme cycles hydrocarbonés insaturés, on peut citer le cycle cyclohexényle ou phényle. Comme cycles hydrocarbonés accolés, utilisables, on peut citer le radical naphtyle.

Tels que décrits ici, R₁ et/ou R₂ peuvent représenter un cycle hydrocarboné tel que défini ci-dessus, en particulier saturé. Tels que décrits ici R₁ et/ou R₂ peuvent aussi représenter un hétérocycle Hy₂ comportant de 3 à 7 atomes et mieux de 5 à 6 atomes, et comportant de 1 à 4 hétéroatomes choisi parmi N, O, S et leur association. Cet hétérocycle peut, en outre comporter, une ou plusieurs fonctions carbonyle ou thiocarbonyle. A titre d'exemple Hy₂ représente l'un des hétérocycles suivants : azétidine, pyrrole, dihydropyrrole, pyrrolidine, furanne, dihydrofuranne, tétrahydrofuranne, thiophène, dihydrothiophène, tétrahydrothiophène, imidazole, dihydro-imidazole, imidazolidine, dihydrothiazole, thiazolidine, dihydropyrazole, pyrazolidine, oxazole, dihydro-oxazole, oxazolidine, isoxazole, dihydro-isoxazole, isoxazolidine, isothiazole, dihydro-isothiazole, isothiazolidine, triazole, dihydrotriazole, triazolidine, oxadiazole, dihydro-oxadiazole, oxadiazolidine, thiadiazole, dihydrothiadiazole, thiadiazolidine, tétrazole, pyridine, dihydropyridine, tétrahydropyridine, pipéridine, pyranne, dihydropyranne, tétrahydropyranne, pyrimidine, dihydropyrimidine, tétrahydropyrimidine, pipérazine, pyridazine, pyrazine, triazine, morpholine, azépine, diazépine. De préférence, Hy₂ représente un cycle pypéridine. Cet hétérocycle Hy₂ peut, en outre, être accolé à un cycle C₂ de 4 à 7 atomes, saturé ou insaturé, C₂ contenant éventuellement au moins un hétéroatome choisi parmi O, N, S pour former un hétérocycle Hy₁. C₂ peut en outre comporter une ou plusieurs fonctions carbonyle ou thiocarbonyle. Comme exemple d'hétérocycle Hy₁ utilisable décrit ici, on peut citer les cycles pyridine, pipéridine, morpholine, pyrrole, furanne, thiophène, imidazole, oxazole, thiazole, pyrazole, pyrimidine, pipérazine, pyrazine, pyridazine, triazine, pyrrolidine, thiazolidine.

Tels que décrits ici, R₁ et/ou R₂ peuvent, en outre, représenter un radical alkyle dont l'un au moins des hydrogènes est substitué par un hétérocycle Hy₇. Cet hétérocycle peut comporter de 3 à 7 atomes, et mieux de 5 à 6 atomes, et comprendre de 1 à 4 hétéroatomes choisis parmi N, O, S et leur association. Selon un mode particulier décrit ici, cet hétérocycle Hy₇ peut comporter jusqu'à 15 atomes comme un éther couronne à 5 motifs -CH₂CH₂O-. Selon un autre mode, Hy₇ comporte une fonction carbonyle. Comme hétérocycle Hy₇ utilisable décrit ici, on peut citer les cycles azétidine, pyrrole, dihydropyrrole, pyrrolidine, furanne, dihydrofuranne, tétrahydrofuranne, thiophène, dihydrothiophène, tétrahydrothiophène, imidazole, dihydro-imidazole, imidazolidine, thiazole, dihydrothiazole, thiazolidine, pyrazole, dihydropyrazole, pyrazolidine, oxazole, dihydro-oxazole, oxazolidine, isoxazole, dihydro-isoxazole, isoxazolidine, isothiazole, dihydro-isothiazole, isothiazolidine, triazole, dihydrotriazole, triazolidine, oxadiazole, dihydro-oxadiazole, oxadiazolidine, thiadiazole, dihydrothiadiazole, thiadiazolidine, tétrazole, pyridine, dihydropyridine, tétrahydropyridine, pyranne, dihydropyranne, tétrahydropyranne, pyrimidine, dihydropyrimidine, tétrahydropyrimidine, pipérazine, pyridazine, pyrazine, triazine, morpholine, azépine, diazépine, éther couronne à 15 atomes et 5 motifs -CH₂CH₂O-, pyrrolidinone. De préférence, Hy₇ représente un des cycles pyrrole, oxyazole, pyridine, furanne, pyrrolidine, morpholine, tétrahydrofuranne, éther couronne à 15 atomes (5 motifs -CH₂CH₂O) ou pyrrolidinone.

Tel que décrit ici R₃ peut représenter un cycle hydrocarboné C₃ de 3 à 7 atomes de carbone, saturé ou insaturé, et mieux de 5 à 6 atomes de carbones, éventuellement accolé à un hétérocycle Hy₃. Cet hétérocycle Hy₃ peut comporter de 4 à 7 atomes et mieux de 5 à 6 atomes et comprendre de 1 à 4 hétéroatomes choisis parmi N, O, S et leur combinaison. En outre, cet hétérocycle Hy₃ peut comporter une ou plusieurs fonctions carbonyle ou thiocarbonyle.

Comme hétérocycle Hy₃ utilisable décrit ici on peut citer les cycles azétidine, pyrrole, dihydropyrrole, pyrrolidine, furanne, dihydrofuranne, tétrahydrofuranne, thiophène, dihydrothiophène, tétrahydrothiophène, imidazole, dihydro-imidazole, imidazolidine, dihydrothiazole, thiazolidine, pyrazole, dihydropyrazole, pyrazolidine, oxazole, dihydro-oxazole, oxazolidine, isoxazole, dihydro-isoxazole, isoxazolidine, isothiazole, dihydro-isothiazole, isothiazolidine, triazole, dihydrotriazole, triazolidine, oxadiazole, dihydro-oxadiazole, oxadiazolidine, thiadiazole, dihydrothiadiazole, thiadiazolidine, tétrazole, pyridine, dihydropyridine, tétrahydropyridine, pipéridine, pyranne, dihydropyranne, tétrahydropyranne, pyrimidine, dihydropyrimidine, tétrahydropyrimidine, pipérazine, pyridazine, pyrazine, triazine, morpholine, azépine, diazépine. De préférence, Hy₃ représente un des cycles pyrrole, pyridine, pyrimidine, imidazole, triazole, furanne, thiophène, oxazole, thiazole.

Selon l'invention, R₃ peut aussi représenter :
- un cycle pyridine éventuellement accolé à un cycle C₂ de nature chimique identique ou différente,
- un hétérocycle Hy₄ choisi parmi les cycles pyrrole, furanne, thiophène et pyrazole éventuellement accolé à un cycle phényle, pyridine ou pyrimidine,
- un autre hétérocycle Hy₅ comportant de 3 à 7 atomes, et mieux de 5 à 6 atomes, et de 1 à 4 hétéroatomes choisi parmi N, O, S et leur association. Cet hétérocycle Hy₅ peut, en outre, comporter une ou plusieurs fonctions carbonyle ou thiocarbonyle et être accolé à un autre cycle C₂ de nature chimique identique ou différente.

A titre d'exemple, l'hétérocycle Hy₅ est choisi parmi les cycles azétidine, dihydropyrrole, pyrrolidine, dihydrofuranne, tétrahydrofuranne, dihydrothiophène, tétrahydrothiophène, imidazole, dihydro-imidazole, imidazolidine, thiazole, dihydrothiazole, thiazolidine, dihydropyrazole, pyrazolidine, oxazole, dihydro-oxazole, oxazolidine, isoxazole, dihydro-isoxazole, isoxazolidine, isothiazole, dihydro-isothiazole, isothiazolidine, triazole, dihydrotriazole, triazolidine, oxadiazole, dihydro-oxadiazole, oxadiazolidine, thiadiazole, dihydrothiadiazole, thiadiazolidine, tétrazole, dihydropyridine, tétrahydropyridine, pipéridine, pyranne, dihydropyranne, tétrahydropyranne, pyrimidine, dihydropyrimidine, tétrahydropyrimidine, pipérazine, pyridazine, pyrazine, triazine, morpholine, azépine, diazépine, 2-benzothiazolyle, thiazolo[2,3-c][1,2,4]triazole. De préférence, Hy₅ représente un des cycles 2-benzothiazolyle, thiazolo[2,3-c][1,2,4]triazole, imidazole, thiazole, triazole, oxazole, pyrimidine, pyrazine, pyridazine.

Comme exemple d'hétérocycle Hy₁, Hy₆, Hy₈, Hy₉, Hy₁₀ utilisable décrit ici, on peut citer indépendamment les cycles azétidine, pyrrole, dihydropyrrole, pyrrolidine, furanne, dihydrofuranne, tétrahydrofuranne, thiophène, dihydrothiophène, tétrahydrothiophène, imidazole, dihydro-imidazole, imidazolidine, thiazole, dihydrothiazole, thiazolidine, pyrazole, dihydropyrazole, pyrazolidine, oxazole, dihydro-oxazole, oxazolidine, isoxazole, dihydro-isoxazole, isoxazolidine, isothiazole, dihydro-isothiazole, isothiazolidine, triazole, dihydrotriazole, triazolidine, oxadiazole, dihydro-oxadiazole, oxadiazolidine, thiadiazole, dihydrothiadiazole, thiadiazolidine, tétrazole, pyridine, dihydropyridine, tétrahydropyridine, pipéridine, pyranne, dihydropyranne, tétrahydropyranne, pyrimidine, dihydropyrimidine, tétrahydropyrimidine, pipérazine, pyridazine, pyrazine, triazine, morpholine, azépine, diazépine. De préférence, on utilise les cycles pyrrole, pyrrolidine, imidazole, furanne, pyrazole, pyridine, pyrimidine, triazole, pyrazine, pyridazine, pipéridine, pipérazine, morpholine.

Les hétérocycles de la formule (I) peuvent, en outre, être accolés à un cycle de nature chimique identique ou différente.

Comme hétérocycles accolés, utilisables décrits ici on peut citer les cycles purine, ptéridine. Comme hétérocycles accolés à un cycle hydrocarboné, utilisables décrits ici on peut citer, les radicaux benzofuranne, benzothiophène, benzothiazole, indole, benzimidazole, quinoline, isoquinoline, quinazoline.

Comme atome d'halogène utilisable décrit ici, on peut citer les atomes de chlore, de fluor ou de brome, et mieux les atomes de fluor et de chlore.

Tels que décrits ici, les composés de formule (I) sont sous forme isolée, c'est-à-dire non polymérique.
Selon un mode particulier de réalisation décrit ici, l'un au moins des R₁ et R₂ représente un atome d'hydrogène ; un radical alkyle en C₁-C₂₀ dont au moins un hydrogène est substitué par au moins un substituant A₁ ; un cycle hydrocarboné saturé ou non de 3 à 6 atomes de carbone, dont au moins un hydrogène est éventuellement substitué par le groupe halogène comme CF₃ ou des atomes d'halogènes et notamment par 1 ou 2 atomes de chlore ou de brome lorsque le cycle est un phényle. Ces groupe et atomes d'halogènes peuvent être situés en position para, méta ou ortho par rapport à l'atome d'azote portant R₁ et R₂. Le cycle hydrocarboné est notamment un radical cyclopentyle, cyclopropyle, cyclobutyle, cyclohexyle ou un radical aryle comme le phényle. En particulier R₁ représente H et R₂ représente un radical cyclopentyle, cyclopropyle, cyclobutyle, cyclohexyle ou un radical phényle substitué par un atome de brome ou 2 atomes de chlore.
Selon un autre mode de réalisation, R₁ représente H et R₂ représente un hétérocycle de 5 à 6 atomes, saturé ou insaturé, contenant de 1 à 4 hétéroatomes choisis parmi N, O, S et éventuellement substitué par au moins un radical alkyle, CF₃, OR, SR, NRR', COR, COOR, CONRR' ou NRCOR' avec R et R' représentant H ou alkyle. Par exemple R₂ représente un cycle pipéridine éventuellement substitué par au moins un radical alkyle en C₁-C₁₀, et notamment méthyle.

Selon un autre mode de réalisation, R₁ représente H et R₂ représente un alkyle en C₁-C₂₀, saturé, éventuellement substitué par au moins un substituant A₁. En particulier, A₁ représente un cycle de 5 à 6 atomes, saturé ou insaturé, contenant éventuellement de 1 à 4 hétéroatomes choisis parmi N, O, S, pouvant contenir au moins une fonction carbonyle et être éventuellement substitué par au moins un groupe choisi parmi alkyle, F, CF₃, OR, SR, NRR', COR, COOR, CONRR' ou NRCOR' avec R et R' représentant H ou alkyle ; un cycle éther-couronne à 15 atomes et 5 motifs -CH₂CH₂O- ; un groupe choisi parmi CF₃, OR, SR, NRR', COR, COOR, CONRR', NRCOR' ou SiRR'R" avec R, R' et R" représentant H ou alkyle.

Selon un autre mode de réalisation décrit ici, R₃ représente un phényle ou un hétérocycle de 5 à 6 atomes, substitué par un ou deux substituant A₃ ou accolé à un cycle hydrocarboné ou hétérocyclique de 5 à 6 atomes. En particulier, R₃ représente un hétérocycle comportant comme hétéroatome au moins un atome d'azote et éventuellement un atome de soufre, cet hétérocycle étant éventuellement accolé à un cycle phényle ou thiazole lui-même éventuellement accolé à un cycle hydrocarboné, notamment phényle, ou substitué par un groupe COR ou CN ou deux groupes respectivement COR et CN. En particulier, R₃ représente un groupe phényle, 2-benzothiazolyle, 2-pyridyle, 6-acétyl-nicotinonitrile, triazolyle ou 4a,8a-dihydro-benzo[4,5]thiazolo[2,3-c][1,2,4]triazolyle.

Selon un mode particulier de réalisation décrit ici, le composé 2-thioacétamide présente une des formules (II) et (III) suivantes : 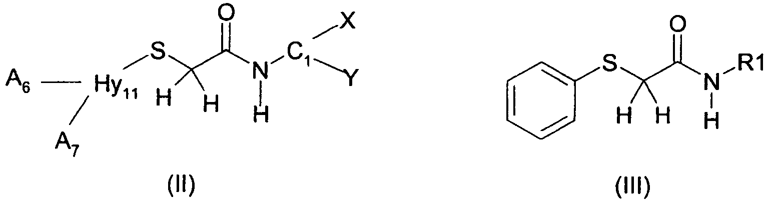 dans lesquelles X et Y représentent indépendamment un atome d'hydrogène ou un halogène ; C₁ représente un cycle hydrocarboné de 3 à 6 à atomes de carbone, saturé ou insaturé ; Hy₁₁ représente un hétérocycle de 5 à 6 atomes contenant au moins un hétéroatome choisi parmi N, S et leur combinaison ; A₆ et A₇ représentent indépendamment un substituant choisi parmi hydrogène, alkyle, COR, OR, SR, CN, COOR et les cycles C₈ à 5 ou 6 atomes, saturés ou non comportant éventuellement de 1 à 4 hétéroatomes choisis parmi S, N et leurs associations et/ou étant éventuellement accolés à un cycle C₉ hydrocarboné de 5 à 6 atomes de carbone ; R₁ représente un radical alkyle en C₁-C₂₀ linéaire ou ramifié, saturé ou insaturé, éventuellement substitué par au moins un substituant A₁, un cycle hydrocarboné ou un hétérocycle Hy₂ éventuellement substitué par au moins un substituant A₃, C₈, C₉, A₁, Hy₂ et A₃ ont la signification donnée précédemment.

Par sels de composé de formule (I), on entend ici, les sels organiques ou inorganiques d'un composé de formule (I).

Comme sels inorganiques utilisables décrits ici on peut citer : les sels de sodium ou de potassium ainsi que les sels de zinc (Zn²⁺), de calcium (Ca²⁺), de cuivre (Cu²⁺), de fer (Fe²⁺), de strontium (Sr²⁺), de magnésium (Mg²⁺) et de manganèse (Mn²⁺) ; les hydroxydes et les carbonates.

Les sels organiques utilisables décrits ici sont par exemple les sels de tri-éthanolamine, mono-éthanolamine, di-éthanotamine, hexadécylamine et N,N,N',N'-tétrakis-(hydroxy-propyl-2) éthylène diamine, tris-hydroxyméthylaminométhane.

Comme exemple de composés 2-thioacétamide de formule (I) utilisable décrits ici on peut citer les composés suivants :

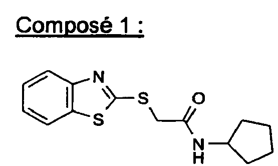

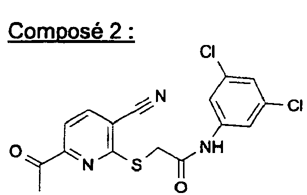

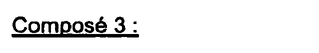

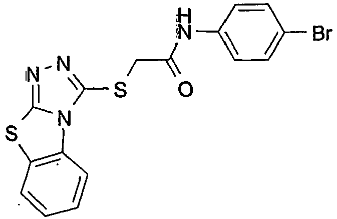

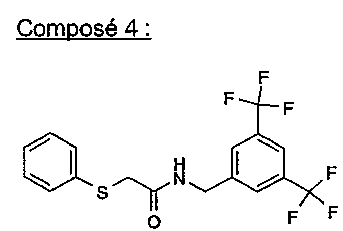

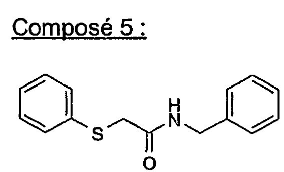

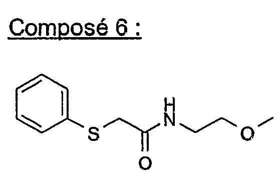

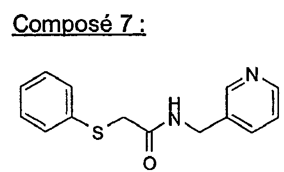

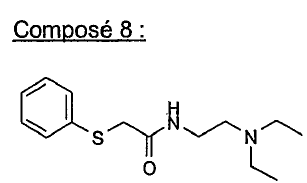

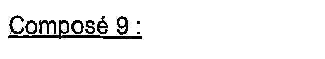

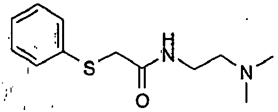

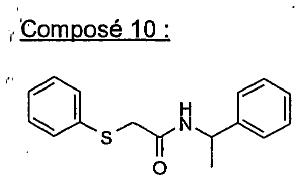

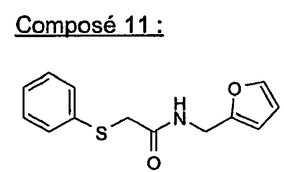

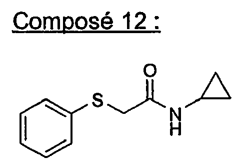

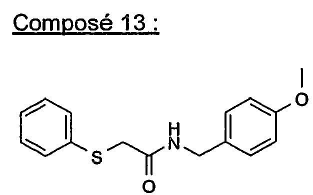

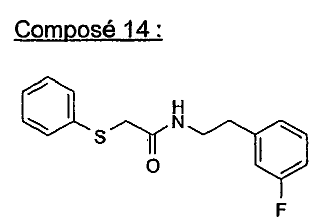

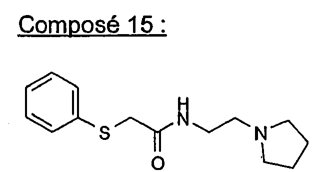

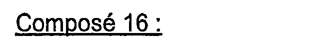

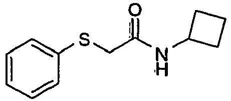

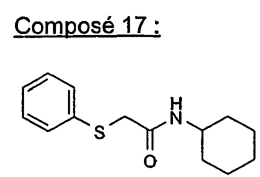

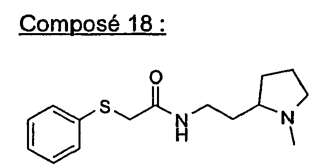

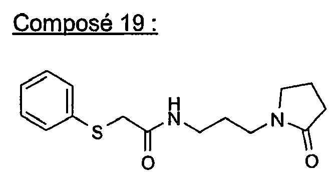

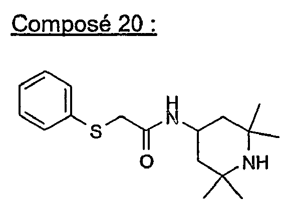

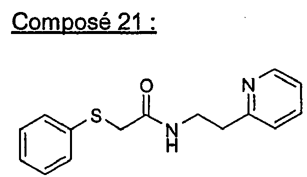

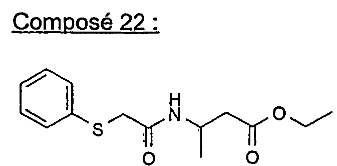

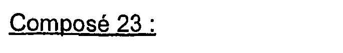

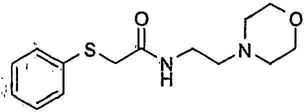

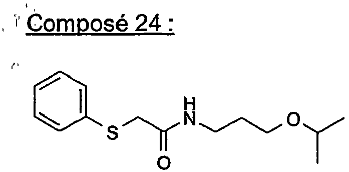

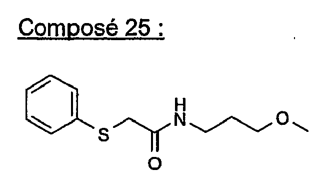

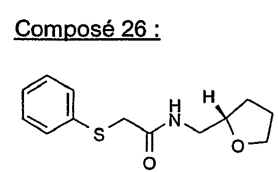

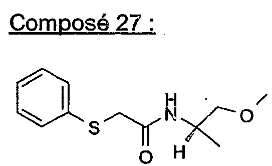

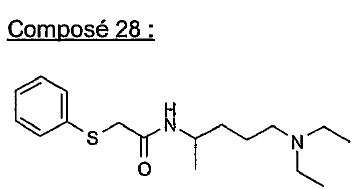

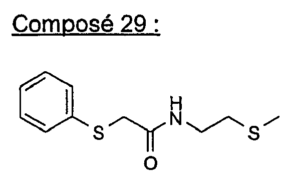

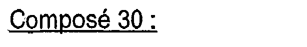

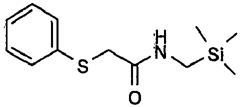

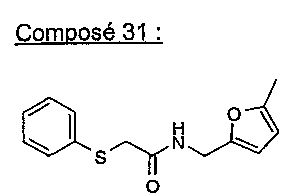

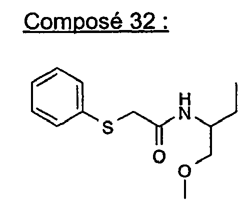

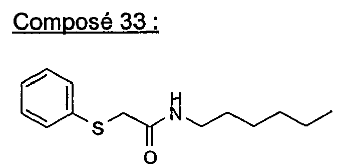

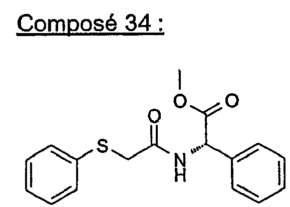

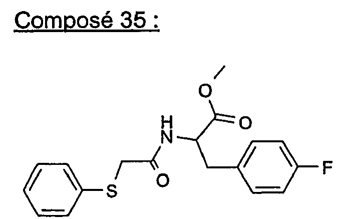

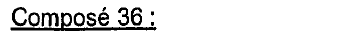

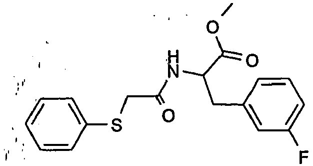

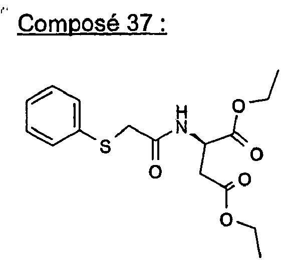

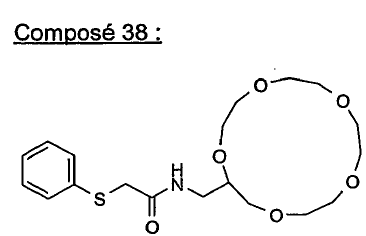

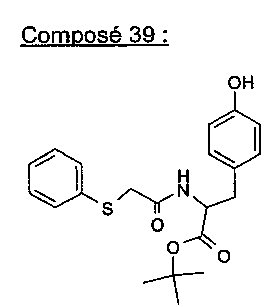

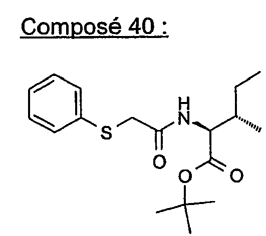

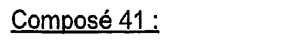

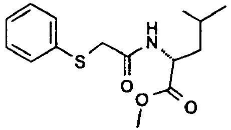

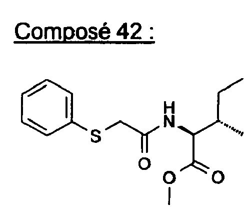

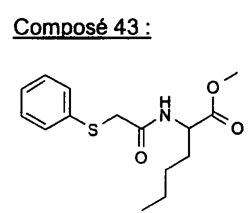

Les composés de formule (I), salifiés ou non peuvent être fabriqués de façon classique par condensation successive de deux molécules nucléophiles sur le chlorure de l'acide chloro-acétyle. Par exemple, la fonction amide est réalisée par condensation d'une amine sur le chlorure de chloro-acétyle en milieu basique. Le produit obtenu est lui-même condensé avec un thiolate.

Pour donner un ordre de grandeur, tel que décrit ici, le composé de formule (I) ou l'un de ses sels ou un mélange de composés de formule (I) et/ou de leurs sels peut être utilisé en une quantité représentant de 10⁻³ % à 5% du poids total de la composition et préférentiellement en une quantité représentant de 10⁻² % à 2% du poids total de la composition, par exemple de 0,5 à 2 %.

Le composé de formule (I), salifié ou non, peut être utilisé dans une composition qui doit être ingérée, injectée ou appliquée sur la peau ou sur les fibres kératiniques (sur toute zone cutanée ou des fibres à traiter).

Tel que que décrit ici, le composé de formule (I) ou un mélange de composés de formule (I) peut être utilisé par la voie orale en une quantité de 0,1 à 300 mg par jour, 5 à 10mg/j.

### COMPOSES AZOÏQUES:

Selon encore un autre mode de réalisation, l'inhibiteurs de 15-PGDH adapté décrit dans la présente demande comprend au moins un composé azoïque de formule (I) ou l'un de ses sels; 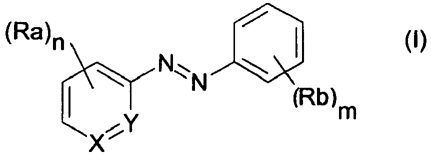 dans laquelle:
**1°) Ra et Rb** identiques ou différents sont choisis parmi :
   ***a)*** un atome d'hydrogène, un atome d'halogène, un groupe CF₃, CN, OR, SR, NRR', NRC(=NR')NR"R"', COR, CSR, COOR, CONRR', CH₂COOR, CSNRR', NRCSR', NRCSNR'R", NRCOR', NRCONR'R", SO₂NRR', NRSO₂R', NRSO₂NR'R", SO₂R, NO₂,
   ***b)*** un radical alkyle en C₁-C₁₀, linéaire ou ramifié, saturé ou non, éventuellement substitué par au moins un substituant A₁ ou par les cycles C¹ saturés ou insaturés, comportant de 3 à 7 atomes et/ou incluant éventuellement au moins un hétéroatome et/ou étant éventuellement substitués par au moins un substituant A₂ et/ou éventuellement accolés à au moins un cycle C³ incluant éventuellement au moins un hétéroatome (pour couvrir les cycles condensés) ou
   ***c)*** un cycle aromatique C² incluant éventuellement au moins un hétéroatome et/ou étant éventuellement substitué par au moins un substituant A₂ et/ou éventuellement accolé à un cycle C³ incluant éventuellement au moins un hétéroatome (pour couvrir les cycles condensés) ;
**2°) X et Y** identiques ou différents représentent -CRc ou un atome d'azote éventuellement sous forme NO ou quaternisé, Rc ayant la signification de Ra ou Rb, à condition que X et Y ne représentent pas simultanément un atome d'azote sous forme NO ou quaternisé ;
**3°) n et m** représentent des entiers allant de 0 à 5 ;
   - où R, R', R" et R"' identiques ou différents désignent :
      - l'atome d'hydrogène ou un radical alkyle en C₁-C₁₀, linéaire ou ramifié, saturé ou non,
      - un cycle aromatique C² incluant éventuellement au moins un hétéroatome, éventuellement substitué par au moins un substituant A₂ ;
   - où A₁ est choisi parmi un atome d'halogène, un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', NRSO₂NR'R", SO₂R, NO₂ ;
   - où A₂ est choisi parmi les radicaux alkyle en C₁C₁₀, linéaires ou ramifiés, OR₁, SR₁, NR₁R'₁, COOR₁, COR₁, CH₂COOR₁, avec R₁ et R'₁ identiques ou différents désignant l'hydrogène, un radical alkyle en C₁-C₁₀, linéaire ou ramifié, saturé ou non ;
   - où l'hétéroatome est choisi parmi N, O, S ou Se.

Dans la suite du texte, et sauf mention exprès, l'emploi du terme composé de formule (I) doit être compris comme signifiant aussi bien le composé de formule (I), que l'un de ses sels.

"Au moins un" selon l'invention signifie un ou plusieurs (2, 3 ou plus). En particulier, la composition peut contenir un ou plusieurs composés de formule (I). Ce ou ces composés peuvent être des isomères cis ou trans ou un mélange d'isomères cis/trans.

Par "radical alkyle" on entend ici un radical hydrocarboné qui peut être linéaire ou ramifié et saturé ou insaturé. En particulier, le radical alkyle comporte de 1 à 10 atomes de carbone et notamment de 1 à 5 atomes. Comme exemple de radical alkyle utilisable décrit ici, on peut citer les radicaux méthyle, éthyle, isopropyle, tertio-butyle, isopropyle.

Comme atome d'halogène, on peut utiliser les atomes de chlore, de fluor ou de brome, et mieux les atomes de chlore et de fluor.

Tels que décrits ici, les cycles C¹, C² et C³ de la formule (I) comportent de 3 à 7 atomes et mieux de 5 à 6 atomes et peuvent comporter un ou plusieurs hétéroatomes tels que S, N, O, Se ou leurs associations et former ainsi des hétérocycles Hy. Les atomes d'hydrogène de ces cycles peuvent, en outre, être en partie substitués par un substituant A₂. De plus les cycles C₁ et C₃ peuvent être saturés ou non.

Comme cycles hydrocarbonés saturés utilisables décrits ici on peut citer le radical cyclopropyle, cyclobutyle, cyclopentyle, cyclohexyle ou cycloheptyle. Comme cycles hydrocarbonés insaturés, on peut citer le radical cyclohexényle ou phényle.

Tels que décrits ici, C¹ et/ou C² et/ou C³ peuvent représenter un hétérocycle Hy comportant de 3 à 7 atomes et mieux de 5 à 6 atomes, et incluant de 1 à 4 hétéroatomes choisis parmi N, O, S, Se et leurs associations, et mieux choisis parmi N, O et leurs associations. A titre d'exemple d'hétérocycle saturé utilisable décrit ici, on peut citer les hétérocycles suivants : azétidine, pyrrolidine, tétrahydrofuranne, tétrahydrothiophène, imidazolidine, thiazolidine, pyrazolidine, oxazolidine, isoxazolidine, isothiazole, dihydro-isothiazole, isothiazolidine, triazolidine, oxadiazolidine, thiadiazolidine, tétrahydropyridine, pipéridine, tétrahydropyranne, tétrahydropyrimidine, pipérazine, hexahydrotriazines, morpholine.

A titre d'exemple d'hétérocycle insaturé (ou aromatique), on peut utiliser les hétérocycles suivants : pyrrole, dihydropyrrole, furanne, dihydrofuranne, thiophène, dihydrothiophène, imidazole, dihydro-imidazole, dihydrothiazole, dihydropyrazole, oxazole, dihydro-oxazole, isoxazole, dihydro-isoxazole, triazole, dihydrotriazole, oxadiazole, dihydro-oxadiazole, thiadiazole, dihydrothiadiazole, tétrazole, pyridine, dihydropyridine, pyranne, dihydropyranne, pyrimidine, dihydropyrimidine, pyridazine, pyrazine, pyrazine, diazépine, triazine.

En outre, les cycles C² et les cycles C³ peuvent formés des (hétéro)cydes condensés, saturés ou non et par exemple des cycles naphtalène, tétrahydronaphtalène, quinoléine, isoquinoléine, indole, benzimidazole, benzothiophène, benzofuranne, purine ou encore ptéridine ou carbazole.

Tels que décrits ici, les composés de formule (I) sont sous forme isolée, c'est-à-dire non polymérique. En outre, les substituants Rₐ et R_{b}, peuvent être situés en toute position du cycle phényle les portant.

Tels que décrits ici, les Rₐ et R_{b} portés par un même cycle peuvent être identiques ou différents. De même les substituants A₁ et A₂ portés respectivement par un même radical alkyle ou un même cycle peuvent être identiques ou différents.

De préférence, n est un entier représentant 0, 1, 2, 3 ou 5. Par ailleurs, m est un entier valant notamment 0,1 ou 2.

Avantageusement, l'un au moins des Rₐ et R_{b} représentent un groupe COOR, OR, NO₂, CF₃, SO₂NRR', CH₂COOR, un radical alkyle en C₁-C₅, saturé ou non, tel que méthyle, éthyle ou allyle, éventuellement substitué par un hétérocycle morpholine ou un groupe COOH, avec R et R' ayant la signification indiquée précédemment. En particulier R et/ou R' représentent un atome d'hydrogène, un radical méthyle ou un groupe pyridine.

En outre, X et Y représentent indépendamment un atome d'azote ou mieux un groupe CRc avec Rc représentant notamment un atome d'hydrogène, NO₂, OH, un groupe COOR, un radical alkyle en C₁-C₅, saturé ou non, tel que méthyle ou allyle, où R représente H, méthyle ou éthyle.

Par sels de composé de formule (I), on entend ici, les sels organiques ou inorganiques d'un composé de formule (I).

Comme sels inorganiques utilisables décrits ici on peut citer les sels de sodium ou de potassium ainsi que les sels de zinc (Zn²⁺), de calcium (Ca²⁺), de cuivre (Cu²⁺), de fer (Fe²⁺), de strontium (Sr²⁺), de magnésium (Mg²⁺) et de manganèse (Mn²⁺), d'ammonium ; les hydroxydes, les carbonates, les halogénures (comme les chlorures), les sulfates, les nitrates, les phosphates. De préférence, le sel est un sel de sodium.

Les sels organiques utilisables décrits ici sont par exemple les sels tri-éthanolamine, mono-éthanolamine, diéthanolamine, hexadécylamine et N,N,N',N'-tétrakis-(hydroxy-propyl-2) éthylène diamine.

Comme exemple de composé azoïque de formule (I) utilisable décrit ici on peut citer les composés suivants :

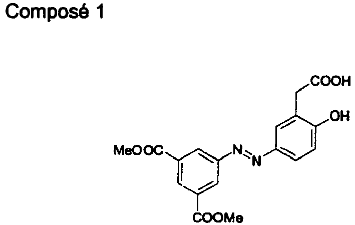

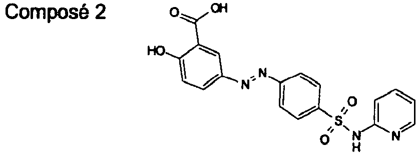

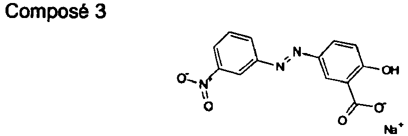

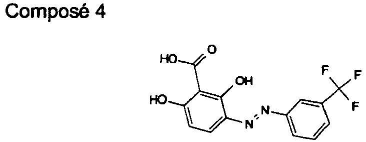

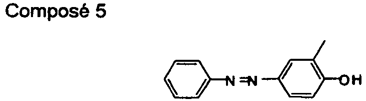

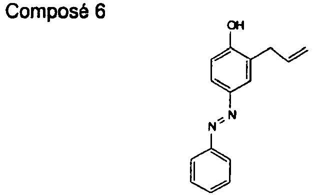

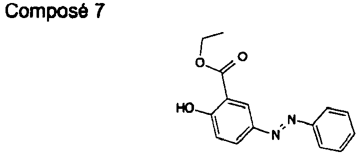

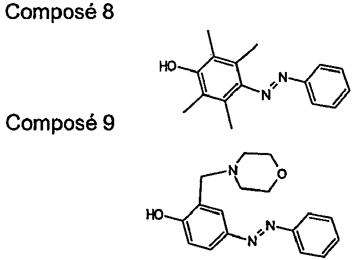

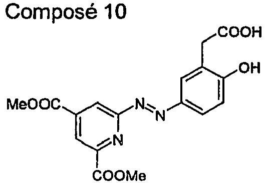

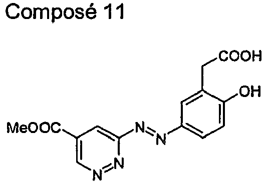

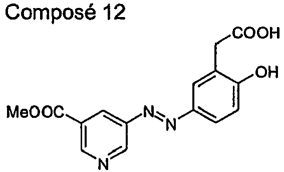

Les composés de formule (I), salifiés ou non sont connus en tant que tels. Ils peuvent être fabriqués de façon connus et notamment comme décrit dans les documents VOGEL's Textbook of Practical Organic Chemistry, 5th edition, 1989.

En particulier le composé 1 de formule : 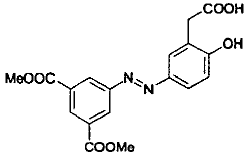 sera également désigné par l'abréviation PhCL28A dans ce qui suit.

Selon un mode de réalisation préféré, les compositions selon l'invention comprennent en outre au moins un agent bénéfique pour les cheveux tels que notamment les silicones, les huiles végétales, animales, minérales ou de synthèse, les cires, les céramides, les pseudocéramides, les polymères cationiques, les filtres solaires, les vitamines.

Les silicones utilisables conformément à l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

Selon un de ses aspects, la présente demande décrit l'utilisation non thérapeutique d'une composition cosmétique contenant au moins un inhibiteur de la 15 hydroxy-prostaglandine déshydrogénase pour le traitement de la canitie. L'utilisation d'une telle composition selon l'invention sera en particulier destinée à prévenir ou limiter l'apparition de poils ou de cheveux blancs, ou à en réduire le nombre.
L'un des avantages de l'utilisation d'inhibiteurs de 15-PGDH selon l'un au moins des aspects de l'invention est de rétablir une pigmentation de la peau et /ou des cheveux correspondant à une synthèse physiologique des médiateurs de la pigmentation, ce qui conduit à une coloration proche de la coloration naturelle. Les inhibiteurs de 15-PGDH n'ont pas pour activité principale de colorer directement la peau et/ou les phanères, en particulier lorsqu'il s'agit de composés de la famille des azoïques tel que le PhCL28A.

Selon un mode de réalisation préféré, la composition utilisable selon l'invention contient en outre au moins un agent accélérateur de pénétration.

De tels agents sont connus de l'homme du métier et comprennent notamment l'urée ou les composés cités dans la demande WO 01/74313. Ils seront classiquement présents à des concentrations de 0,01 à 20%,et notamment de 0,1 à 5%.

Avantageusement, les compositions utilisables selon l'invention contiennent en outre au moins un agent favorisant la pigmentation de la peau, des cheveux et/ou des poils différent d'un inhibiteur de la 15 hydroxy-prostaglandine déshydrogénase.
De tels composés sont notamment des substrats de la tyrosinase, tels que la tyrosine ou la L-DOPA, ou des composés activateurs de la voie de l'AMPc tels que des dérivés de pro-opiomélanocortines, l'adénosine, ou la forkoline ou ses dérivés. On peut également citer des extraits de végétaux tels que cyprès, Burnet, Sanguisorba officinalis ou de chrysanthème (Chrysanthemum morifolium), décrit notamment dans EP1014934.

Les compositions comprenant au moins un inhibiteur de 15-PGDH selon l'invention pourront également contenir des systèmes catalytiques comprenant 2 constituants, à savoir un premier constituant choisi parmi les sels et oxydes de Mn(II) et/ou Zn(II) et leurs mélanges et un second constituant choisi parmi les hydrogénocarbonates alcalins, les hydrogénocarbonates alcalino-terreux et leurs mélanges. De telles compositions favorisant la pigmentation sont notamment décrites dans les demandes FR 2 814947, FR 2 814943, FR 2 814946 ou FR 2 817469.
Selon une variante préférée, l'agent inhibiteur de 15 PGDH sera utilisé en association avec un composé actif pour favoriser la pigmentation des cheveux désavantageusement susceptible d'être métabolisé par cette enzyme. En effet, grâce aux travaux de la demanderesse, on sait maintenant que certains composés classiquement proposés à cet effet ont une activité moindre en raison de la présence de 15PGDH, qui par son action diminue la concentration et donc l'efficacité des substances au niveau du site d'action.

Conformément à la présente invention, on peut désormais obtenir des compositions ayant une efficacité renforcée, en associant un composé actif pour favoriser la pigmentation des cheveux, susceptible d'être métabolisé par la 15PGDH et un inhibiteur de la 15PGDH tel que défini précédemment. On obtiendra ainsi une action synergique des actifs pour favoriser la pigmentation des cheveux dans ces compositions.
La composition contiendra par exemple en outre au moins un composé choisi parmi les prostaglandines, notamment la prostaglandine PGE1, PGE2, leurs sels, leurs esters, leurs analogues et leurs dérivés, notamment ceux décrits dans WO 98/33497, WO 95/11003, JP 97-100091, JP 96134242, en particulier les agonistes des récepteurs des prostaglandines. Elle peut notamment contenir au moins un composé tel les agonistes (sous forme acide ou sous forme d'un précurseur notamment sous forme ester) du récepteur de la prostaglandine F2 alpha (FP-R) à l'exemple du latanoprost, du fluprostenol, du cloprostenol, du travoprost , le bimatoprost, les agonistes (et leurs précurseurs notamment les esters) des récepteurs de la prostaglandine E2 (EP1-R, EP2-R, EP3-R, EP4-R) tel le 17 phényle PGE2, le viprostol, le butaprost, le misoprostol, le sulprostone, le 16,16 diméthyle PGE2, 11 désoxy-PGE1, le 1 désoxy PGE1, les agonistes et leurs précurseurs notamment les esters du récepteur de la prostacycline (IP) tel le cicaprost, l'iloprost, l'isopcarbacycline, le beraprost, les agonistes et leurs précurseurs notamment les esters du récepteur de la prostaglandine D2 tel le BW245C ((4S)-(3-[(3R,S)-3-cyclohexyl-3-isopropyl]-2,5-dioxo)4-imidazolidineheptanoic acid), le BW246C ((4R)-(3-[(3R,S)-3-cyclohexyl-3-isopropyl]-2,5-dioxo)4-imidazolidineheptanoic acid)les agonistes et leurs précurseurs notamment les esters du récepteur aux thromboxanes A2 (TP) tel le I-BOP ([1S-[1a,2a(Z), 3b(1E,3S),4a]]-7-[3-[3-hydroxy-4-[4-(iodophenoxy)-1-butenyl]-7-oxabicyclo[2.2.1]hept-2-yl]-5-heptenoic acid Avantageusement, la composition selon l'invention comprendra au moins un inhibiteur de la 15PGDH tel que défini précédemment et au moins une prostaglandine ou un dérivé de prostaglandine comme par exemple les prostaglandines de la série 2 dont notamment PGF 2 α et PGE 2 sous forme salines ou sous forme de précurseurs notamment des esters (exemple les isopropyl esters), leurs dérivés comme le 16,16 dimethyl PGE2, le 17 phényl PGE2, le 16,16 diméthyle PGF2α, le 17 phényl PGF2α les prostaglandines de la série 1 comme le 11 désoxy prostaglandine E1, le 1 désoxy prostaglandine E1 sous forme salines ou sous forme de précurseurs notamment des esters, ou un agoniste non prostanoïque des récepteurs EP2 et/ou EP4 notamment tel que décrit dans EP 1175892.

De préférence, l'inhibiteur de 15 hydroxy-prostaglandine déshydrogénase est peu ou pas inhibiteur des enzymes impliquées dans la synthèse des prostaglandines au niveau du cheveu, ou prostaglandine synthases (notamment de la PGHS-1 ou COX-1, de la prostaglandine endoperoxyde H synthase, ou de la PGFsynthase).

D'autres actifs bénéfiques pour la santé et la vigueur des cheveux ou des poils pourront être présents dans la composition.
On peut notamment citer d'autres composés actifs pour favoriser la repousse et/ou limiter la chute des cheveux.

Ces composés pourront notamment être choisis parmi les inhibiteurs de lipoxygénase tels que décrits dans EP648488, les inhibiteurs de bradykinine décrits notamment dans EP 845700, les prostaglandines et leurs dérivés tels que décrits dans ce qui précède, les analogues non prostanoïques de prostaglandines tels que décrits dans EP 1175891 et EP1175890, WO 01/74307, WO 01/74313, WO 01/74314, WO 01/74315 ou WO 01/72268.
Des agents favorisant la pousse du cheveux pouvant être présents dans les compositions selon l'invention incluent les vasodilatateurs, les antiandrogènes, les cyclosporines et leurs analogues, les anti-microbiens, , les rétinoïdes, les tri-terpènes, seuls ou en mélange.
Les vasodilatateurs tels que les agonistes des canaux potassium incluant le minoxidil et les composés cités dans les brevets US 3 382247, 5 756092, 5 772990, 5 760043, 5466694, 5 438058, 4 973474, la chromakaline, le nicorandil et le diaxozide peuvent ainsi être présents dans les compositions.
Les anti-androgènes utilisables incluent notamment les inhibiteurs de 5α réductase, comme le finastéride et les composés décrits dans US 5 516779, l'acétate de cyprostérone, l'acide azélaïque, ses sels et ses dérivés et les composés décrits dans US 5 480913, le flutamide et les composés décrits dans les brevets US 5 411981, 5 565467 et 4 910226.
Les composés anti-microbiens peuvent être choisis parmi les dérivés du sélénium, le kétoconazole, le triclocarban, le triclosan, le pyrithione zinc, l'itraconazole, l'acide asiatique, l'hinokitiol, mipirocine, et les composés décrits dans EP680745, le chlorhydrate de clinycine, le peroxyde de benzoyle ou de benzyl et la minocycline.

Les rétinoïdes pourront être choisi parmi l'isotrétinoïne, l'acitrétine et le tazarotène. D'autres composés actifs pour favoriser la pousse et/ou limiter la chute des cheveux présents dans les compositions pourront également être choisi dans le groupe comprenant l'aminexil et ses dérivés, le 6-O-[(9Z,12Z)-octadéca-9,12-diènoyl]hexapyranose, chlorure de benzakonium, chlorure de benzethonium, phénol, oestradiol, maléate de chlorphéniramine, les dérivés de chlorophylline, cholestérol, cystéine, méthionine, nicotinate de benzyle, menthol, huile de menthe poivrée, panthoténate de calcium, panthénol, résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, éthylènes aryl substitués, les amino acides N-acylés, les flavonoïdes sans action sur les COX aux doses utilisées, les dérivés et analogues d'ascomycine, les antagonistes d'histamine, les triterpènes comme l'acide ursolique et les composés décrits dans US 5529769, US 5468888, US 5631282, les saponines, les inhibiteurs de protéoglycanase, les agonistes et antagonistes d'estrogènes, pseudotriènes, les cytokines et les promoteurs de facteurs de croissance, les inhibiteurs d'IL-1 ou d'IL-6, les promoteurs d'IL-10, les inhibiteurs de TNF, les vitamines, comme la vitamine D, les analogues de la vitamine B12 et le panthoténol, les hydroxyacides, benzophénones et l'hydantoïne.
La demande décrit également un procédé cosmétique pour favoriser la pigmentation de la peau, des ongles, des poils et/ou des cheveux, caractérisé en ce que l'on applique sur la peau et/ou le cuir chevelu et/ou les phanères au moins un inhibiteur de 15 hydroxy-prostaglandine déshydrogénase, ou une composition telle que définie précédemment. La composition ou l'inhibiteur de 15-PGDH seront appliqués au site d'action et seront laissés en contact pendant un temps plus ou moins long, l'application pouvant être répétée à intervalles réguliers ou non pendant plusieurs heures, jours, semaines ou mois.
L'inhibiteur de 15PGDH pourra être appliqué simultanément avec les autres actifs proposés en association ou de façon séquentielle dans le temps.

Les exemples qui suivent sont destinés à illustrer l'invention sans en limiter la portée. Dans ces exemples on se référera aux figures suivantes.
Figure 1: expression de le 15 hydroxy-prostaglandine déshydrogénase par des mélanocytes de cheveux en culture.
Figure 2: expression de prostaglandine H synthase 1 par des mélanocytes de cheveux en culture.

**Exemple 1a:** Mise en évidence de l'expression de l'ARNm codant pour la PGHS-1 (témoin ARNm codant pour la Glycéraldéhyde 3 phosphate déshydrogénase) et codant pour la 15-PGDH (témoin ARNm pour l'actine) dans les mélanocytes de cheveux pigmentés.

### a- Isolement des mélanocytes de cheveux pigmentés:

La biopsie de scalp humain est découpée en petits morceaux, placés dans une solution de dispase (2,4 U/ml, Boehringer Mannheim, Gmbh, D) et incubés dix huit heures à +04°C. Les fragments sont ensuite lavés dans du PBS. Le compartiment épithélial est séparé du derme à l'aide de pinces sous binoculaire. Les structures épithéliales sont ensuite micro-disséquées pour séparer les follicules pileux et l'épiderme, puis triées. Les cheveux isolés sont regroupés et traités par la trypsine cinq minutes à 37°C, puis la trypsine est neutralisée (Trypsin Neutralising System (TNS), C41110, Promocell, Heidelberg, D). La suspension cellulaire obtenue est ensemencée dans le milieu pour culture primaire pour mélanocytes (M2, Promocell, Heidelberg, D). Après six jours de culture, le milieu est renouvelé tous les deux ou trois jours. Après dix à vingt jours la culture est constituée de kératinocytes et de mélanocytes. Les mélanocytes sont sélectionnés par trypsination différentielle en incubant la culture avec de la trypsine-EDTA 0,05%-0,02% (w/v) pendant trois minutes à 37°C, puis la trypsine est neutralisée par le TNS. Il peut arriver que la culture primaire contienne quelque fibroblastes contaminants, ces derniers sont éliminés par un traitement répété à la généticine 25 µg/ml.

### b- Extraction et purification des ARN messagers :

L'extraction des ARN messagers est réalisée à partir de deux cultures de mélanocytes de follicules pileux distinctes, originaires de deux prélèvements issus d'individus différents. L'extraction des ARN messagers (réalisée sur les passages P3 ou P4, boite de 35 mm à confluence) est effectuée selon le protocole et avec les réactifs du kit QuickPrepr mRNA (Pharmacia Biotech, Bruxelles, Belgique).
Pour chaque échantillon (une culture cellulaire à confluence dans une boite de 35 mm de diamètre) mis à l'étude, le protocole suivant sera appliqué.
Le surnageant de culture cellulaire est éliminé et remplacé par 800 µl de tampon de lyse, le lysat obtenu est récupéré et introduit dans un microtube en polypropylène de 1,5 ml (tube 1). 1 ml de suspension de microsphères Oligo(dT-18) cellulose est introduit dans un microtube de 1,5 ml (tube 2) et centrifugé à 14000 trs/min pendant 1 minute. Le surnageant est éliminé. Le contenu du tube 1 est alors introduit dans le tube 2, les microsphères sont remise en suspension dans le lysat par agitation douce du tube pendant 3 minutes.
Les ARNs polyA+ fixés sur les microsphères sont isolés des contaminants par des lavages. Le tube est centrifugé à 14000 trs/min pendant 1 minute, le surnageant est éliminé et remplacé par 1 ml de tampon de lavage (high salt buffer). Les microsphères sont remises en suspension comme précédemment et le tube est agité doucemment pendant 1 minute. Le tube est centrifugé à 14000 trs/min pendant 1 minute, le surnageant est à nouveau éliminé et remplacé par 1ml de low salt buffer.
Au total cinq lavages avec le low salt buffer suivis de trois lavages avec le high salt buffer seront ainsi effectués.

Le contenu du troisième lavage (microsphères + tampon) est introduit sur une microcolonne contenant un filtre à la base (colonne microspin™) placée dans un microtube de 1,5 ml. L'ensemble est centrifugé à 14000 trs/min pendant 1 minute. La microcolonne est récupérée et placée dans un microtube de 1,5 ml. Les ARN messagers polyA+ sont alors élués par un volume final total de 0,4 ml de tampon d'élution préalablement porté à 65°C.

### c- Synthèse des brins complémentaires d'ADN (ADNc) :

Cette étape est réalisée par l'utilisation du kit First strand cDNA synthesis (Pharmacia Biotech, Bruxelles, Belgique).
Les échantillons d'ADN complémentaires obtenus sont dilués au 1/10 ème dans de l'eau stérile avant PCR.

### d- Choix des amorces, PCR (Poly Chain Reaction)

Des amorces spécifiques des séquences d'intérêts seront utilisées après synthèse à façon par Genset SA, rue Robert et Sonia Delaunay, Paris.
Le premier couples d'amorces s'hybridant sur la séquence codant pour une protéine ubiquitaire (la glycéraldéhyde 3 phosphodéshydrogénase). Les deux autres couples d'amorces s'hybridant sur la séquence codant pour la prostaglandine H synthase 1 et la 15 hydroxyprostaglandine déshydrogénase respectivement.

### β Actine humaine; N° accession genbank: NM_001101

AMORCE SENS: 5'-ATGGATGATGATATCGCCGCGCT-3'
Amorce anti-sens: 5'-CGGACTCGTCATACTCCTGCTTG-3'
Fragment amplifié: 1096 paires de bases.

### Prostaglandine H synthase 1 (PGHS-1) ; N° accession genbank : S36271

Amorce sens: 5'-CTCATAGGGGAGACCATCAAG-3'
Amorce anti-sens: 5'-CCTTCTCTCCTACGAGCTCCT-3'
Fragment amplifié: 451 paires de bases.

### 15 hydroxyprostaglandine humaine (15PGDH); N°accession genbank: NM_000860

Amorce sens: 5'-TGCCAATGGATTGATAACACTCAT-3'
Amorce anti-sens: 5'-ACAGCAGTTTTCATCTGGGATATG-3'
Fragment amplifié: 706 paires de bases.

Les réactions PCR sont réalisées selon le protocole TAKARA Taq™, TAKARA Shuzo Co., LTD. Biomedical Group, Seta 3-4-1, Otsu, Shiga, 520-2193, Japon. Les températures d'hybridation (THs) sont respectivement de 54°C (15-PGDH) et 57°C (Actine, GAPDH et PGHS-1) le nombre de cycles =35.
1 µl de chacun des échantillons d'ADN complémentaires est en réaction.
5 µl (2,5 µl + 2,5 µl) des couples d'amorces à 40 ng/µl sont en réaction.

| | |
|---|---|
| 4' à 95°C | 1 cycle |
| | |
| 30" à 94°C | 35 cycles |
| 1' à THs°C | |
| 1' à 72°C | |
| | |
| 7' à 72°C | 1 cycle |

### d- Lecture

### 1.1 Préparation d'un gel d'agarose.

Pesée de 0,65 g d'agar (Molecular biology certified agarose, Bio-Rad laboratories 2000 Alfred Nobel Dr., Hercules, CA 94547, USA).
Ajout de 50 ml de tampon 1X TAE, Amresco, Solon, OHIO 44139, USA.
L'agarose en suspension est portée à ébullition puis introduite dans une cuve contenant une goutte de bromure d'éthydium (25 µg), Amresco, Solon, OHIO 44139, USA.
Un "peigne" permettant le dépôt des échantillons est placé à une extrémité de la cuve.
Après 30 minutes de refroidissement (température de la pièce), 20 µl des résultats de PCRs sont introduits individuellement dans un puits du gel, de même que 10 µl d'un standard échelle de poids moléculaires (Amplisize ™, molecular Ruler, 170-8200), Bio-Rad.

L'ensemble est soumis en immersion dans un large excès de tampon TAE 1X à un champ électrique de 100 volts pendant 45 minutes.
L'exposition du gel sous un éclairage ultra-violet permet d'observer par fluorescence, les résultats obtenus.
Les résultats de l'étude de l'expression de la 15PGDH sont montrés sur la figure 1
A. source de cDNA issue de mélanocytes de cheveux donneur 1
B. source de cDNA issue de mélanocytes de cheveux donneur 2
M. Echelle de marqueurs moléculaires en paires de bases (pb)
A1, B1: expression de la 15-PGDH (706 pb)
A2,B2: expression de l'actine (gène de référence), (1096 pb)

Les gels montrant l'expression de la PGHS-1 sont représentés sur la figure 2:
A. source de cDNA issue de mélanocytes de cheveux donneur 1
B. source de cDNA issue de mélanocytes de cheveux donneur 2
M. Echelle de marqueurs moléculaires en paires de bases (pb)
A1, B1: expression de la PGHS-1 (451 pb)
A2,B2: expression de l'actine (gène de référence), (1096 pb).

**Exemple 2a:** Mise en évidence de l'expression de l'ARNm de la 15PGDH dans les fibroblastes de papilles dermiques de cheveux en culture.

### 1. Dissection des follicules pileux.

Des follicules pileux provenant de lifting de donneurs volontaires sont disséqués selon la méthode décrite dans le brevet B.Bernard/O.Gaillard FR2736721A1 du 17/01/97; US5712169A du 28/01/98.
Les follicules isolés sont placés en immersion dans une boite de pétri contenant 20 ml de milieu de culture 199 Gibco référence 31153-018, Life technologie, BP 96, Cergy Pontoise Cedex, supplémenté à 1% (v/v) d'une solution d'antibiotiques Gibco référence 15240-096.
A l'aide de deux aiguilles, référence NN-2516R, Terumo europe N.V. Leuven, Belgique montées chacune sur une seringue de 1ml, référence BS-01 N, Terumo, 15 papilles dermiques sont extraites des bulbes folliculaires.
Ces 15 papilles sont introduites dans une boite de pétri de 35 mm de diamètre contenant 2 ml de milieu 199 précédemment utilisé contenant 20 % de sérum de veau foetal Gibco, référence 10091-130. La boite est ensuite placée dans un incubateur thermostaté à 37°C sous 5% de CO2.
Un premier passage est réalisé après 3 semaines d'incubation sans que le milieu n'ait été préalablement remplacé. Le milieu est éliminé, 1 ml de solution de trypsine Gibco référence 25050-022 est introduit dans la boite de pétri, la boite est replacée dans l'incubateur 4 minutes. Les cellules (fibroblastes de papilles) ainsi remises en suspension dans la solution de trypsine (contrôle microscopique) sont introduites dans un tube de 15 ml, référence Falcon, 352097, Becton Dickinson, Chemin des sources, BP 37, Meylan 38241, contenant 10 ml de milieu 199 précédemment utilisé (contenant 20% de sérum de veau foetal).
Après centrifugation 5 min à 1500 tours par minute, le surnageant est éliminé et le culot cellulaire repris par 5 ml de milieu 199 précédemment utilisé (contenant ici 10% de sérum de veau foetal).
La suspension cellulaire est introduite dans une boite de pétri de 35 mm de diamètre et replacée dans l'incubateur (37°C, 5% de CO2).
Les passages suivant P2,P3,P4 sont réalisés selon le même principe. Ceux-ci sont effectués dès lors que les cellules ont atteint la confluence.

### 2. Extraction et purification des ARN messagers.

L'extraction des ARN messagers des fibroblastes de papilles dermiques (réalisée sur les passages P3 ou P4, boite de 35 mm à confluence) est effectuée selon le protocole et avec les réactifs du kit QuickPrepr mRNA (Pharmacia Biotech, Bruxelles, Belgique). Pour chaque échantillon (une culture cellulaire à confluence dans une boite de 35 mm de diamètre) mis à l'étude, le protocole suivant sera appliqué.
Le surnageant de culture cellulaire est éliminé et remplacé par 800 µl de tampon de lyse, le lysat obtenu est récupéré et introduit dans un microtube en polypropylène de 1,5 ml (tube 1).
1 ml de suspension de microsphères Oligo(dT-18) cellulose est introduit dans un microtube de 1,5 ml (tube 2) et centrifugé à 14000 trs/min pendant 1 minute. Le surnageant est éliminé. Le contenu du tube 1 est alors introduit dans le tube 2, les microsphères sont remise en suspension dans le lysat par agitation douce du tube pendant 3 minutes.

Les ARNs polyA+ fixés sur les microsphères sont isolés des contaminants par des lavages. Le tube est centrifugé à 14000 trs/min pendant 1 minute, le surnageant est éliminé et remplacé par 1 ml de tampon de lavage (high salt buffer). Les microsphères sont remises en suspension comme précédemment et le tube est agité doucemment pendant 1 minute. Le tube est centrifugé à 14000 trs/min pendant 1 minute, le surnageant est à nouveau éliminé et remplacé par 1 ml de low salt buffer.
Au total cinq lavages avec le low salt buffer suivis de trois lavages avec le high salt buffer seront ainsi effectués.
Le contenu du troisième lavage (microsphères + tampon) est introduit sur une microcolonne contenant un filtre à la base (colonne microspin™) placée dans un microtube de 1,5 ml. L'ensemble est centrifugé à 14000 trs/min pendant 1 minute. La microcotonne est récupérée et placée dans un microtube de 1,5 ml. Les ARN messagers polyA+ sont alors élués par un volume final total de 0,4 ml de tampon d'élution préalablement porté à 65°C.

### 3. Précipitation des ARN messagers.

Dans le tube contenant l'éluat sont introduits 10 µl de solution de glycogène, 40 µl d'acétate de potassium 2,5M et 1 ml d'éthanol absolu à -20°C. Le tube est placé dans de la carboglace (-80°C). Après 1h00, le tube est centrifugé à 4°C à 17500 trs/min pendant 15 minutes. Le surnageant est précautionneusement éliminé (les ARNms forment un tout petit culot) et remplacé par 1 ml d'éthanol 80% (éthanol/eau ; v/v) à -20°C. Le tube est centrifugé 15 min à 17500 trs/min à 4°C et le surnageant complétemment éliminé. Le culot est repris par 8 µl d'eau distillée stérile.

### 4. Synthèse des brins complémentaires d'ADN (ADNc)

Cette étape est réalisée par l'utilisation du kit First strand cDNA synthesis (Pharmacia Biotech, Bruxelles, Belgique).
Le tube contenant les ARNms sont placés à 65°C pendant 10 minutes puis dans la glace pendant 5 minutes, sont ensuite introduits :
5 µl d'une solution tamponnée contenant une suspension de transcriptase inverse.
1µl d'amorces OligodT(18) à 0,8 µg/ml.
1 µl de solution aqueuse de dithiothréitol titrant 200 nM.
Le tube est incubé à 37°C pendant 1h00. La réaction est bloquée en plaçant le tube dans la glace.

### 5. Choix des amorces, réaction de polymérisation en chaîne (PCR)

1µl (d'une dilution au 1/10 ème dans de l'eau distillée stérile) d'ADNs complémentaires ainsi obtenus est soumis dans un milieu tamponné à une réaction de polymérisation en chaîne (PCR), en présence de couples d'amorces spécifiques (titrant 40 ng/ml), de TAQ Polymérase et de nucléotides selon les données du fournisseur.

Les amorces spécifiques des séquences d'intérêts qui seront utilisées sont obtenues par synthèse à façon par Genset SA, rue Robert et Sonia Delaunay, Paris.
Le premier couples d'amorces s'hybridant sur la séquence codant pour une protéine ubiquitaire (la βactine). Le deuxième couple d'amorces s'hybridant sur la séquence codant pour la 15 hydroxyprostaglandine déshydrogénase.

### β Actine humaine; N° accession genbank: NM_001101

AMORCE SENS: 5'-ATGGATGATGATATCGCCGCGCT-3'
Amorce anti-sens: 5'-CGGACTCGTCATACTCCTGCTTG-3'
Fragment amplifié: 1096 paires de bases.

### 15 hydroxyprostaglandine humaine (15PGDH); N°accession genbank: NM_000860

Amorce sens: 5'-TGCCAATGGATTGATAACACTCAT-3'
Amorce anti-sens: 5'-ACAGCAGTTTTCATCTGGGATATG-3'
Fragment amplifié: 706 paires de bases.

La réaction PCR est réalisée selon une adaptation du protocole TAKARA Taq™, TAKARA Shuzo Co., LTD. Biomedical Group, Seta 3-4-1, Otsu, Shiga, 520-2193, Japon. La température d'hybridation est de 54°C pour les couples d'amorces (β-actine ;PGFS, 15PGDH), le nombre de cycles =35.
*Une solution tamponnée de nucléotides est réalisée par mélange de 171 µl d'eau distillée stérile, 24,5 µl de tampon 10X du kit et 20 µl de mélange de nucléotides (dNTP) du kit.

Sont introduits dans un microtube adapté à la PCR :
1 µl (d'une dilution au 1/10) d'ADN complémentaire
43 µl du mélange de nucléotides en solution tamponnée *
5 µl (2,5 µl + 2,5 µl) des couples d'amorces à 40 ng/µl sont en réaction.
50 µl d'huile minérale.

Le tube est placé dans un appareil pour PCR et les cycles suivants sont programmés.

| | |
|---|---|
| 4' à 95°C | 1 cycle |
| 30" à 94°C | 35 cycles |
| 1' à 54°C | |
| 1' à 72°C | |
| | |
| 7' à 72°C | 1 cycle |

### 6. Lecture

### a. Préparation d'un gel d'agarose.

Pesée de 0,65 g d'agar (Molecular biology certified agarose, Bio-Rad laboratories 2000 Alfred Nobel Dr., Hercules, CA 94547, USA).
Ajout de 50 ml de tampon 1X TAE, Amresco, Solon, OHIO 44139, USA.
L'agarose en suspension est porté à ébullition puis introduit dans une cuve contenant une goutte de bromure d'éthydium (25 µg), Amresco, Solon, OHIO 44139, USA.
Un "peigne" permettant le dépôt des échantillons est placé à une extrémité de la cuve.
Après 30 minutes de refroidissement (température de la pièce), 20 µl des résultats de PCRs sont introduits individuellement dans un puits du gel, de même que 10 µl d'un mélange de standards de poids moléculaires (Amplisize ™, molecular Ruler, 170-8200, Bio-Rad laboratories 2000 Alfred Nobel Dr., Hercules, CA 94547, USA)..
L'ensemble est soumis en immersion dans un large excès de tampon TAE 1X à un champ électrique de 100 volts pendant 45 minutes.
L'exposition du gel sous un éclairage ultra-violet permet d'observer par fluorescence, les résultats obtenus.

Poids des amplimères attendus.
Actine = 1096 paires de bases ; 15-PGDH = 706 paires de bases ; PGFS = 1061 paires de bases.
Les échantillons 1,2,3 proviennent de cultures différentes de fibroblastes de papilles dermiques de cheveux humains.

### Recherche de l'expression de la 15 hydroxyprostaglandine déshydrogénase.

On constate que la 15-PGDH est exprimée dans les différents échantillons, avec une bande de PM caractéristique à 706 pb

### Exemple 3 a

### Clonage à partir de fibroblastes de papilles dermiques

Ont été réalisées, à partir de culture de fibroblastes de papilles dermiques de cheveux tel que décrit dans l'exemple 1 l'extraction, la purification des ARN messagers poly-A+ puis la synthèse d'ADN complémentaire (ADNc).
Des couples d'amorces (synthétisé par Genset) auxquels ont été ajoutés des séquences codant pour des sites de restrictions ont été choisis pour la 15-PGDH (n° d'accession Genbank = NM_000860)
a) Amorces pour la 15-PGDH
   5'-**GGG GAT CC**A TGC ACG TGA ACG GCA AAG TG-3' ; Amorce sens site BamH1 (en gras)
   5'-**TCT CGA G**AG CTG TTC ATT GGG T-3' ; Amorce anti-sens site Xho1 (en gras)
b) Réaction de polymérisation en chaîne (PCR)
   Le protocole de PCR appliqué pour le clonage de la 15-PGDH reprend dans les grandes lignes celui décrits précédemment à la différence près ci-dessous :
   La Taq Polymérase utilisée selon les données du fabricant (Pfu Turbo^{r} DNA Polymerase), Stratagene cloning systems, 11011 North Torrey Pines Road , La Jolla, CA 92037.
   Température d'hybridation 59°C, temps d'élongation 2 min, 25 cycles au total pour la 15-PGDH, amplimère attendu = 815 pb.
c) Les produits de PCR sont digérés par les enzymes de restrictions (BamH1 ; Xho1 pour la 15-PGDH) selon les données du fabricant (Amersham Pharmacia biotech, 12 avenue des Tropiques, ZA Courtaboeuf, 91944 Les Ulis) puis mis à migrer individuellement sur un gel d'agarose à 1,3 % (voir exemple 1 ; 6. Lecture)
d) Découpe des bandes correspondantes aux amplimères attendus (voir ac) à l'aide d'un scalpel (après repérage sous ultra-violets) et purification de ces découpes selon les recommandations du fabricant du kit Wizard^{r} PCR Preps DNA Purification system (Promega Corporation, 2800 Woods Hollow Road, Madison, WI 53711-5399).
e)
   15-PGDH
   Ligation dans un vecteur pGEX 4T3 (Amersham Pharmacia biotech, 12 avenue des Tropiques, ZA Courtaboeuf, 91944 Les Ulis) préalablement digéré (BamH1 / Xho1) et purifié, suivant les indications du fabricant du kit Fast-Link™ DNA ligation kit, Epicentre, 1202 Ann Street, WI 53713.
f) Transformations.
   Des bactéries compétentes de type BL21DE3plys seront utilisées pour la transformation avec la construction (pGEX4T3/15-PGDH),. Cette souche est commercialisée par la société Stratagene. Les transformations sont réalisées selon un protocole classiquement appliqué tel que décrit par exemple dans le kit Fast-Link™ DNA ligation précédemment utilisé. Les bactéries infectées (clones) sont sélectionnées (colonies blanches) après dépôt et culture 24h00 à 37°C d'une fraction de ces produits de transformations sur LB-Agar medium coulée en boite de pétri (L-2897, Sigma-Aldrich, L'isle D'Abeau Chesne, BP 701, 38297, Saint Quentin Fallavier) contenant 100 µg/ml d'ampicilline.
g) Production de la 15-PGDH.
   Une colonie provenant de la boite de pétri « transformation 15-PGDH » est prélevée et introduite dans 250 ml de milieu LB (L-3022, Sigma-Aldrich, L'isle D'Abeau Chesne, BP 701, 38297, Saint Quentin Fallavier) contenat 100 µg/ml d'ampicilline, le flacon est incubé sous agitation 16h00 à 37°C.
   Après 16h00, chacun des flacons est introduit dans un erlen contenant 2,5 L de milieu LB contenant 100 µg/ml d'ampicilline. Ces deux erlens sont incubés sous agitation à 37°C pendant 3h00 à 4h00 (jusqu'à ce que la densité optique mesurée à 630 nm soit comprise entre 0,6-0,9.
   Ajout d'isopropyl β-D thiogalactopyranoside (IPTG), (16758, Sigma-Aldrich, L'isle D'Abeau Chesne, BP 701, 38297, Saint Quentin Fallavier) tel que la concentration finale titre 0,1 mM.
   Les erlens sont incubés 24h00 supplémentaires sous agitation à température ambiante (20-25°C).
   Les cultures ainsi obtenues sont centrifugées (par fraction de 250 ml) à 5000 trs/min pendant 7 minutes, les culots sont repris avec 3 ml de tampon phosphate 10 mM pH = 7,00 (4°C) contenant un mélange d'inhibiteurs de protéases (protease inhibitor cocktail set I 539131, Calbiochem-Novabiochem Corporation, 10394 Pacific center Court, San Diego, CA 92121). Les suspensions bactériennes (regroupées en fractions de 40 ml dans un tube en polypropylène) sont bloquées dans la glace et soumis aux ultrasons (Vibra cell 20 kHz, 72434, Bioblock scientific, Parc d'innovation, BP111, 67403 Illkirch) la sonde étant plongée dans chaque tube 15 secondes (6 chocs de 15 secondes par tube). Chaque tube est centrifugé à 4°C 16000 trs/minutes pendant 1h00.
h) Purification de la 15-PGDH.
   Les surnageants sont récupérés et introduits dans un tube polypropylène contenant 1 ml de Gluthatione-Sepharose^{r} 4B (40 ml de surnageant pour 1 ml de Gluthatione-Sepharose^{r} 4B) préalablement lavée selon les recommandations du fabricant (Amersham Pharmacia Biotech, 12 avenue des Tropiques, ZA Courtaboeuf, 91944 Les Ulis).
   Les tubes sont placés verticalement sur un agitateur rotatif, rotation 10 tours par minutes pendant 1h00 à la température de la pièce (20-25°C), les.
   Les tubes sont centrifugés 1000 trs/min pendant 3 minutes, le surnageant est éliminé Sont introduits dans chacun des tubes 40 ml d'un tampon phosphate 10 mM pH = 7,00. Après agitation douce (retournement) les tubes sont à nouveau centrifugés 3 minutes à 1000 trs/min.
   L'opération est réaliseé 5 fois. Un sixième lavage est réalisé avec 40 ml de tampon phosphate salin pH = 7,2 (PBS, Bio-Merieux S , 69280 Marcy-l'Etoile). Après centrifugation le surnageant est à nouveau éliminé.
i) Elution de la 15-PGDH
   Une suspension de *thrombin Protease* est reconstituée à 1 unité/µl dans du PBS selon les recommandations du fabricant (Amersham Pharmacia Biotech, 12 avenue des Tropiques, ZA Courtaboeuf, 91944 Les Ulis).
   Sont introduits dans chaque tube contenant 1 ml de Gluthatione-Sepharose^{r} 4B, 950 µl de PBS et 50 µl de suspension de thrombine reconstituée. Ceux-ci sont agités en position légéremment inclinée 16h00 à 250 trs/min. Après 16h00 les tubes sont centrifugés à 3000 trs/min pendant 5min, les surnageants sont recueillis.
   Une évaluation de la quantité de protéine est effectuée en suivant la procédure de dosage Bio-Rad DC Protein Assay (Bio-Rad Laboratories, 2000 Alfred Nobel Dr, Hercules, CA 94547).
   Ainsi pour la 15-PGDH on obtient entre 0,2 et 5 mg de protéine par ml, 1 mg /ml le plus souvent.
   Les suspensions protéiques ainsi obtenues sont diluées respectivement dans du PBS additionné de 10% de glycérol et du PBS telle que les concentrations protéiques finales titrent 0,2 mg /ml pour la 15-PGDH La suspension est bloquée à -80°C jusqu'à utilisation.
   Des analyses électrophorétiques (SDS-Page) réalisées dans des conditions standards démontrent la qualité des résultats ainsi obtenus.

### Exemple 4a: Evaluation de l'effet de molécules sur ces enzymes et caractérisation de certaine famille molécules comme inhibiteurs de la 15 PGDH.

### a) Test 15-PGDH.

L'enzyme obtenue est à la concentration de 0,3 mg/ml et bloquée à - 80°C. Cette suspension est décongélée et stockée dans la glace.
Préparation d'un tampon Tris 100 mM pH = 7,4 contenant 0,1 mM de dithiothreitol (D5545, Sigma-Aldrich, L'isle D'Abeau Chesne, BP 701, 38297, Saint Quentin Fallavier), 1,5 mM de β-NAD (N6522, Sigma-Aldrich, L'isle D'Abeau Chesne, BP 701, 38297, Saint Quentin Fallavier), 50 µM de Prostaglandine E₂ (P4172, Sigma-Aldrich, L'isle D'Abeau Chesne, BP 701, 38297, Saint Quentin Fallavier).
Dans la cuve d'un spectrophotomètre (Perkin-Elmer, Lambda 2) thermostaté à 37°C dont la longueur d'onde de mesure est réglée à 340 nm sont introduits 0,965 ml de ce tampon (préalablement porté à 37°C). 0,035 ml de suspension enzymatique à 37°C sont introduits dans la cuve concommitament à l'enregistrement (augmentation de la densité optique à 340 nm).
La vitesse maximale de réaction est relevée.
Les valeurs essais (molécules) sont comparées à la valeur témoin (sans molécule), les résultats sont exprimés en % de la valeur témoin.

Les résultats obtenus pour les molécules A et B sont les suivants:

| à 50 µM | % Inhibition 15-PGDH |
|---|---|
| Molecule A | 43 |
| Molecule B | 57 |

### EXEMPLE I: exemples de composés hétérocycliques phényl-furannes, phényl-thiophènes et phényl-pyrroles de formule (I) utilisables dans l'invention:

### Composé 1 : acide 4-{5-[(2,4-dioxo-1,3-thiazolidin-5-ylidène)méthyl]-2-furyl}benzoïque

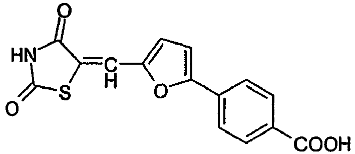

et plus spécialement le composé 1 a :

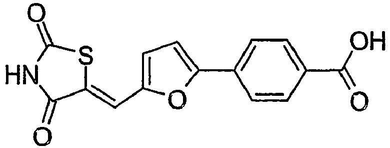

### Composé 2 : 4-{5-[(2,4-dioxo-1,3-thiazolidin-5-ylidène]méthyl]-2-furyl}benzoate d'éthyle

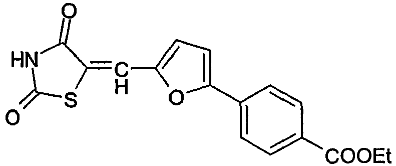

### Composé 3: 5-({5-[3,5-bis(trifluorométhyl)phényl]-2-furyl}-méthylène-1,3-thiazolidine-2,4-dione

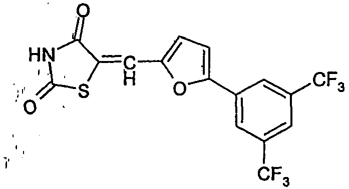

### Composé 4: acide 3-{5-[(2,4-dioxo-1,3-thiazolidin-5-ylidène)méthyl]-2-furyl}benzoïque

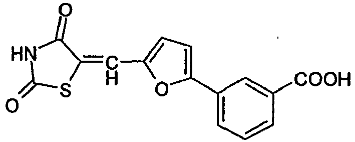

### Composé 5 : acide 4-{5-[(2,4-dioxo-1,3-thiazolidin-5-ylidène)méthyl]-2-thiophyl} benzoïque

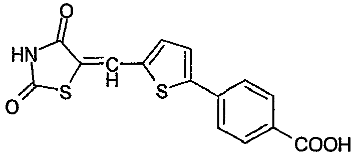

### Composé 6 : acide 4-{5-[(2-sulfo-4-oxo-1,3-thiazolidin-5-ylidène)méthyl]-2-furyl} benzoïque

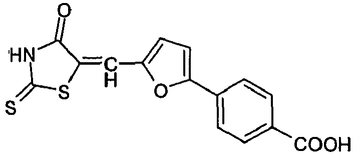

### Composé 7 : acide 4-{5-[(2,4-disulfo-1,3-thiazolidin-5-ylidène)méthyl]-2-furyl}benzoïque

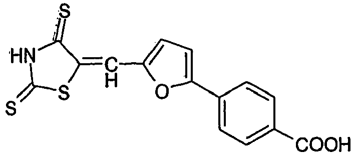

### Composé 8 : disel de sodium de l'acide 4-{5-[(2,4-disulfo-1,3-thiazolidin-5-ylidène)méthyl-2-furyl}benzoïque (isomère Z) :

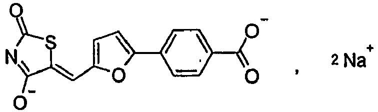

Avantageusement le composé de formule (I) est le disel de sodium de l'acide 4-{5-[(2,4-disulfo-1,3-thiazolidin-5-ylidène)méthyl]-2-furyl} benzoïque, et en particulier l'isomère sous forme Z.

Comme autres composés de formule (I) utilisables dans l'invention, on peut encore citer :

| Structure hétérocyclique D | **Aspect** | **Pureté LC *** | **MS **** | **Nom** |
|---|---|---|---|---|
| 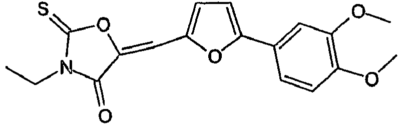 | poudre rousse | 93 | M+H | 5-[5-(3,4-Dimethoxyphenyl)-furan-2-ylmethylene]-3-ethyl-2-thioxo-oxazolidin-4-one |
| Composé D1 | | | M+Na | |
| 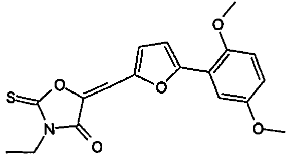 | poudre rouge | 100 | M+Na | 5-[5-(2,5-Dimethoxyphenyl)-furan-2-ylmethylene]-3-ethyl-2-thioxo-oxazolidin-4-one |
| Composé D2 | | | | |
| 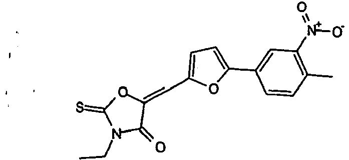 | poudre rouge | 100 | M-H M+Na | 3-Ethyl-5-[5-(4-methyl-3-nitro-phenyl)-furan-2-ylmethylene]-2-thioxooxazolidin-4-one |
| Composé D3 | | | | |
| 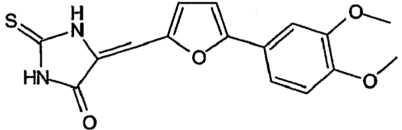 | poudre rouge | 91 | M+H | 5-[5-(3,4-Dimethoxyphenyl)-furan-2-ylmethylene]-2-thioxoimidazolidin-4-one |
| | | | M+Na | |
| Composé E1 | | | M-H | |
| 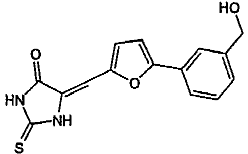 | gomme rouge | 100 | M+H | 5-[5-(3-Hydroxymethylphenyl)-furan-2-ylmethylene]-2-thioxoimidazolidin-4-one |
| Composé E2 | | | M-H | |
| 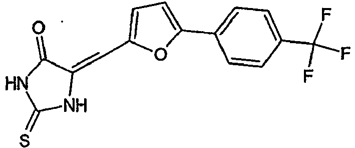 | solide orange | 100 | M-H | 2-Thioxo-5-[5-(4-trifluoromethyl-phenyl)-furan-2-ylmethylene]-imidazolidin-4-one |
| Composé E3 | | | | |
| 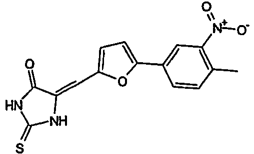 | poudre rouge | 73 | M-H | 5-[5-(4-Methyl-3-nitrophenyl)-furan-2-ylmethylene]-2-thioxoimidazolidin-4-one |
| Composé E4 | | | | |
| 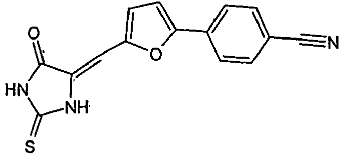 | poudre marron | 93 | M-H | 4-[5-(5-Oxo-2-thioxoimidazolidin-4-ylidenemethyl)-furan-2-yl]-benzonitrile |
| Composé E5 | | | | |
| 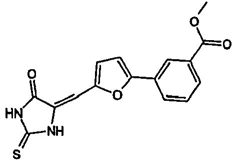 | poudre orange | 89 | M+H | 3-[5-(5-Oxo-2-thioxoimidazolidin-4-ylidenemethyl)-furan-2-yl]-benzoic acid methyl ester |
| | | | M+Na | |
| Composé E6 | | | M-H | |
| 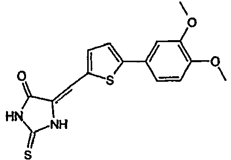 | poudre marron | 100 | M-H | 5-[5-(3,4-Dimethoxyphenyl)-thiophen-2-ylmethylene]-2-thioxoimidazolidin-4-one |
| Composé E7 | | | | |
| 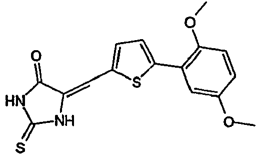 | poudre bordeaux | 65 | M-H | 5-[5-(2,5-Dimethoxyphenyl)-thiophen-2-ylmethylene]-2-thioxoimidazolidin-4-one |
| Composé E8 | | | | |
| 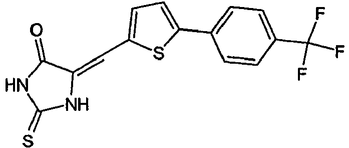 | poudre orange | 90 | M-H | 2-Thioxo-5-[5-(4-trifluoromethyl-phenyl)-thiophen-2-ylmethylene]-imidazolidin-4-one |
| Composé E9 | | | | |
| 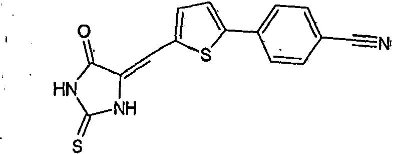 | poudre noire | 66 | M-H | 4-[5-(5-Oxo-2-thioxoimidazolidin-4-ylidenemethyl)-thiophen-2-yl]-benzonitrile |
| Composé E10 | | | | |
| 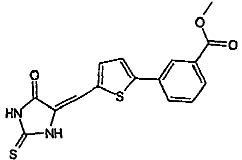 | poudre marron | 90 | M-H | 3-[5-(5-Oxo-2-thioxoimidazolidin-4-ylidenemethyl)-thiophen-2-yl]-benzoic acid methyl ester |
| Composé E11 | | | | |
| 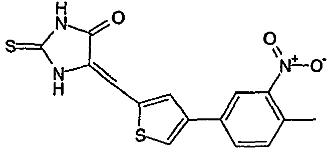 | poudre orange | 64 | M-H | 5-[4-(4-Methyl-3-nitrophenyl)-thiophen-2-ylmethylene]-2-thioxoimidazolidin-4-one |
| Composé E12 | | | | |
| 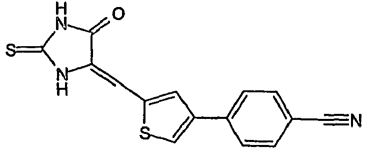 | poudre jaune | 53 | M-H | 4-[5-(5-Oxo-2-thioxoimidazolidin-4-ylidenemethyl)-thiophen-3-yl]-benzonitrile |
| Composé E13 | | | | |
| 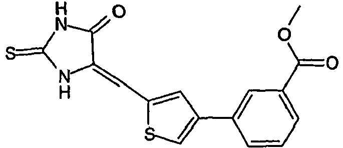 | poudre jaune | 91 | M-H | 3-[5-(5-Oxo-2-thioxoimidazolidin-4-ylidenemethyl)thiophen-3-yl]-benzoic acid methyl ester |
| Composé E14 | | | | |
| 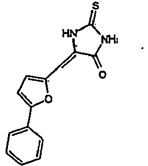 | gomme rouge | 100 | M-H | 5-(5-Phenyl-furan-2-ylmethylene)-2-thioxoimidazolidin-4-one |
| Composé E15 | | | | |
| 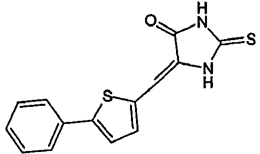 | solide orange | 81 | M-H | 5-(5-Phenyl-thiophen-2-ylmethylene)-2-thioxoimidazolidin-4-one |
| Composé E16 | | | | |

| Structure hétérocyclique F | | | | |
|---|---|---|---|---|
| 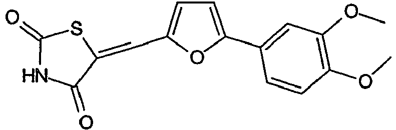 | poudre orange | 90 | M-H | 5-[5-(3,4-Dimethoxyphenyl)-furan-2-ylmethylene]-thiazolidine-2,4-dione |
| Composé F1 | | | | |
| 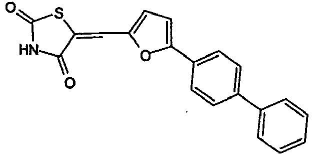 | poudre jaune | 88 | M-H | 5-(5-Biphenyl-4-yl-furan-2-ylmethylene)-thiazolidine-2,4-dione |
| Composé F2 | | | | |
| 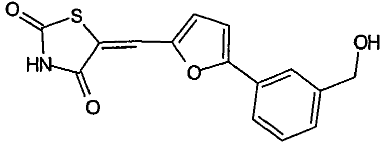 | poudre jaune | 91 | M-H | 5-[5-(3-Hydroxymethylphenyl)-furan-2-ylmethylene]-thiazolidine-2,4-dione |
| Composé F3 | | | | |
| 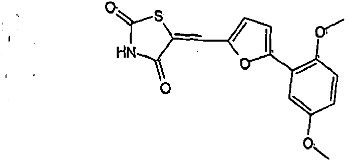 | coton orange | t 100,00 | M-H | 5-[5-(2,5-Dimethoxyphenyl)-furan-2-ylmethylene]-thiazolidine-2,4-dione |
| Composé F4 | | | | |
| 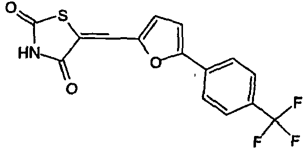 | poudre jaune | 100 | M-H | 5-[5-(4-Trifluoromethylphenyl)-furan-2-ylmethylene]-thiazolidine-2,4-dione |
| Composé F5 | | | | |
| 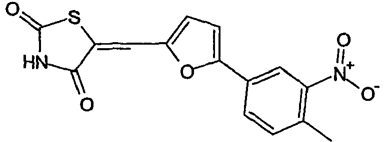 | poudre jaune | 69 | M+Na | 5-[5-(4-Methyl-3-nitrophenyl)-furan-2-ylmethylene]-thiazolidine-2,4-dione |
| Composé F6 | | | M-H | |
| 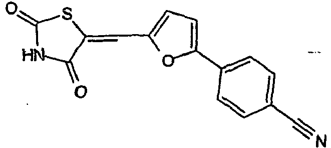 | gomme marron | 100 | M-H | 4-[5-(2,4-Dioxothiazolidin-5-ylidenemethyl)-furan-2-yl]-benzonitrile |
| Composé F7 | | | | |
| 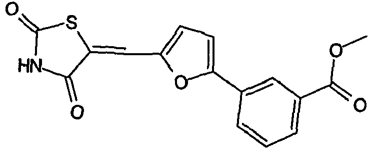 | poudre jaune | 61 | M-H | 3-[5-(2,4-Dioxothiazolidin-5-ylidenemethyl)-furan-2-yl]-benzoic acid methyl ester |
| Composé F8 | | | | |
| 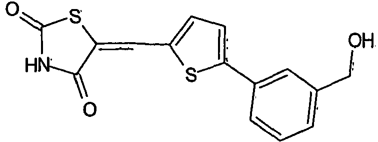 | poudre orange | 93 | M-H | 5-[5-(3-Hydroxymethylphenyl)-thiophen-2-ylmethylene]-thiazolidine-2,4-dione |
| Composé F9 | | | | |
| 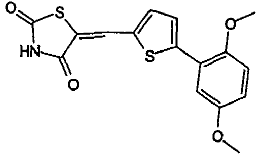 | poudre orange | 59 | M-H | 5-[5-(2,5-Dimethoxyphenyl)-thiophen-2-ylmethylene]-thiazolidine-2,4-dione |
| Composé F10 | | | | |
| 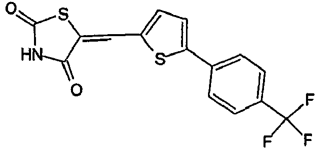 | solide rouge | 100 | M-H | 5-[5-(4-Trifluoromethylphenyl)-thiophen-2-ylmethylene]-thiazolidine-2,4-dione |
| Composé F11 | | | | |
| 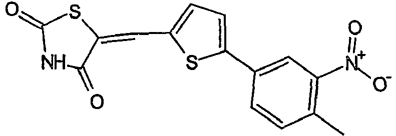 | poudre rousse | 56 | M-H | 5-[5-(4-Methyl-3-nitrophenyl)-thiophen-2-ylmethylene]-thiazolidine-2,4-dione |
| Composé F12 | | | | |
| 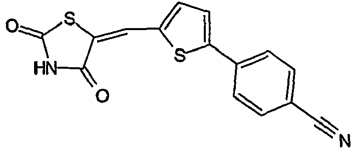 | poudre rouge | 41 | M-H | 4-[5-(2,4-Dioxothiazolidin-5-ylidenemethyl)-thiophen-2-yl]-benzonitrile |
| Composé F13 | | | | |
| 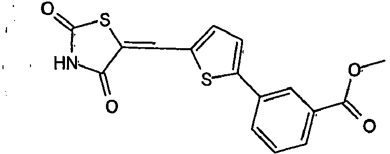 | poudre rouge | 54 | M-H | 3-[5-(2,4-Dioxothiazolidin-5-ylidenemethyl)-thiophen-2-yl]-benzoic acid methyl ester |
| Composé F14 | | | | |
| 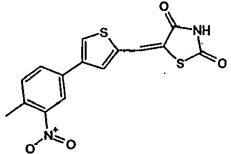 | gomme orange | 50 | M-H | 5-[4-(4-Methyl-3-nitrophenyl)-thiophen-2-ylmethylene]-thiazolidine-2,4-dione |
| Composé F15 | | | | |
| 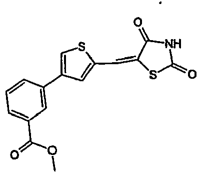 | poudre jaune | 59 | M-H | 3-[5-(2,4-Dioxothiazolidin-5-ylidenemethyl)-thiophen-3-yl]-benzoic acid methyl ester |
| Composé F16 | | | | |
| 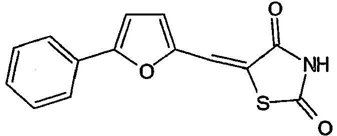 | paillettes jaunes | 71 | M-H | 5-(5-Phenyl-furan-2-ylmethylene)-thiazolidine-2,4-dione |
| Composé F17 | | | | |
| 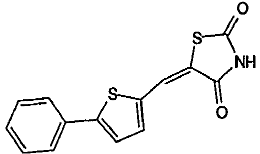 | poudre jaune | 84 | M-H | 5-(5-Phenyl-thiophen-2-ylmethylene)-thiazolidine-2,4-dione |
| Composé F18 | | | | |

| **Structure hétérocyclique G** | | | | |
|---|---|---|---|---|
| 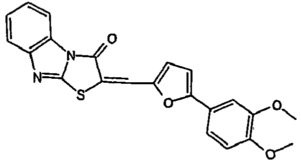 | poudre orange | 62 | M+H | 2-[5-(3,4-Dimethoxyphenyl)-furan-2-ylmethylene]-benzo[4,5]imidazo[2,1-b]thiazol-3-one |
| Composé G1 | | | | |

| | | | | |
|---|---|---|---|---|
| * LC : Chromatographie en phase liquide. ** MS : Spectrométrie de masse. | | | | |

Les composés selon l'invention peuvent être synthétisés selon le procédé décrit ci-après.

### Mode opératoire général de synthèse des composés de structure D, E, F ou G :

Ces structures hétérocycliques correspondent respectivement au 3-éthyl-2-thioxo-4-oxazolidinone (numéro CAS : 10574-66-0, masse molaire : 145, structure D), au 2-thiohydantoin (numéro CAS : 503-87-7, masse molaire : 116, structure E), au 2.4-thiazolidinedione (numéro CAS : 2295-31-0, masse molaire : 117, structure F), au thiazolo(2,3-b)benzimidazole-3(2H)-one (numéro CAS : 3042-01-1, masse molaire : 190, structure G).

Dans un tube réactionnel en Pyrex® du système de synthèse, sous irradiation microondes Discover de la société STEM, sont introduits 100mg d'aldéhyde, 1 équivalent d'hétérocycle de structure D, E, F ou G, de 20µL de pipéridine puis 1,5mL d'éthanol absolu.
Le tube est équipé d'un barreau aimanté puis fermé par un bouchon à sertir.
Le milieu réactionnel est ensuite irradié dans l'appareil Discover selon les paramètres suivants :
- Puissance restituée : 250W
- Température de consigne : 150°C
- Temps d'irradiation : 2 minutes
- Temps maximum pour atteindre la consigne : 4 minutes.

Après refroidissement, le milieu réactionnel est filtré sur fritté, le solide est lavé par une quantité minimum d'éthanol absolu puis séché sous vide.

### Rendement : 40-100%

Les échantillons sont analysés par LC-UV-MS selon les conditions suivantes :
Gradient : acétonitrile 10 - eau 90 à acétonitrile 90 - eau 10 en 8 minutes
Colonne : X-terra_MS C18 3.5µm 3 * 50 mm
Débit : 0.5mL/min
UV : barrette de diodes 290nm-450nm
MS : Electrospray à ionisation à pression atmosphérique positive et négative.

A titre d'exemple, on donne ci-après le schéma réactionnel des composés 1, 3, 4 et 8.

### EXEMPLE I-1 : Composé 1

### Préparation de l'acide 4-{5-[(2,4-dioxo-1,3-thiazolidin-5-ylidène)méthyl]-2-furyl}benzoïque

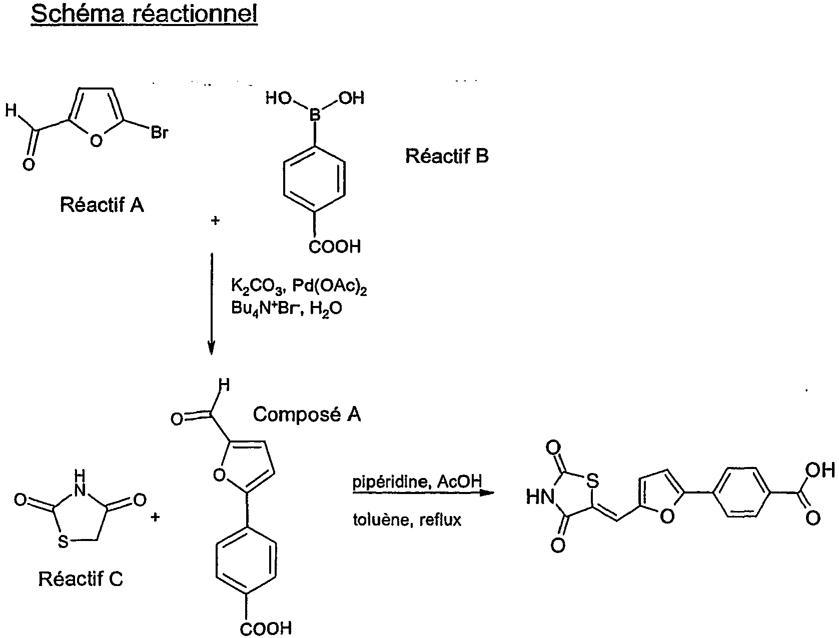

### Mode Opératoire

### Etape 1

Dans un ballon tricol de 50 ml muni d'un système réfrigérant, ainsi que d'une agitation magnétique, on dissout 1,99 g (6,16 mmol) de bromure de tétrabutylamonium dans 100 ml d'eau, puis on introduit 1,12 g (6,7 mmol) d'acide 4-carboxyphénylboronique (réactif B), 1,08 g (6,16 mmol) de 5-bromo-2-furaldéhyde (réactif A), 30 mg (2 mol %) d'acétate de palladium et 2,12 g (15,4 mmol) de carbonate de potassium. Le milieu réactionnel est laissé à température ambiante (20-25°C) pendant 12 heures. Le mélange est ensuite lavé avec de l'acétate d'éthyle (3 fois avec 50 ml). La phase aqueuse est acidifiée à pH-1-2 avec une solution d'acide chlorhydrique à 35%. Le solide beige-jaune formé (composé A) est filtré puis rincé avec de l'eau (3 fois avec 20 ml) et séché sous vide en présence de 1,2 g de pentoxyde de phosphore. Le rendement réactionnel est de 90%.

### Etape 2

Dans un ballon tricol de 50 ml muni d'un système "Dean-Stark", d'un thermomètre, ainsi que d'une agitation magnétique, on dissout 0,38 g (3,25 mmol) de thiazolidin-2,4-dione dans 20 ml de toluène, puis on introduit 0,7 g (3,25 mmol) de solide beige-jaune précédemment formé (composé A). On additionne ensuite 0,15 ml d'acide acétique et 0.15 ml de pipéridine puis le mélange est porté à reflux pendant 5 heures. Il se forme un solide jaune qui est filtré puis rincé avec du toluène (2 fois avec 20 ml). Le produit est alors séché sous vide en présence de 0,85 g pentoxyde de phosphore. Le rendement brut réactionnel est de 78%.

### Analyse

*Résonance Magnétique Nucléaire* : Le spectre obtenu est en accord avec la structure proposée.

### EXEMPLE I-2 : Composé 3

### Préparation du 5-({5-[3,5-bis(trifluorométhyl)phényl]-2-furyl}-méthylène-1,3-thiazolidine-2,4-dione

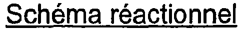

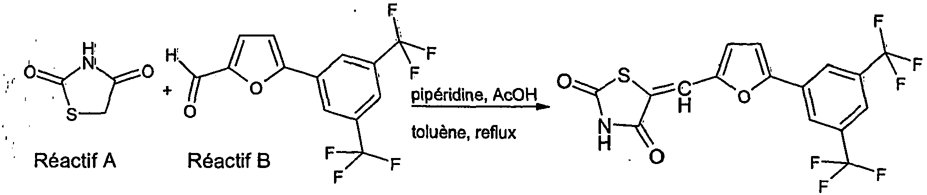

### Mode Opératoire

Dans un ballon tricol de 50 ml muni d'un système "Dean-Stark", d'un thermomètre, ainsi que d'une agitation magnétique, on dissout 0,38 g, (3,25 mmol) de thiazolidin-2,4-dione (réactif A) dans 20 ml de toluène, puis on introduit 1 g (3,25 mmol) de 5-[3,5-bis(trifluorométhyl)phényl]-2-furaldéhyde (réactif B). On additionne ensuite 0,15 ml d'acide acétique (AcOH) et 0,15 ml de pipéridine puis le mélange est porté à reflux pendant 5 heures. Un solide jaune s'est formé lors de la réaction. Il est filtré puis rincé avec du toluène (2 fois avec 20 ml) et séché sous vide en présence de 0,86 g de pentoxyde de phosphore. Le rendement réactionnel est de 65%.

### Analyse

- *Spectrométrie de Masse :* L'ion quasi-moléculaire (M-H)- de la molécule attendue, C₁₆H₇F₆NO₃S, est principalement détecté.
- *Résonance Magnétique Nucléaire* : Le spectre obtenu est en accord avec la structure proposée.

### EXEMPLE I-3 : Composé 4

### Préparation de l'acide 3-{5-[(2,4-dioxo-1,3-thiazolidin-5-ylidène)méthyl]-2-furyl}benzoïque

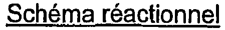

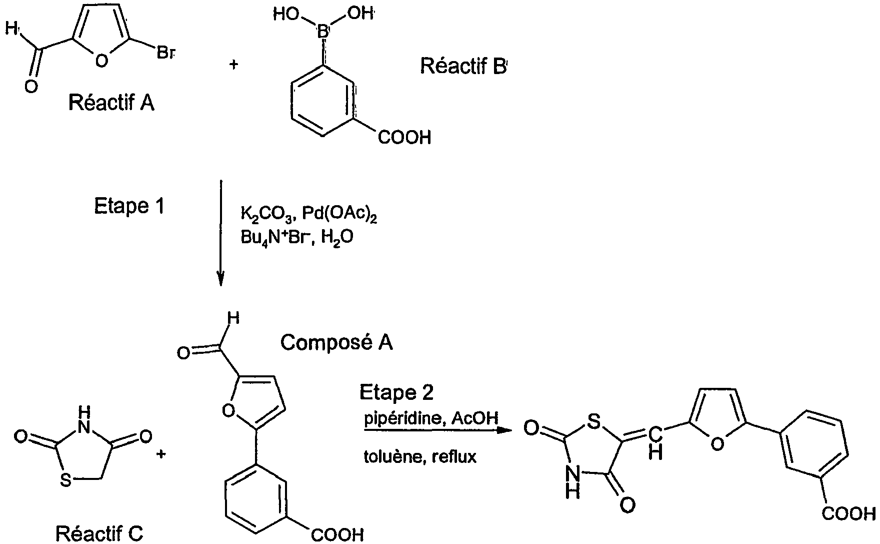

### Mode Opératoire

### Etape 1

Dans un ballon tricol de 50 ml muni d'un système réfrigérant, ainsi que d'une agitation magnétique, on dissout 1,99 g (6,16 mmol) de bromure de tétrabutylamonium dans 100 ml d'eau, puis on introduit 1,12 g (6,7 mmol) d'acide 3-carboxyphénylboronique (réactif B), 1,08 g (6,16 mmol) de 5-bromo-2-furaldéhyde (réactif A), 30 mg (2 mol %) d'acétate de palladium et 2,12 g (15,4 mmol) de carbonate de potassium. Le milieu réactionnel est laissé à température ambiante (20-25°C) pendant 12 heures. Le mélange est ensuite lavé avec de l'acétate d'éthyle (3 fois avec 50 ml). La phase aqueuse est acidifiée à pH-1-2 avec une solution aqueuse d'acide chlorhydrique : 35%. Le solide beige-rosé formé (composé A) est filtré puis rincé avec de l'eau (3 fois avec 20 ml) et séché sous vide en présence de 1,1 g de pentoxyde de phosphore. Le rendement réactionnel obtenu est de 82%.

### Etape 2

Dans un ballon tricol de 50 ml muni d'un système "Dean-Stark", d'un thermomètre, ainsi que d'une agitation magnétique, on dissout 0,542 g (4,62 mmol) de thiazolidin-2,4-dione dans 20 ml de toluène, puis on introduit 1 g (4,62 mmol) du solide beige-rosé précédemment formé (composé A). On additionne ensuite 0,15 ml d'acide acétique (AcOH) et 0,15 ml de pipéridine puis le mélange est porté à reflux pendant 5 heures. On observe la formation d'un solide jaune qui est filtré puis rincé avec du toluène (2 fois avec 20 ml). Le solide est ensuite dispersé dans 100 ml d'eau. On ajoute alors une solution aqueuse de soude 2N jusqu'à dissolution totale du produit puis on acidifie jusqu'à un pH de 1-2 avec une solution aqueuse d'acide chlorhydrique 1 N. Le solide marron formé est filtré puis lavé avec de l'eau (2 fois avec 50 ml) et séché sous vide en présence de 0,86 g de pentoxyde de phosphore. Le rendement est de 63%.

### Analyse

*Résonance Magnétique Nucléaire* : Le spectre obtenu est en accord avec la structure proposée.

### EXEMPLE I-4 : Composé 8

### Préparation du disel de sodium de l'acide 3-{5-[(2,4-dioxo-1,3-thiazolidin-5-ylidène)méthyl]-2-furyl}benzoïque

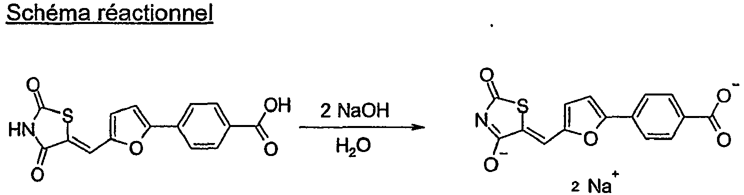

### Mode Opératoire

15 g de l'acide 3-{5-[(2,4-dioxo-1,3-thiazolidin-5-ylidène)méthyl]-2-furyl)benzoïque sont dissous dans 500mL d'une solution aqueuse de soude (2 équivalents). Cette solution est lavée avec 2 fois 50 mL de dichlorométhane puis concentrée partiellement. Cette solution est alors coulée sur de l'acétone. 11 g d'un précipité jaune-orange correspondant au disel de sodium de l'acide 3-{5-[(2,4-dioxo-1,3-thiazolidin-5-ylidène)méthyl]-2-furyl}benzoïque, sous forme Z sont ainsi obtenus.

### Analyse

*Résonance Magnétique Nucléaire* : Le spectre obtenu est en accord avec la structure proposée.

### EXEMPLE I-5 : Mise en évidence des propriétés inhibitrices spécifiques de la 15-PGDH des composés de formule (I).

### 1°) Test sur 15-PGDH

L'essai est réalisé comme décrit dans l'exemple 4a

Les valeurs essais (contenant les composés (I)) sont comparées à la valeur témoin (sans composé (I)) ; les résultats indiqués représentent la concentration à laquelle le composé (I) inhibe 50 % de l'activité enzymatique de 15-PGDH, notée IC50 dh.

### 2°) Test sur PGFS.

L'enzyme PGF synthase est obtenue comme décrit dans le document FR-A-02/05067, à la concentration de 0,5 mg/ml, en suspension dans un milieu approprié, et bloquée à - 80°C. Pour les besoins du test, cette suspension est décongelée et stockée dans la glace.

Par ailleurs, on prépare dans un flacon brun (abri de la lumière) un tampon Tris 100, mM, pH = 6,5 contenant 20 µM de 9,10 phénanthrène quinone* (P2896, Sigma-Aldrich, L'isle D'Abeau Chesne, BP 701, 38297, Saint Quentin Fallavier) et 100 µM de β-NADPH (N1630, Sigma-Aldrich, L'isle D'Abeau Chesne, BP 701, 38297, Saint Quentin Fallavier).

* Une solution mère titrant 1 mM est préparée dans de l'éthanol absolu, portée à 40°C ; le flacon est placé dans une cuve à ultrason pour faciliter la solubilisation du produit.

Dans la cuve d'un spectrophotomètre (Perkin-Elmer, Lambda 2) thermostaté à 37°C, dont la longueur d'onde de mesure est réglée à 340 nm sont introduits 0,950 ml de ce tampon (préalablement porté à 37°C). 0,05 ml de suspension enzymatique à 37°C sont introduits dans la cuve concomitamment à l'enregistrement (correspondant à une baisse de la densité optique à 340 nm). La vitesse maximale de réaction est relevée.

Les valeurs essais (contenant le composé (I)) sont comparées à la valeur témoin (sans composé (I)), les résultats indiqués représentent la concentration à laquelle le composé (I) inhibe l'activité enzymatique de PGFS de 50%, notée IC₅₀fs.

| composé | structure | Inhibition de 15-PGDH IC₅₀dh(µM) | Inhibition de PGFS IC₅₀fs(µM) |
|---|---|---|---|
| 1 | 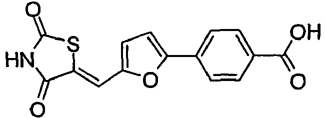 | 0,3 | 4 |

Il ressort de ce tableau que le rapport IC₅₀fs/IC₅₀dh du composé 1 est supérieur à 13. Le composé 1 a donc bien une activité inhibitrice vis-à-vis de 15-PGDH et en particulier sélective par rapport à PGFS.

### EXEMPLE I-6 : Mise en évidence de l'efficacité d'inhibition de 15PGDH spécifique sur un modèle cellulaire.

La présente étude vise à évaluer les composés de l'exemple I dans un modèle cellulaire. Cette étude permet de connaître la pénétration de l'actif dans le cytosol ainsi que son efficacité en tant qu'inhibiteur sélectif de 15-PGDH, dans des conditions expérimentales plus complexes qu'un simple milieu réactionnel.

### Matériel et Méthodes

**J-2.** Culture de U937 (CRL-1593 American Type Cells Collection) en milieu RPMI 1640 + 10 % de sérum de veau foetal + 2 mM de L-glutamine + des antibiotiques, à 37°C sous 5% de CO₂.
**J-1.** Préparation d'une suspension de U937 (1.10⁶ cellules / mL) dans le milieu RPMI 1640 + 10% de sérum de veau foetal + 2 mM de glutamine + des antibiotiques + 10 nM de PMA (phorbol 12 myristate 13 acétate) ; introduction de 200 µL par puits de tests de cette suspension dans une boite de 96 puits (3 puits par molécule et par concentration à tester + des témoins correspondants) ; incubation 36h00 à 37°C sous 5% de CO₂.
**J0**. Elimination des surnageants (les cellules ont adhéré au fond des puits : contrôle microscopique), et introduction dans chaque puits de 100 µl de RPMI 1640 + 2 mM de L-glutamine + 10 ng de LipoPolySaccharide (LPS) (sauf témoin absolu) + la molécule à tester à la concentration désirée (ici 5 et 25 µM).
Incubation 6h00 à 37°C sous 5% de CO₂.
Les solutions mères de molécules à tester sont à 25 mM dans le DiMéthylSulfOxide.

Tous les puits contiennent la même quantité finale de DMSO.
Evaluation immédiate de la quantité de PGF2α sécrétée par les cellules (50 µL) dans les différentes conditions (molécules ou témoins) par l'utilisation d'un kit de dosage immunoenzymatique (Cayman réf. 516011).

### Résultats ci-après en % du témoin LPS

| Référence Molécule (5µM) | % du témoin |
|---|---|
| Composé 1 : | + 76 ±20 |
| Composé 8 : | + 44 ±16 |

Ceci confirme que les composés selon l'invention sont des inhibiteurs sélectifs de 15-PGDH dans un environnement cellulaire et protègent les prostaglandines.

Les compositions ci-après sont obtenues par les techniques habituelles couramment utilisées dans le domaine cosmétique ou pharmaceutique.

### EXEMPLE I-7 : Lotion capillaire

- Composé 1 0,80 g
- Propylène glycol 10,00g
- Alcool isopropylique qsp 100,00 g

On applique cette lotion sur le cuir chevelu, une à deux fois par jour, à raison d'1 ml par application, en massant légèrement le cuir chevelu pour faire pénétrer l'actif. La chevelure est ensuite séchée à l'air libre.

### EXEMPLE I-8 : Lotion capillaire

- Composé 2 1,00g
- Propylène glycol 30,00g
- Alcool éthylique 40,00g
- Eau qsp 00,00 g

On applique cette lotion sur le cuir chevelu, une à deux fois par jour, à raison d'1 ml par application, en massant légèrement le cuir chevelu pour faire pénétrer l'actif. La chevelure est ensuite séchée à l'air libre.

### EXEMPLE I-9 : Lotion capillaire

- Composé 1 1 g
- Alcool éthylique 40,00g
- NaOH qsp (*)
- Eau qsp 100,00 g
(*) quantité suffisante pour neutraliser la fonction acide portée par noyau phényle (R₁)

On applique cette lotion sur le cuir chevelu, une à deux fois par jour, à raison d'1 ml par application en massant légèrement le cuir chevelu pour faire pénétrer l'actif.

### EXEMPLE 1-10 : Lotion capillaire

- Composé 8 1 g
- Alcool éthylique 40,00g
- Propylène glycol 30,00g
- Eau qsp 100,00 g

### EXEMPLE I-11 : Mascara cire/eau

- Cire d'abeilles 6,00%
- Cire de paraffine 13,00%
- Huile de jojoba hydrogénée 2,00%
- Polymère filmogène hydrosoluble 3,00%
- Stéarate de triéthanolamine 8,00%
- Composé 5 1,00%
- Pigment noir 5,00%
- Conservateur qs
- Eau qsp 100,00 %

Ce mascara s'applique sur les cils comme un mascara classique avec une brosse à mascara.

### EXEMPLE I-12 : Lotion capillaire

- Composé 8 0,10 g
- Latanoprost 0,10 g
- Propylène glycol 30,00g
- Alcool éthylique 40,00g
- Eau qsp 100,00 g

### EXEMPLE 1-13 : Lotion capillaire

- Composé 8 1 %
- Alcool éthylique 49,5 %
- Eau qsp 100 %

On applique cette lotion sur le cuir chevelu, une à deux fois par jour, à raison d'1 ml par application, en massant légèrement le cuir chevelu pour faire pénétrer l'actif. La chevelure est ensuite séchée à l'air libre.

### Exemple 5a: Effet d'une sélection d'inhibiteurs de 15-PGDH sur une lignée de mélanocytes MeWo (protection de PGF_{2α}).

### Méthodologie

### J=0

- Une ampoule de mélanocytes Mewo (American type cell collection, HTB-65) est amplifiée (flasque de 75 cm2, Costar) dans le milieu DMEM (Gibco) + 10 % de sérum de veau foetal + 2 mM de L-glutamine + 1% d'antibiotiques (Gibco). Après confluence, ces cellules (48h00 à 72h00) sont remises en suspension dans ce même milieu de culture, après action de trypsine (selon une procédure classiquement mise en oeuvre en culture cellulaire) à raison de 600 000 cellules par ml.
- Sont introduits 200 µl de cette suspension dans n fois 6 puits (n étant le nombre total d'essais soit x molécules à 2 concentrations) d'une boite 96 puits (Costar). La boite est placée dans un incubateur à 37°C sous 5% de CO₂.

### J=1

- Préparation dans le DMSO des solutions mères 1000X des actifs à évaluer (soit 5 et 25 mM).
- Elimination des surnageants des cultures réalisées précédemment (boite 96 puits), et rinçage avec 100 µl / puits de milieu DMEM + ajouts (voir description précédente) préchauffé à 37°C.
- Introduction de 100 µl / puits de milieu DMEM (+ ajouts) contenant 0,1 µl de DMSO (témoin) ou 0,1 µl de la solution mère de l'actif à évaluer (6 puits sont en essais par molécule et par concentration).

Pratiquement 1 ml de solution est préparé pour chacun des actifs (et par concentration) à tester donc 1 ml de milieu + 1 µl de DMSO (témoin) ou de solution mère 1000X. - La boite est à nouveau placée dans un incubateur à 37°C sous 5% de CO₂ pendant 4h00 puis un dosage de PGF2α est réalisé sur 50 µl de chacun des surnageants (des différents essais) à l'aide d'un kit immunoenzymatique (selon les recommandations du fabricant (PGF_{2α}, EIA kit, ref.516011, Cayman).

Résultats (en % de la quantité PGF2α mesuré dans les conditions témoins)

| **Molécules** | **5 µM** | **25 µM** |
|---|---|---|
| PhCL28A | + 43% | + 147% |
| Composé1a de l'exemple I | + 67% | + 51% |
| Composé 8 de l'exemple I | + 78% | + 64% |

Ces résultats montrent que la sélection d'inhibiteurs de 15-PGDH conduit, par rapport aux conditions témoin à la préservation de PGF_{2α}.
Ces actifs ont donc un intérêt dans la pigmentation dans la mesure où les prostaglandines sont décrites pour leur action sur la mélanogénèse.
La description des exemples II, II-1, II-2, III et IV, pages 118 à 142, ne contient pas d'objets selon l'invention.

### EXEMPLE II:

Comme exemples de composés pyrazoliques (non appartenant à l'invention) on peut citer les composés suivants : 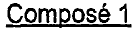 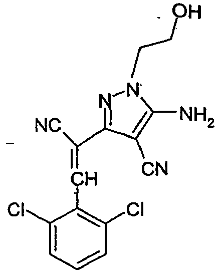 et plus spécialement le composé 1a (cycle en position Z de la double liaison)

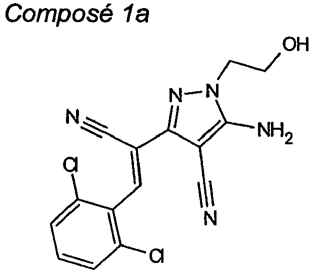

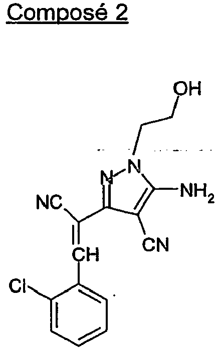

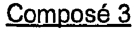

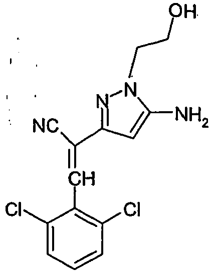

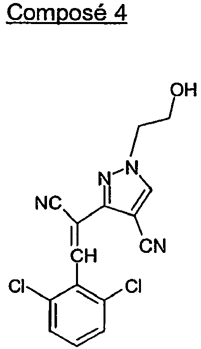

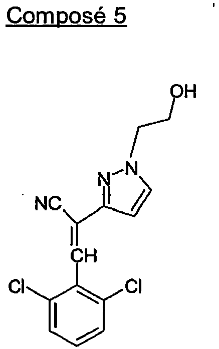

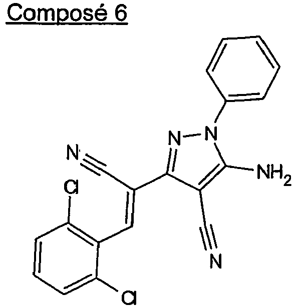

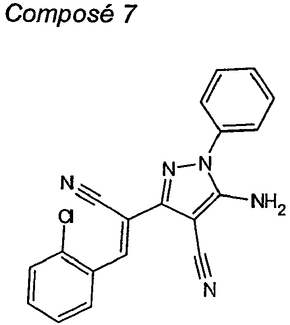

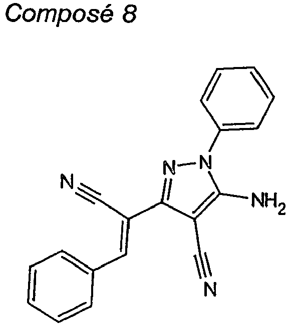

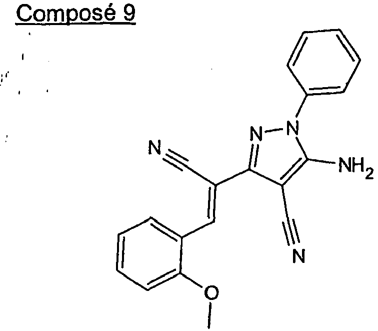

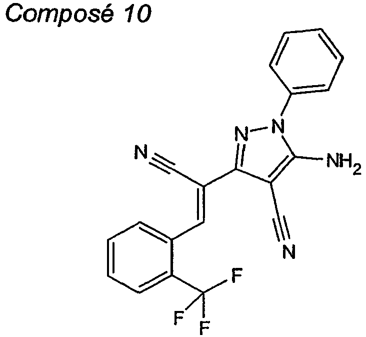

Comme composé pyrazolique nouveau de formule (I) et (III), on peut citer le composé 11

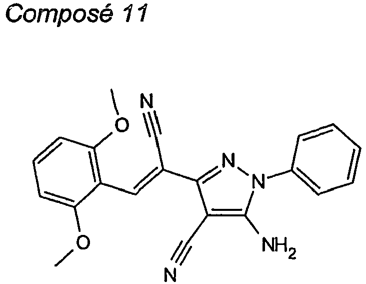

### EXEMPLE II-1 : Mode opératoire de la synthèse du 5-amino-3-[1-cyano-2-(2,6-diméthoxyphényl)-vinyl]-1-phényl-1H-pyrazole-4-carbonitrile (Composé 11).

Dans un ballon sous atmosphère d'argon surmonté d'un appareil de Dean Stark, 1 g (4,48 mmol) de 5-amino-4-cyano-1-phényl-3-pyrazoleacétonitrile est mis en suspension dans 15 mL de toluène. 0,744 g (1 éq.) de 2,6-diméthoxybenzaldéhyde et 0,030 mL de pipéridine sont ajoutés au mélange. Le milieu réactionnel est chauffé à reflux pendant une nuit, puis laissé redescendre jusqu'à température ambiante. Un précipité blanchâtre se forme et est filtré et lavé au toluène. Le filtrat est concentré à sec et le résidu est repris dans l'éthanol sous agitation pendant 15 minutes. La suspension est filtrée et le filtrat concentré à sec. Le résidu est regroupé avec le précipité précédemment obtenu, et purifié sur gel de silice (éluant : dichlorométhane / méthanol 98 / 2). 619 mg de produit sont ainsi obtenus avec un rendement de 37 %

### Analyses :

**Sepctrométrie Masse :** (ESI +/- dans CH₃OH/H₂O) : 372(MH)⁺, 394(MNa)⁺, 743(2MH)⁺, 765(2MNa)⁺, 370(M-H)⁻;
**Résonance Magnétique Nucléaire:** ¹H (400 MHz ; DMSO-d₆) δppm : 3,85 (s, 6H, OCH₃(13) et OCH₃(9)) ; 6,78 (d, 2H, H(10) et H(12)) ; 6,95 (s, 2H, NH₂(3)) ; 7,42 à 7,57 (m, 6H, H(2') à H(6') et H(11)) ; 7,86 (s, 1 H, H(7))
**Analyse élémentaire :**

| | | | | |
|---|---|---|---|---|
| *Théorie :* | C 67,91% ; | H 4,61% ; | N 18,86% ; | O 8,62% |
| *Analyse :* | C 67,30% ; | H 4,46% ; | N 18,88% ; | O 8,96% |

### EXEMPLE II-2 : Mise en évidence des propriétés inhibitrices spécifiques de la 15-PGDH des composés de l'exemple II.

### 1°) Test sur 15-PGDH

### la méthodologie est celle décrite dans l'exemple I-5

Les valeurs essais (contenant les composés testés) sont comparées à la valeur témoin (sans composé) ; les résultats indiqués représentent la concentration à laquelle le composé (I) inhibe 50 % de l'activité enzymatique de 15-PGDH, notée IC_{50dh}.

### 2°) Test sur PGF Synthase

### la méthodologie est celle décrite à l'exemple I-6

Les valeurs essais (contenant le composé testés) sont comparées à la valeur témoin (sans composé) ; les résultats indiqués représentent la concentration à laquelle le composé (I) inhibe 50% de l'activité enzymatique de PGFS, notée IC_{50fs}.

| composé | structure | Inhibition de 15-PGDH IC_{50dh} µM | Inhibition de PGF synthase IC_{50fs} µM | Sélectivité |
|---|---|---|---|---|
| 1a | 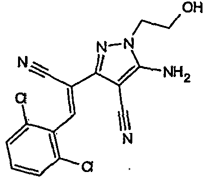 | 3 | > 50 | > 16,6 |
| 6 | 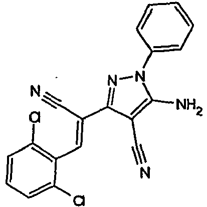 | 0,8 | > 50 | >62 |
| 7 | 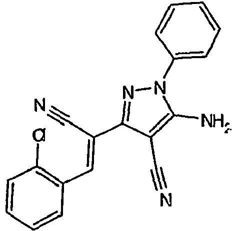 | 3 | > 50 | > 16 |
| 8 | 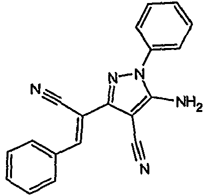 | 50 | > 75 | > 1,5 |
| 9 | 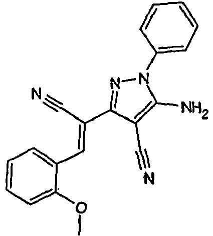 | 5 | >50 | >10 |

Il ressort de ce tableau que le rapport IC_{50fs}/IC_{50dh} des composés 1a, 6, 7, 8 et 9 est > 161,56. Les composés 1 a, 6, 7, 8 et 9 et plus spécialement 1a, 6, 7 et 9 ont donc une activité inhibitrice sélective vis-à-vis de 15-PGDH par rapport à PGF synthase.

### EXEMPLE III: exemples de composés pyrazolique (Non appartenant à l'invention)

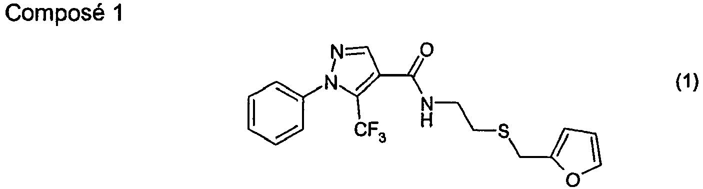

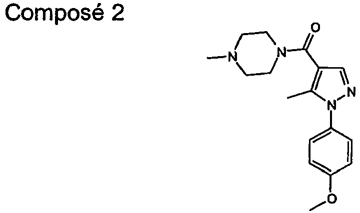

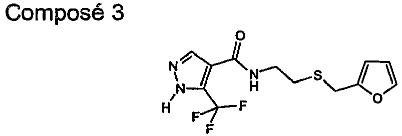

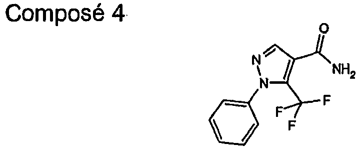

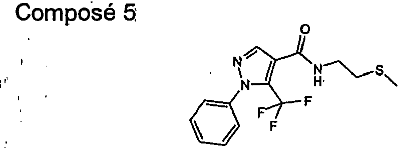

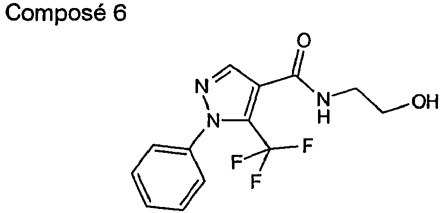

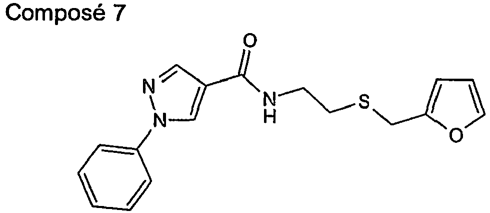

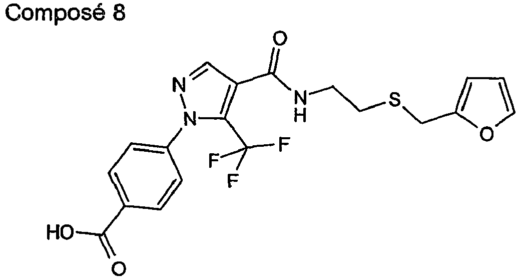

On donne ci-après les exemples de synthèse des composés 3, 5, 6, 7 et 8 de l'exemple III.

### SYNTHESE DU COMPOSE 3

### Synthèse du ethyl-5-trifluoromethyl-1H-pyrazole-4-carboxylate

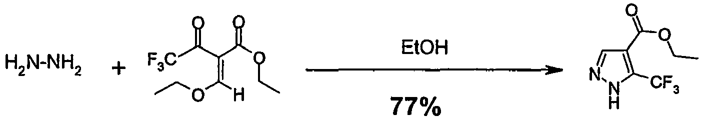

### Réactifs :

### Ethyl 2-(ethoxymethylene-4,4,4-trifluoro)-3-oxobutyrate

| | | | | |
|---|---|---|---|---|
| | C₉H₁₁F₃O₄ | PM : 240,18 | m =4,49 g | 18,71 mmol/1 eq. |
| Hydrazine (1M TétraHydroFuranne) | | H₄N₂ | V = 18,71 mL | 18,71 mmol/1 eq. |
| Ethanol | | | V = 25 mL | |

### Mode opératoire :

Dans un réacteur tricol de 100mL, sous argon et sous agitation magnétique, une solution d'hydrazine 1M (THF) est additionnée sur 25mL d'éthanol. La suspension est refroidie à -15°C (bain CCl₄/N₂) et l'oxobutyrate est additionné en 30 minutes, goutte à goutte sur l'hydrazine.

Après 2h30 à température ambiante, aucune évolution n'étant visible par Chromatographie sur Couche Mince, le milieu réactionnel est chauffé au reflux de l'éthanol (EtOH) pendant 16 heures. Une fois revenu à température ambiante, le solvant est alors évaporé et le solide obtenu est lavé deux fois par 10ml de pentane et filtré sur fritté.

3g d'un solide blanc cristallin sont ainsi récupérés. (Rendement : 77%).

### Analyses :

Solide beige dont la structure obtenue est conforme (RMN ¹H) (RMN ¹³C).

### Synthèse du 5-trifluoromethyl-1H-pyrazole-4-carboxylic acid

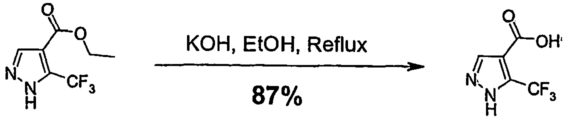

### Réactifs :

### Ethyl 5-trifluoromethyl-1 H-pyrazole-4-carboxylate

| | | | | |
|---|---|---|---|---|
| | C₇H₇N₂O₂F₃ | PM : 208,14 | m = 2,97 g | 14,3 mmol/1 eq. |
| Hydroxide de sodium | HNaO | PM : 39,99 | m = 5,71 g | 142 mmo/10 eq. |
| Ethanol | | | V = 30 mL | |

### Mode opératoire :

Dans un réacteur de 250mL sous agitation magnétique, le pyrazole est solubilisé dans l'éthanol. Après 15 minutes à température ambiante, une solution de soude 1,2N (120mL d'eau) est additionnée. Le milieu réactionnel est alors placé au reflux pendant 18h.

La solution est ensuite ramenée à 10°C puis acidifiée par HCl 1N. Après évaporation sous pression réduite de la totalité de l'éthanol et des deux tiers de l'eau, le précipité blanc formé est récupéré par filtration sur fritté, lavé à l'eau puis séché sous vide poussé.

La fine poudre blanche (2,30g) obtenue est caractérisée et correspond au produit attendu (Rendement : 87%).

### Analyses :

Solide blanc dont la structure obtenue est conforme (RMN ¹H) (RMN ¹³C).

### Synthèse du N-{2-[(2-furylmethyl)thio]ethyl}-5-trifluoromethyl-1H-pyrazole-4-carboxylic acid

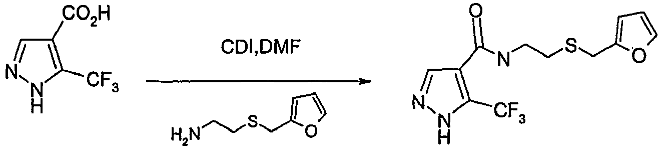

### Réactifs :

| | | | | |
|---|---|---|---|---|
| . 5-trifluoromethyl-1H-pyrazole -4-carboxylic acid | C₅H₃N₂O₂F₃ | PM : 180,08 | m = 0,80 g | 4,44 mmol/1 eq |
| . Carbonyl diimidazole (CDl) | C₇H₆N₄O | PM : 162,15 | m = 0,815 g | 5,39 mmol/1,4 eq. |
| . 2-(furfurylthio)éthylamine | C₇H₁₁NOS | PM : 157,234 | m = 3,22 g | 21,9 mmol/4,6 eq. |
| DiMéthylFormamide | | | V = 10 mL | |

### Mode opératoire :

Dans un réacteur de 100mL sous azote et sous agitation magnétique, le pyrazole est solubilisé dans le DMF. CDI est alors additionné rapidement en une seule portion et le mélange est maintenu sous agitation pendant 45 minutes environ. Un produit insoluble persistant est observé. L'amine est ensuite additionnée à la seringue avec un goutte à goutte rapide. Après une nuit d'agitation, le milieu réactionnel est noyé sur 100mL d'un mélange glace/eau. Le précipité blanc formé est récupéré par filtration et le filtrat est extrait à l'acétate d'éthyle (2*25mL). La phase organique est jointe au précipité et concentrée à sec.

Ce mélange brut est alors chromatographié sur gel de silice (chromatographie Flash, élution hexane/ acétate d'éthyle 3/1, 1 % ammoniaque). La fraction correspondant au produit attendu (Rf : 0,45 dans CH₂Cl₂/1% NH₃) est alors isolée et concentrée à sec. L'huile obtenue est reprise dans 2mL d'éthanol et noyée sur 100mL d'un mélange glace/eau. Le précipité obtenu est récupéré par filtration, filtré sur fritté et concentré à sec.

402mg d'un solide blanc sont ainsi obtenus (Rendement : 30%).

### Analyses :

### Solide blanc

RMN ¹H : (DMSO); 7,93 (s, 1H, CH pyrazole), 7,26 (s, 1H, CH furyl), 7,19 (m, 2H, NH + CONH), 6,10 (d, 2H, H furyl), 3,61 (s, 2H, CH₂), 3,37 (q, 2H, CH₂), 2,43 (t, 2H, CH₂).
RMN ¹³C : (DMSO/CDCl₃); 158,43 (CONH), 143,1 (CH), 111,4 (2CH), 108,7 (CH), 39,3 (CH₂), 32,1 (CH₂), 28,8 (CH₂). Carbones quaternaires non visibles du fait de la faible quantité de composé dans le tube.

### SYNTHESE DES COMPOSES 5 et 6

### Synthèse du ethyl 5-methyl-1-phenyl-1H-pyrazole-4-carboxylate

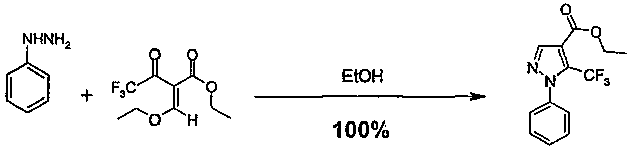

### Réactifs :

| | | | | |
|---|---|---|---|---|
| .Ethyl 2-(ethoxymethylene-4,4,4-trifluoro-3-oxobutyrate | | | | |
| | C₉H₁₁F₃O₄ | PM : 240,18 | m = 15,00 g | 62 mmol/1 eq. |
| Phénylhydrazine | C₇H₈N₂O₂ | PM : 108,14 | m = 6,38 g | 60 mmol/1,1 eq. |
| Ethanol | | | V = 500 mL | |

### Mode opératoire :

Dans un réacteur tricol d'1L, sous argon et sous agitation magnétique, la phénylhydrazine est placée en suspension dans 500mL d'éthanol absolu. La solution est refroidie à -15°C (bain CCl₄/N₂) et l'oxobutyrate est additionné en 45 minutes au goutte à goutte sur l'hydrazine. Après 4h à température ambiante, la solution est concentrée à sec. La poudre jaune obtenue est lavée au pentane, séchée sous vide et 18,5g d'un solide blanc sont ainsi isolés (Rendement : sup. 100%).

### Analyses :

Solide blanc dont la structure obtenue est conforme (RMN¹) (H: RMN ¹³C).

### Synthèse du ethyl 5-methyl-1-phenyl-1H-pyrazole-4-carboxylate

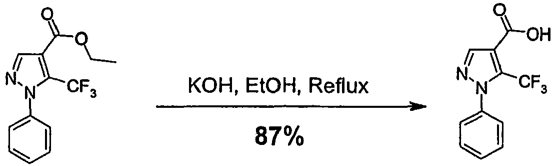

### Réactifs :

| | | | | |
|---|---|---|---|---|
| . Ethyl 5-trifluoro mrthyl-1-phenyl-1H-pyrazole-4-carboxylate | | | | |
| | C₂₁H₁₇N₂O₄F₃ | PM : 418,37 | m = 18,5 g | 65 mmol/1 eq. |
| . Hydroxyde de potassium (85%) | HKO | PM : 56,11 | m = 6,43 g | 97,5 mmol/1,5 eq. |
| . Ethanol | | | V = 150 mL | |

### Mode opératoire :

Dans un réacteur de 250mL sous agitation magnétique, le pyrazole est additionné sur une solution de potasse dans l'éthanol. Après 15 minutes à température ambiante, le milieu réactionnel est alors placé au reflux pendant 3h. Une fois ramenée à température ambiante, la solution est additionnée sur 600mL d'eau. Le mélange est lavé 3 fois par 250mL d'éther. La phase aqueuse est acidifiée par HCl 37% jusqu'à pH=1. L'éthanol résiduel est évaporé et un précipité jaune apparaît en solution. Le précipité est filtré sur fritté, lavé à l'eau et séché sous vide poussé pendant 72 heures.

14,5g d'une poudre jaune sont ainsi obtenus (Rendement : 87%).

Analsyses : Solide jaune dont la structure obtenue est conforme (RMN ¹H) (RMN ¹³C).

### Synthèse du N-(2-methylthio-ethyl)-1-phenyl-5-trifluoromethyl-1H-pyrazole-4-carboxamide (composé 5)

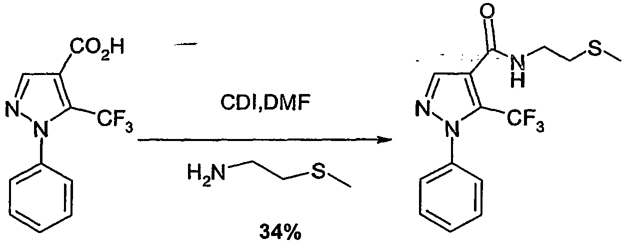

### Réactifs :

| | | | | |
|---|---|---|---|---|
| . 1-phényl-5-trifluorométhyl 1H pyrazole-carboxylic acid - | C₁₀H₈N₂O₂ | PM : 188,19 | m = 1,20 g | 4,71 mmol/1 eq |
| . Carbonyl diimidazole (CDI) | C₇H₆N₄O | PM : 162,15 | m = 0,88 g | 5,42 mmol/ 1,15 eq. |
| . 2-thiométhyl-éthylamine | C₃H₉NS | PM:91,18 | m = 2,00 g | 21,97 mmol/ 4,7 eq. |
| . DMF | | | V = 8,3 mL | |

### Mode opératoire :

Dans un réacteur sous azote et sous agitation magnétique, le pyrazole est solubilisé dans le DMF. CDI est alors additionné rapidement en une seule portion et le mélange ' est maintenu sous agitation pendant 20 minutes environ. L'amine est ensuite additionnée à la seringue avec un goutte à goutte rapide. Après 3h d'agitation, le suivi CCM de la réaction indique la disparition de la totalité du produit de départ.

Le milieu réactionnel est ensuite noyé sur 80mL d'un mélange glace/eau. Le précipité blanc formé après 15 minutes d'agitation est alors récupéré par filtration sur fritté et séché par succion. Le solide orangé obtenu est alors repris dans 50mL d'éther éthylique, lavé une fois à l'eau et séché sur sulfate de sodium. Après filtration sur fritté et évaporation sous vide partiel, 0,8g d'un solide jaune sont ainsi obtenus. Repris à nouveau dans 50 mL de dichlorométhane puis séché sur sulfate de sodium, le produit est chromatographié sur silice (élution : hexane-acétate d'éthyle 7/3) puis recristallisé dans le toluène pour donner 530mg d'un solide blanc. (Rendement : 34%) caractérisé par RMN sous forme monohydrate (pic à 1,6ppm).

### Analyses :

### Solide blanc

RMN ¹H : (CDCl₃);_7,93 (s, 1H, H pyrazole), 7,52-7,32 (m, 5H, H arom.), 6,35 (m, 1H, NH), 3,68 (q, 2H, CH₂), 2,72 (t, 2H, CH₂), 2,15 (s, 3H, CH₃),
RMN ¹³C : (CDCl₃); 161,7 (CO-NH), 140,0 (CH arom.), 139,6 (C arom.), 130,3 (CH arom.), 129,8 (C), 129,6 (2 CH arom.), 126,2 (2 CH arom.), 121,2 (C), 119 (non visible, CF₃), 38,3 (CH₂), 34,0 (CH₂), 15,2 (CH₃).

### Synthèse du N-(2-hydroxyethyl)-1-phenyl-5-trifluoromethyl-1H-pyrazole-4-carboxamide (composé 6)

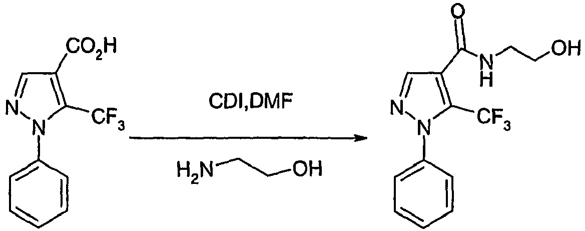

### Réactifs :

| | | | | |
|---|---|---|---|---|
| . 1-phenyl-5-trifluoromethyl -1 H-pyrazole-carboxylic acid | C₁₀H₈N₂O₂ | PM : 188,19 | m = 0,83 g | 4,41 mmol/1 eq. |
| . Carbonyl diimidazole (CDl) | C₇H₆N₄O | PM : 162,15 | m = 0,82 g | 5,05 mmol/1,15 eq. |
| . 2-éthanolamine | C₂H₇NO | PM : 61,08 | m = 1,34 g | 20,5 mmol/4,7 eq. |
| . DMF | | V = 10 mL | | |

### Mode opératoire :

Dans un réacteur sous azote et sous agitation magnétique, le pyrazole est solubilisé dans le DMF. CDI est alors additionné rapidement en une seule portion et le mélange est maintenu sous agitation pendant 20 minutes environ. L'amine est ensuite additionnée à la seringue avec un goutte à goutte rapide. Après 3h d'agitation, le suivi CCM de la réaction indique la disparition de la totalité du produit de départ.

Le milieu réactionnel est ensuite noyé sur 75mL d'un mélange glace/eau. Le précipité blanc formé après 15 minutes d'agitation est alors récupéré par filtration sur fritté et séché par succion. Le solide orangé obtenu est alors repris dans 50mL de dichlorométhane, lavé une fois à l'eau et séché sur sulfate de sodium. Après filtration sur fritté et évaporation sous vide partiel, 1.1 g d'un solide jaune sont ainsi obtenus.

Ce dernier est repris dans un minimum de toluène, mais le produit se révèle partiellement soluble dans le solvant. Le produit est donc recristallisé à froid pour aboutir à la formation de 470mg d'un solide blanc (Rendement : 33%) caractérisé sous forme d'un dérivé associé à 0.5 molécule d'eau.

### Analyses :

### Solide blanc

RMN ¹H : (CDCl₃);_7,93 (s, 1H, H pyrazole), 7,60-7,20 (m, 5H, H arom.), 6,48 (m, 1H, NH), 3,85 (t, 2H, CH₂), 3,63 (t, 2H, CH₂), 2,00 (s large, 2H, OH +H₂O).
RMN ¹³C : (CDCl₃); 162,4 (CO-NH), 140,0 (CH arom.), 139,4 (C arom.), 130,4 (CH arom.), 130,3 (C), 129,6 (2 CH arom.), 126,2 (2 CH arom.), 121,1 (C), 119,8 (q, CF₃).

### SYNTHESE DU COMPOSE 7

### Synthèse du 1-phenyl-1H-pyrazole-carbaldehyde

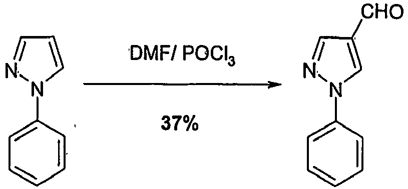

### Réactifs :

| | | | | |
|---|---|---|---|---|
| 1-Phenyl-1 H-pyrazole | C₂H₃N [75-05-8] | PM : 41,05 | V = 39,9 mL | 77 mmol/1 eq. |
| DMF | C₂H₆O [64-17-5] | PM : 46,06 | V = 1,1 L | |
| Oxychlorure de phosphore | [7803-49-8] | PM : 33,03 | V = 100 mL | 1,63 mmol/ 2,13 eq. |

### Mode opératoire : (Réaction de formylation de type Vilsmeier)

Dans un réacteur de 100mL, sous azote et sous agitation magnétique, 10mL de DMF sont chargés et immédiatement refroidis à 0°C à l'aide d'un bain glace/eau. L'oxychlorure de phosphore est additionné à la seringue, goutte à goutte en 12 minutes. Après 1 heure à 0°C, une solution de 1-phenyl-pyrazole (dans 10mL de DMF) est additionnée en 2 minutes à l'aide d'une seringue au cours d'un versement goutte à goutte rapide. Après 5 minutes supplémentaires à 0°C, le mélange est ramené à température ambiante pendant 15 minutes puis placé 2h30 à 100°C. La disparition totale du produit de départ est observée en CCM (Hexane/ Acide acétique (AcOE)t 9/1 ; Rf : 0,35). Une fois revenu à température ambiante, le milieu réactionnel est additionné avec précaution sous hotte sur 20g d'eau glacée.

Après 18 heures d'agitation, le mélange est extrait à deux reprises par 250mL d'acétate d'éthyle. Les phases organiques, jointes sont séchées sur sulfate de sodium, filtrées sur fritté, évaporées à sec puis le résidu est filtré sur un patch de silice sur fritté (élution : Hexane pur, Hexane/CH₂Cl₂ 8/2, Hexane/CH₂Cl₂ 1/1, CH₂Cl₂ 100%) : Les fractions purement chlorées permettent d'isoler 1,17g d'une huile jaune qui cristallise spontanément une fois reprise dans l'hexane. Une seconde fraction (éluée à Hexane/CH₂Cl₂ 1/1) reprise à l'hexane permet également d'isoler un solide. Les solides sont joints et lavés 3 fois par 10mL d'hexane pour obtenir 2,21g d'un solide blanc (Rendement 37%).

### Analyses :

### Solide blanc.

CCM: (dichlorométhane pur): Rf: 0.05 (UV), RMN ¹H, RMN ¹³C.

### Synthèse du 1-phenyl-1H-pyrazole-carboxylicacid

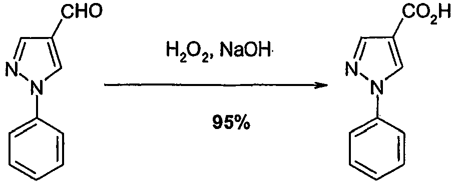

### Réactifs :

| | | | | |
|---|---|---|---|---|
| . 1-phenyl-1H-pyrazole carbaldéhyde - | C₁₀H₈N₂O | PM : 172,18 | m = 2,12 g | 12,31 mmol/1 eq. |
| . H₂O₂ (30% aq.) | H₂O₂ | PM : 34,01 | m = 8,02 g | 70,6 mmol/6,1 eq. |
| . Hydroxide de sodium | HNaO | PM : 40,0 | m = 1,04 g | 26 mmol/2,1 eq. |

### Mode opératoire:

Dans un réacteur tricol de 100mL, sous azote et sous agitation magnétique, la soude est dissoute dans 20mL d'eau. Le pyrazole est alors additionné en une seule fois. Un produit insoluble persistant est observé même à une température de 45-50°C. L'eau oxygénée est additionnée sur la suspension en 6 portions en 50 minutes. Après 5 heures à 50°C, un suivi en CCM permet d'observer la persistance d'une part importante de substrat. 10mL de NaOH 1 N (0,4g de NaOH) et 5g d'eau oxygénée sont additionnés. Après une nouvelle heure d'agitation à 50°C, le produit insoluble a totalement disparu et le suivi en CCM permet d'observer la consommation de l'intégralité du produit de départ (Révélation : DiNitroPhénylHydrazine).

Le milieu réactionnel ramené à température ambiante est alors additionné sur 150mL d'un mélange glace/ HCl 2N (2/1). Le précipité blanc formé est filtré sur Büchner après 30 minutes d'agitation et lavé 3 fois à l'eau. Resolubilisé dans 250mL d'acétate d'éthyle, séché sur MgSO₄ puis filtré et évaporé à sec, 2,25g d'un solide blanc sont ainsi isolés (Rendement : 96%).

### Analyses :

Solide blanc dont la structureobtenue est conforme (RMN ¹H) (RMN).

### Synthèse du N-{2-[(2-furylmethyl)thio]ethyl}-1-phenyl-1H-pyrazole-4-carboxamide

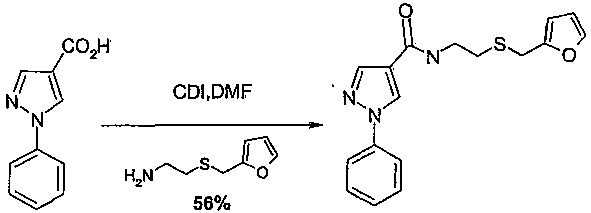

### Réactifs :

| | | | | |
|---|---|---|---|---|
| . 1-phényl-1H-pyrazole- carboxylic acid | C₁₀H₈N₂O₂ | PM : 188,19 | m = 0,83 g | 4,41 mmol/1 eq. |
| . Carbonyl diimidazole (CDI) | C₇H₆N₄O | PM : 162,15 | m = 0,82 g | 5,05 mmol/1,15 eq. |
| . 2-(furfurylthio)-ethylamine | C₇H₁₁NOS | PM : 157,234 | m = 3,23 g | 20,5 mmol/4,7 eq. |
| . DMF | | V=10mL | | |

### Mode opératoire:

Dans un réacteur sous azote et sous agitation magnétique, le pyrazole est solubilisé dans le DMF. CDI est alors additionné rapidement en une seule portion et le mélange est maintenu sous agitation pendant 20 minutes environ. L'amine est ensuite additionnée à la seringue avec un versement goutte à goutte rapide. Après 3h30 d'agitation, le suivi en CCM de la réaction indique la disparition de la totalité du produit de départ.

Le milieu réactionnel est ensuite noyé sur 80mL d'un mélange glace/eau. Le précipité blanc formé après 15 minutes d'agitation est alors récupéré par filtration sur fritté et séché par succion. Le solide orangé obtenu est alors repris dans 50mL de dichlorométhane, lavé une fois à l'eau et séché sur sulfate de sodium. Après filtration sur fritté et évaporation sous vide partiel, 0,8g d'un solide jaune sont ainsi obtenus.

Ce dernier est chromatographié sur silice (élution : hexane-acétate d'éthyle 7/3) puis recristallisé dans le toluène.
0,80g d'un solide beige est ainsi récupéré. (Rendement : 56% ;. Il est caractérisé sous forme d'un composé associé à une demi molécule d'eau.

### Analyses :

### Solide beige

CCM (Hexane/AcOEt 3/7) : Rf = 0,70.
RMN ¹H : (CDCl₃); 8,38 (s, 1H, CH), 7,95 (s, 1H, CH), 7,72 (m, 2H, H arom.), 7,45 (m, 2H, H arom.), 7,36 (m, 2H), 6,31 (m, 3H, 2CH + NH), 3,78 (s, 2H, S-CH₂), 3,60 (q, 2H, N-CH₂), 2,78 (t, 2H, CH₂-S).
RMN ¹³C : (CDCl₃); 162,5 (CO), 151,6 (C), 14,.5 (CH), 139,7 (2 CH), 139,4 (C), 129,8 (CH), 128,6 (CH), 127,6 (CH), 120,3 (C), 119,7 (CH), 110,8 (CH), 108,1 (CH), 38,2 (CH₂), 31,8 (CH₂), 28,2 (CH₂).

### SYNTHESE DU COMPOSE 8

### Synthèse du 4-[4-(ethoxycarbonyl)-5-methyl-1H-pyrazol-1-yl]benzoic acid

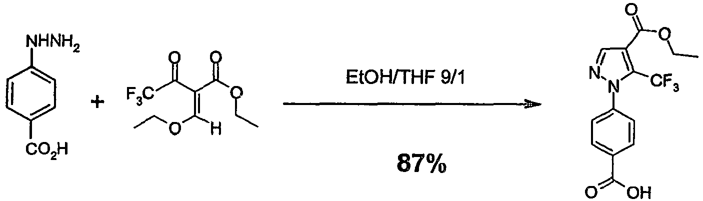

### Réactifs :

### Ethyl 2-(ethoxymethylene-4,4,4-trifluoro-3-oxobutyrate

| | | | | |
|---|---|---|---|---|
| | C₉H₁₁F₃O₄ | PM : 240,18 | m = 6,50 g | 27,07 mmol/1 eq. |
| acide 4-hydrazinobenzoique | C₇H₈N₂O₂ | PM : 152,17 | m = 4,12 g | 27,07 mmol/1 eq. |
| Ethanol | | V = 90 mL | | |
| THF | | V = 10 mL | | |

### Mode opératoire :

Dans un réacteur tricol de 250mL, sous argon et sous agitation magnétique, l'acide hydrazinobenzoïque est placé en suspension dans 90mL d'éthanol absolu. 10mL de THF sont additionnés pour favoriser la solubilisation du réactif (sans succès). La suspension est refroidie à -15°C (bain CCl₄/N₂) et l'oxobutyrate est additionné en 30 minutes, goutte à goutte sur l'hydrazine. Après 2h30 à température ambiante, la solution est devenue totalement limpide ( 1 seul spot visible en CCM avec une révélation caractéristique des pyrazoles en UV à 254nm). Le solvant est alors évaporé et le solide jaune obtenu est lavé deux fois par 20ml de pentane et filtré sur fritté. La poudre jaune obtenue est séchée sous vide et 7,70g d'un solide beige sont ainsi isolés (Rendement : 87%).

### Analyses :

Solide beige dont la structure obtenue est conforme (CCM, RMN ¹H, RMN ¹³C).

### Synthèse du 1-(4-carboxyphenyl)-5-trifluoromethyl-1H-pyrazole-4-carboxylic acid

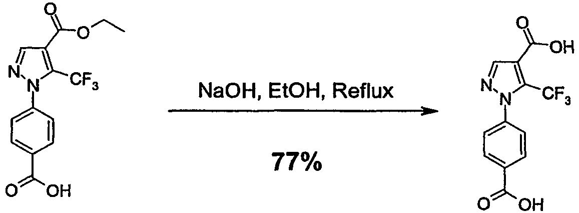

### Réactifs :

### 4-[4-(ethoxycarbonyl)-5-methyl-1 H-pyrazol-1-yl]-benzoic acid

| | | | | |
|---|---|---|---|---|
| | C₁₄H₁₁N₂O₄F₃ | PM : 328,24 | m = 3,25 g | 9,9 mmol/1 eq. |
| Hydroxyde de sodium | HNaO | PM : 39.99 | m = 1,38 g | 150 mmol/15 eq. |
| Ethanol | | | V = 30 mL | |

### Mode opératoire :

Dans un réacteur de 100mL (muni d'un réfrigérant) sous agitation magnétique, le pyrazole est dissout dans l'éthanol. Après 15 minutes à température ambiante, une solution de soude (1,38g dans 50mL d'eau) est additionnée. Le milieu réactionnel est agité à température ambiante pendant 5 minutes puis placé au reflux pendant 13h.

Le mélange est alors ramené à température ambiante et acidifié par une solution d'HCl 3N. Le précipité blanc obtenu est alors filtré sur fritté, rincé à l'eau puis séché à l'évaporateur rotatif puis à la pompe sèche.

2,03g de solide sont ainsi obtenus (Rendement : 77%).

### Analyses :

Solide blanc dont la structure est conforme (RMN ¹H, RMN ¹³C).

### Synthèse du 4-{4-[({2-[(2-furylmethyl)thio]ethyl}amino)carbonyl]-5-trifluoromethyl-1H-pyrazol-1-yl}benzoic acid

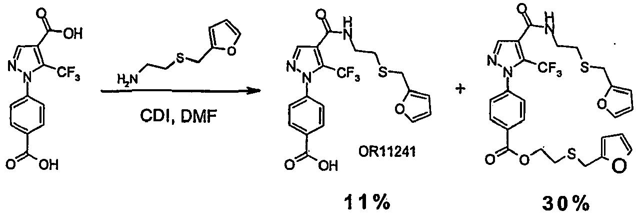

### Réactifs :

| | | | | |
|---|---|---|---|---|
| . 1-(4-carboxyphenyl)-5-trifluoromethyl-1 H-pyrazole-4-carboxylic acid | C₁₂H₇N₂O₂F₃ | PM : 268,19 | m = 1,03 g | 3,04 mmol/1 eq. |
| . Carbonyl diimidazole (CDI) | C₇H₆N₄O | PM : 162,15 | m = 0,81 g | 5,0 mmol/1,3 eq. |
| 2-(furfurylthio)-éthylamine | C₇H₁₁NOS | PM : 157,23 | m = 2,25 g | 14,3 mmol/4,7 eq. |
| DMF | | | V = 12 mL | |

### Mode opératoire:

Dans un réacteur sous azote et sous agitation magnétique, le pyrazole est solubilisé dans le DMF. CDI est alors additionné rapidement en une seule portion et le mélange est maintenu sous agitation pendant 30 minutes environ. L'amine est ensuite additionnée à la seringue avec un versement goutte à goutte rapide. Après une nuit d'agitation, le suivi en CCM de la réaction indique la disparition de la totalité du produit de départ.

Le milieu réactionnel est ensuite noyé sur 100mL d'un mélange glace/eau. Le précipité blanc formé après 15 minutes d'agitation est alors récupéré par filtration sur fritté et séché par succion. Le solide blanc obtenu est alors analysé (CCM, RMN) et deux produits sont ainsi identifiés. Ce mélange brut est alors chromatographié sur gel de silice (chromatographie Flash, élution hexane/ acétate d'éthyle 2/1 puis 1/1 avec 1%d' acide formique).

La première fraction (Rf : 0,65 dans CH₂Cl₂ pur, 1% HCO₂H) contient 140mg du produit attendu (Rendement 11 %). L'identification a été réalisée par RMN par analogie avec les autres composés synthétisés.

La seconde fraction (Rf : 0,48) contient 500mg du produit possédant deux fonctions amide (Rendement 30%).

### Analyses :

Solide blanc dont la structure obtenue est conforme (RMN ¹H: RMN ¹³C).

### EXEMPLE IV: exemples de composés 2-alkylidèneaminooxy-acétamide non appartenant à l'invention

### Composé 1 : N-phenyl-2-({[(1Z)-1-thien-2-ylethylidene}amino}oxy)acetamide

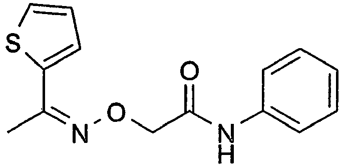

### Composé 2 : 2-({[(1Z)-1-(2-furyl)ethylidene]amino}oxy)-N-phenylacetamide

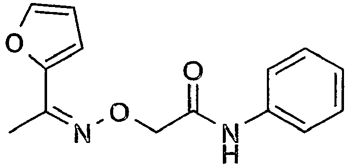

### Composé 3 : N-(4-methoxyphenyl-2-({[(1Z)-1-thien-2-ylethylidene]amino}oxy) acetamide

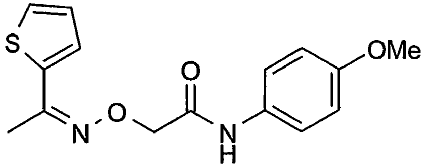

### Composé 4 : N-phenyl-2-({[(1Z)-1-thien-2-ylpropylidene]amino}oxy)acetamide

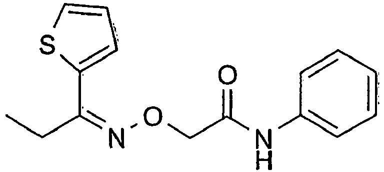

### Composé 5: 2-{[(1-methylethlidene)amino]oxy}-N-phenylacetamide

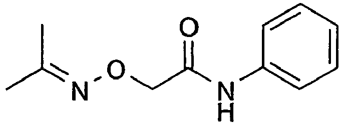

### Composé 6 : N-methyl-2-({[(1Z)-1-thien-2-ylethylidene]amino}oxy)acetamide

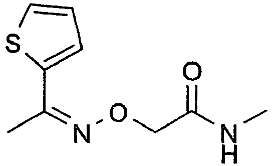

### Composé : 7 N-methyl-N-phenyl-2-({[(1Z)-1-thien-2-ylethylidene]amino}oxy)acetamide

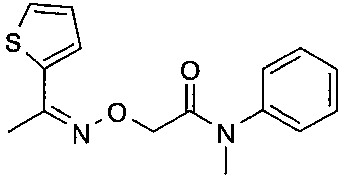

### Composé 8 : N-(2-hydroxyethyl)-N-phenyl-2-({[(1Z)-1-thien-2-ylethylidene]amino}oxy) acetamide

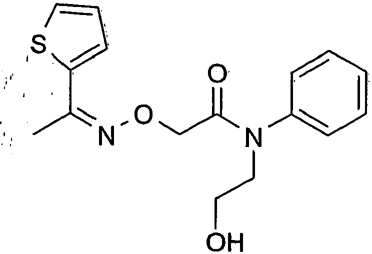

des compositions cosmétiques peuvent être réalisées selon les modalités décrites aus exemples I-7 à I-13 avec les composés des exemples I à IV ou cités dans la demande.

## Revendications

1. Utilisation non thérapeutique d'au moins un inhibiteur de la 15 hydroxy-prostaglandine déshydrogénase (ou; 15-PGDH) pour favoriser la pigmentation de la peau ou des phanères, ledit inhibiteur étant un composé hétérocyclique de formule (I) ou l'un de ses sels: 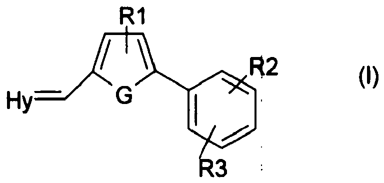 dans laquelle :
- Hy représente un héterocycle à 4, 5, 6 ou 7 atomes comportant éventuellement au moins une fonction carbonyle et/ou une fonction thiocarbonyle, le dit hétérocycle étant éventuellement substitué par au moins un substituant choisi parmi un halogène, les groupes OR, SR, NRR', COR, CSR, NRCONR'R", C(=NR)R', C(=NR)NR'R", NRC(=NR')NR"R"', OCOR, COSR, SCOR, CSNRR', NRCSR', NRCSNR'R", COOR, CONRR', CF₃, CN, NRCOR', SO₂R', SO₂NRR', NRSO₂R', les radicaux alkyle linéaires ou ramifiés, saturés ou insaturés, en C₁-C₂₀, les cycles de 4 à 7 atomes, saturés ou insaturés, contenant éventuellement au moins un hétéroatome, ces cycles pouvant être séparés ou accolés, les radicaux alkyle et les cycles pouvant, en outre, être substitués, où R, R', R" et R"', identiques ou différents, désignent un hydrogène, un radical alkyle en C₁-C₂₀ linéaire ou ramifié ou un radical aryle, éventuellement substitué ;
- G représente O, S, NH ;
- R₁, R₂ et R₃ représentant indépendamment l'un de l'autre un hydrogène, un halogène, un groupe OR₀, SR₀, NR₀R₀', COR₀, CSR₀, NR₀CONR₀'R₀", C(=NR₀)R₀', C(=NR₀)NR₀'R₀", NR₀C(=NR₀')NR₀"R₀'", OCOR₀, COSR₀, SCOR₀, CSNR₀R₀', NR₀CSR₀', NR₀CSNR₀'R₀", COOR₀, CONR₀R₀', CF₃, NO₂, CN, NR₀COR'₀, SO₂R'₀, SO₂NR₀R'₀. NR₀SO₂R'₀, un radical alkyle linéaire ou ramifié, saturé ou insaturé, en C₁-C₂₀, au moins un cycle de 4 à 7 atomes, saturé ou insaturé, contenant éventuellement au moins un hétéroatome, les cycles pouvant être séparés ou accolés, les radicaux alkyle et les cycles pouvant, en outre être substitués, où R₀, R₀', R₀" et R₀"', identiques ou différents, désignent un hydrogène, un radical alkyle en C₁-C₂₀ linéaire ou ramifié ou un radical aryle, éventuellement substitué.

2. Utilisation d'au moins un inhibiteur de la 15 hydroxy-prostaglandine déshydrogénase selon la revendication 1, **caractérisée en ce que** le composé hétérocyclique présente la formule (IV) suivante: 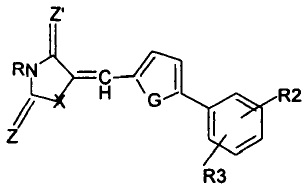 dans laquelle Z, Z' et G représentent indépendamment O ou S ; X représente O, NH ou S ; R représente l'hydrogène ou un radical alkyle linéaire ou ramifié, saturé en C₁-C₁₀ ; l'un au moins des R₂ et R₃ représentent un hydrogène, CN, NO₂, CF₃, un radical phényle, OR₀ ou COOR₀, un radical alkyle linéaire ou ramifié, saturé en C₁-C₂₀ et mieux en C₁-C₁₀, éventuellement substitue par OR₀, avec R₀ valant H ou un radical alkyle, linéaire ou ramifié, saturé en C₁-C₂₀ et mieux en C₁-C₁₀, à condition que lorsque X = S et Z = Z' = G ou Z ≠ Z', alors R₂ et R₃ soient différents de COOH.

3. Utilisation selon la revendication 2, **caractérisée en ce que** lorsque Z = Z' = G, l'un au moins des R₂ et R₃ représente CF₃ ou COOR₀ avec R₀ valant un radical alkyle, linéaire ou ramifié, saturé en C₁-C₁₀ et mieux en C₁-C₅ ; ou lorsque Z = Z' et sont différents de G, l'un au moins des R₂ et R₃ représente CF₃ ou COOR₀, avec R₀ valant H.

4. Utilisation selon l'une au moins des revendications 1 à 3, **caractérisée en ce que** le sel du composé de formule (I) est un sel choisi parmi les sels de sodium, de potassium, les sels de zinc (Zn²⁺), de calcium (Ca²⁺), de cuivre (Cu²⁺), de fer (Fe²⁺), de strontium (Sr²⁺), de magnésium (Mg²⁺), de manganèse (Mn²⁺), d'ammonium, les sels de tri-éthanolamine, mono-éthanolamine, di-éthanolamine, hexadécylamine, N,N,N',N'-tétrakis-(hydroxy-propyl-2) éthylène di-amine, tris-hydroxyméthyl aminométhane, les hydroxydes, les carbonates, les halogénures, les sulfates, les phosphates, les nitrates.

5. Utilisation d'au moins un inhibiteur de 15-PGDH selon l'une des revendications précédentes, **caractérisé en ce que** l'inhibiteur est un inhibiteur spécifique de la 15-PGDH, pour lequel le rapport entre l'activité inhibitrice de 15-PGDH et l'activité inhibitrice de la prostaglandine F synthase est supérieur à 1.

6. Utilisation selon l'une quelconque des revendications 1 à 5 selon laquelle ledit inhibiteur de 15-PGDH est contenu dans une composition.

7. Utilisation d'au moins un inhibiteur de la 15 hydroxy-prostaglandine déshydrogénase selon la revendication 6 **caractérisée en ce que** la composition est destinée à une administration par voie topique.

8. Utilisation selon l'une des revendications 6 à 7, **caractérisée en ce que** la composition est destinée à être appliquée sur la peau, les cheveux et/ou les poils.

9. Utilisation d'au moins un inhibiteur de la 15 hydroxy-prostaglandine déshydrogénase selon l'une des revendications 1 à 8, **caractérisée en ce que** l'inhibiteur de 15 hydroxy-prostaglandine déshydrogénase est encapsulé dans une structure choisie parmi les microsphères, les nanosphères, les oléosomes et les nanocapsules.

10. Utilisation non thérapeutique d'une composition cosmétique contenant au moins un inhibiteur de la 15 hydroxy-prostaglandine déshydrogénase selon l'une quelconque des revendications précédentes, pour le traitement de la canitie.

11. Utilisation d'au moins un inhibiteur de la 15-PGDH selon l'une des revendications 7 à 10, **caractérisée en ce que** l'inhibiteur est un inhibiteur spécifique de la 15-PGDH de type 1.

12. Utilisation selon l'une au moins des revendications 1 à 11 **caractérisée en ce que** la composition contient en outre au moins un agent favorisant la pigmentation des cheveux et/ou des poils différent d'un inhibiteur de la 15 hydroxy-prostaglandine déshydrogénase.

13. Utilisation selon au moins l'une des revendications 1 à 12, **caractérisée en ce que** la composition contient en outre au moins un agent accélérateur de pénétration.

14. Utilisation selon l'une des revendications 1 à 13, **caractérisée en ce que** la composition contient en outre au moins une prostaglandine ou un dérivé de prostaglandine.

15. Utilisation selon la revendication 14, **caractérisée en ce que** la composition contient en outre au moins un composé choisi parmi la prostaglandine PGE1, PGE2, leurs sels, leurs esters, leurs analogues et leurs dérivés, les agonistes du récepteur de la prostaglandine F2 alpha (FP-R) notamment le latanoprost, le fluprostenol, le cloprostenol, le travoprost , le bimatoprost, les agonistes des récepteurs de la prostaglandine E2 (EP1-R, EP2-R, EP3-R, EP4-R) tel le 17 phényle PGE2, le viprostol, le butaprost, le misoprostol, le sulprostone, le 16,16 diméthyle PGE2, 11 désoxy-PGE1, le 1 désoxy PGE1, les agonistes et leurs esters du récepteur de la prostacycline (IP) tel le cicaprost, l'iloprost, l'isopcarbacycline, le beraprost, les agonistes et leurs esters du récepteur de la prostaglandine D2 telle BW245C ((4S)-(3-[(3R,S)-3-cyclohexyl-3-isopropyl]-2,5-dioxo)4-imidazolidineheptanoic acid), le ((4R)-(3-[(3R,S)-3-cyclohexyl-3-isopropyl]-2,5-dioxo)4-imidazolidineheptanoic acid), les agonistes du récepteur aux thromboxanes A2 (TP) tel le I-BOP ([1S-[1a,2a(Z), 3b(1E,3S),4a]]-7-[3-[3-hydroxy-4-[4-(iodophenoxy)-1-butenyl]-7-oxabicyclo[2.2.1]hept-2-yl]-5-heptenoic acid, les précurseurs de ces composés, leurs esters et leurs dérivés.

16. Utilisation selon l'une des revendications 1 à 15, **caractérisée en ce que** la composition comprend un milieu cosmétologiquement acceptable constitué d'eau ou d'un mélange d'eau et d'au moins un solvant choisi parmi les solvants organiques hydrophiles, les solvants organiques lipophiles, les solvants organiques amphiphiles et leurs mélanges.

17. Utilisation selon l'une des revendications 1 à 16, **caractérisée en ce que** l'inhibiteur de 15 hydroxy-prostaglandine déshydrogénase est présent dans la composition à une concentration comprise entre 0,001% et 5% p/v.

18. Utilisation selon l'une au moins des revendications 1 à 17, **caractérisée en ce que** le composé est choisi parmi:

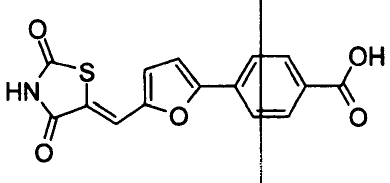

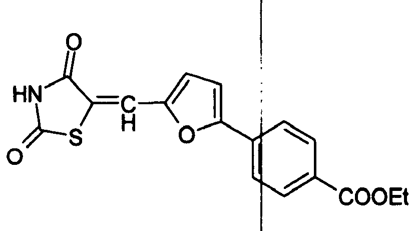

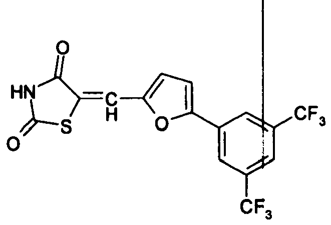

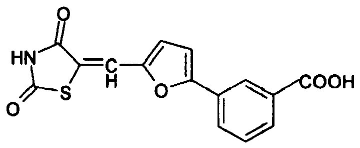

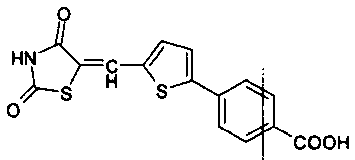

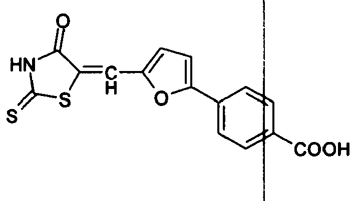

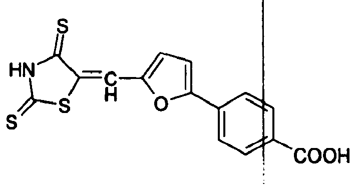

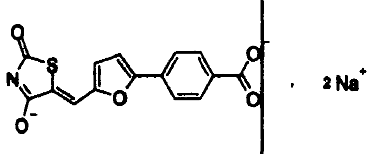

## Patentansprüche

1. Nicht-therapeutische Verwendung mindestens eines Inhibitors der 15-Hydroxyprostaglandin-Dehydrogenase (oder 15-PGDH) zum Fördern der Pigmentierung der Haut oder von Hautanhangsgebilden, wobei der Inhibitor eine heterozyklische Verbindung der Formel (I) oder eines von deren Salzen ist: 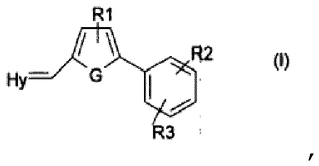 worin:
- Hy für einen Heterozyklus mit 4, 5, 6 oder 7 Atomen steht, die gegebenenfalls mindestens eine Carbonylfunktion und/oder eine Thiocarbonylfunktion tragen, wobei der Heterozyklus gegebenenfalls mit mindestens einem Substituenten substituiert ist, der ausgewählt wird aus Halogen, den Gruppen OR, SR, NRR', COR, CSR, NRCONR'R'', C(=NR)R', C(=NR)NR'R", NRC(=NR')NR''R''', OCOR, COSR, SCOR, CSNRR', NRCSR', NRCSNR'R'', COOR, CONRR', CF₃, CN, NRCOR', SO₂R', SO₂NRR', NRSO₂R', geraden oder verzweigten, gesättigten oder ungesättigten C₁-C₂₀-Alkylresten, gesättigten oder ungesättigten Ringen mit 4 bis 7 Atomen, die gegebenenfalls mindestens ein Heteroatom enthalten, wobei diese Ringe getrennt oder aneinander gefügt sein können, wobei die Alkylreste und die Ringe außerdem substituiert sein können, wobei R, R', R'' und R''', die gleich oder verschieden sind, für ein Wasserstoffatom, einen geraden oder verzweigten C₁-C₂₀-Alkylrest oder einen Arylrest stehen, die gegebenenfalls substituiert sind,
- G für O, S, NH steht,
- R₁, R₂ und R₃ unabhängig voneinander für ein Wasserstoff-, ein Halogenatom, OR₀, SR₀, NR₀R₀', COR₀, CSR₀, NR₀CONR₀'R₀'', C(=NR₀)R₀', C (=NR₀) NR₀'R₀'', NR₀C (=NR₀') NR₀''R₀''', OCOR₀, COSR₀, SCOR₀, CSNR₀R₀', NR₀CSR₀', NR₀CSNR₀'R₀'', COOR₀, CONR₀R₀', CF₃, NO₂, CN, NR₀COR'₀, SO₂R'₀, SO₂NR₀R'₀, NR₀SO₂R'₀, einen geraden oder verzweigten, gesättigten oder ungesättigten C₁-C₂₀-Alkylrest, mindestens einen gesättigten oder ungesättigten Ring von 4 bis 7 Atomen, der gegebenenfalls mindestens ein Heteroatom enthält, stehen, wobei die Ringe getrennt oder aneinander gefügt sein können, wobei die Alkylreste und die Ringe außerdem substituiert sein können, wobei R₀, R₀', R₀'' und R₀''', die gleich oder verschieden sind, für ein Wasserstoffatom, einen geraden oder verzweigten C₁-C₂₀-Alkylrest oder einen Arylrest stehen, die gegebenenfalls substituiert sind.

2. Verwendung mindestens eines Inhibitors der 15-Hydroxyprostaglandin-Dehydrogenase nach Anspruch 1, **dadurch gekennzeichnet, dass** die heterozyklische Verbindung die folgende Formel (IV) aufweist: 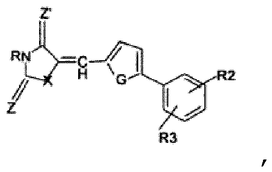 worin Z, Z' und G unabhängig für O oder S stehen, X für O, NH oder S steht, R für Wasserstoff oder einen geraden oder verzweigten gesättigten C₁-C₁₀-Alkylrest steht, mindestens einer der Reste R₂ und R₃ für ein Wasserstoffatom, CN, NO₂, CF₃, einen Phenylrest, OR₀ oder COOR₀, einen geraden oder verzweigten gesättigten C₁-C₂₀- und besser C₁-C₁₀-Alkylrest steht, der gegebenenfalls mit OR₀ substituiert ist, wobei R₀ für H oder einen geraden oder verzweigten gesättigten C₁-C₂₀- und besser C₁-C₁₀-Alkylrest steht, mit der Maßgabe, dass, wenn X = S und Z = Z' = G oder Z ≠ Z', R₂ und R₃ dann nicht COOH sind.

3. Verwendung nach Anspruch 2, **dadurch gekennzeichnet, dass**, wenn Z = Z' = G, mindestens einer der Reste R₂ und R₃ für CF₃ oder COOR₀ steht, wobei R₀ für einen geraden oder verzweigten gesättigten C₁-C₁₀- und besser C₁-C₅-Alkylrest steht, oder wenn Z = Z' und sie verschieden von G sind, mindestens einer der Reste R₂ und R₃ für CF₃ oder COOR₀ steht, wobei R₀ für H steht.

4. Verwendung nach mindestens einem der Ansprüche 1 bis 3, **dadurch gekennzeichnet, dass** das Salz der Verbindung der Formel (I) ein Salz ist, das aus den Natrium-, Kaliumsalzen, den Zink- (Zn²⁺-), Calcium-(Ca²⁺-), Kupfer- (Cu²⁺-), Eisen- (Fe²⁺-), Strontium-(Sr²⁺-), Magnesium- (Mg2⁺-), Mangan- (Mn²⁺-), Ammoniumsalzen, den Triethanolamin-, Monoethanolamin-, Diethanolamin-, Hexadecylamin-, N,N,N',N'-Tetrakis(hydroxypropyl-2)ethylendiamin-, Trishydroxymethylaminomethansalzen, den Hydroxiden, den Carbonaten, den Halogeniden, den Sulfaten, den Phosphaten, den Nitraten ausgewählt wird.

5. Verwendung mindestens eines Inhibitors der 15-PGDH nach einem der vorhergehenden Ansprüche, **dadurch gekennzeichnet, dass** der Inhibitor ein spezifischer Inhibitor der 15-PGDH ist, für den das Verhältnis der inhibitorischen Aktivität gegenüber 15-PGDH und der inhibitorischen Aktivität gegenüber Prostaglandin F-Synthase größer als 1 ist.

6. Verwendung nach einem der Ansprüche 1 bis 5, wobei der Inhibitor der 15-PGDH in einer Zusammensetzung enthalten ist.

7. Verwendung mindestens eines Inhibitors der 15-Hydroxyprostaglandin-Dehydrogenase nach Anspruch 6, **dadurch gekennzeichnet, dass** die Zusammensetzung für eine Verabreichung auf topischem Weg bestimmt ist.

8. Verwendung nach einem der Ansprüche 6 bis 7, **dadurch gekennzeichnet, dass** die Zusammensetzung dafür bestimmt ist, auf die Haut, die Haare und/oder die Körperbehaarung aufgebracht zu werden.

9. Verwendung mindestens eines Inhibitors der 15-Hydroxyprostaglandin-Dehydrogenase nach einem der Ansprüche 1 bis 8, **dadurch gekennzeichnet, dass** der Inhibitor der 15-Hydroxyprostaglandin-Dehydrogenase in einer Struktur gekapselt ist, die aus Mikrosphären, Nanosphären, Oleosomen und Nanokapseln ausgewählt wird.

10. Nicht-therapeutische Verwendung einer kosmetischen Zusammensetzung, die mindestens einen Inhibitor der 15-Hydroxyprostaglandin-Dehydrogenase nach einem der vorhergehenden Ansprüche enthält, für die Behandlung von Canities.

11. Verwendung mindestens eines Inhibitors der 15-PGDH nach einem der Ansprüche 7 bis 10, **dadurch gekennzeichnet, dass** es sich bei dem Inhibitor um einen spezifischen Inhibitor der 15-PGDH Typ 1 handelt.

12. Verwendung nach mindestens einem der Ansprüche 1 bis 11, **dadurch gekennzeichnet, dass** die Zusammensetzung außerdem mindestens ein anderes Mittel, das die Pigmentierung der Haare und/oder der Körperbehaarung fördert, als den Inhibitor der 15-Hydroxyprostaglandin-Dehydrogenase enthält.

13. Verwendung nach mindestens einem der Ansprüche 1 bis 12, **dadurch gekennzeichnet, dass** die Zusammensetzung außerdem mindestens einen Eindringbeschleuniger enthält.

14. Verwendung nach einem der Ansprüche 1 bis 13, **dadurch gekennzeichnet, dass** die Zusammensetzung außerdem mindestens ein Prostaglandin oder ein Prostaglandinderivat enthält.

15. Verwendung nach Anspruch 14, **dadurch gekennzeichnet, dass** die Zusammensetzung außerdem mindestens eine Verbindung enthält, die aus Prostaglandin PGE1, PGE2, deren Salzen, deren Estern, deren Analoga und deren Derivaten, den Agonisten des Prostaglandin F2 alpha-Rezeptors (FP-R), insbesondere Latanoprost, Fluprostenol, Cloprostenol, Travoprost, Bimatoprost, den Agonisten des Prostaglandin E2-Rezeptors (EP1-R, EP2-R, EP3-R, EP4-R), wie 17-Phenyl-PGE2, Viprostol, Butaprost, Misoprostol, Sulproston, 16,16-Dimethyl-PGE2, 11-Desoxy-PGE1, 1-Desoxy-PGE1, den Agonisten und deren Estern des Prostacyclin-Rezeptors (IP), wie Cicaprost, Iloprost, Isopcarbacyclin, Beraprost, den Agonisten und deren Estern des Prostaglandin D2-Rezeptors, wie BW245C ((4S)-(3-[(3R,S)-3-Cyclohexyl-3-isopropyl]-2,5-dioxo)-4-imidazolidinheptansäure), ((4R)-(3-[(3R,S)-3-Cyclohexyl-3-isopropyl]-2,5-dioxo)-4-imidazolidinheptansäure), den Agonisten des Thromboxan A2-Rezeptors (TP), wie I-BOP ([1S-[1a,2a(Z),3b(1E,3S),4a]]-7-[3-[3-Hydroxy-4-[4-(iodphenoxy)-1-butenyl]-7-oxabicyclo[2.2.1]hept-2-yl]-5-heptensäure, den Vorläufern dieser Verbindungen, deren Estern und deren Derivaten ausgewählt wird.

16. Verwendung nach einem der Ansprüche 1 bis 15, **dadurch gekennzeichnet, dass** die Zusammensetzung ein kosmetologisch annehmbares Medium umfasst, das aus Wasser oder einem Gemisch von Wasser und mindestens einem Lösungsmittel besteht, das aus organischen hydrophilen Lösungsmitteln, organischen lipophilen Lösungsmitteln, organischen amphiphilen Lösungsmitteln und deren Gemischen ausgewählt ist.

17. Verwendung nach einem der Ansprüche 1 bis 16, **dadurch gekennzeichnet, dass** der Inhibitor der 15-Hydroxyprostaglandin-Dehydrogenase in der Zusammensetzung in einer Konzentration zwischen 0,001 % und 5% w/v vorhanden ist.

18. Verwendung nach mindestens einem der Ansprüche 1 bis 17, **dadurch gekennzeichnet, dass** die Verbindung aus Folgenden ausgewählt wird:

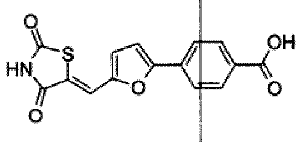

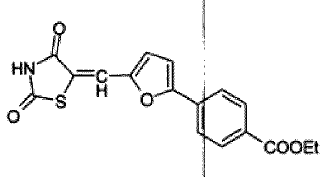

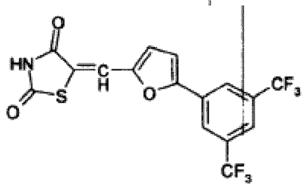

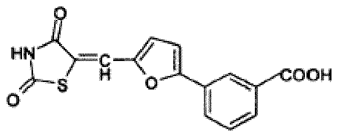

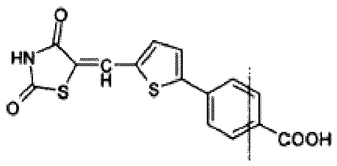

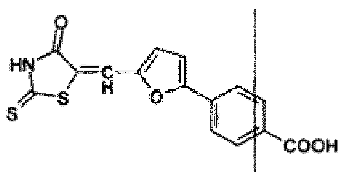

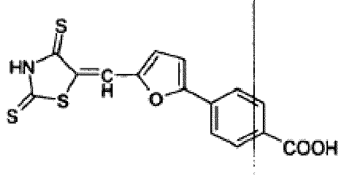

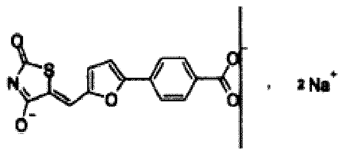

## Claims

1. Non-therapeutic use of at least one 15-hydroxyprostaglandin dehydrogenase (or 15-PGDH) inhibitor for promoting pigmentation of the skin or of the skin appendages, said inhibitor being a heterocyclic compound of formula (I) or a salt thereof: 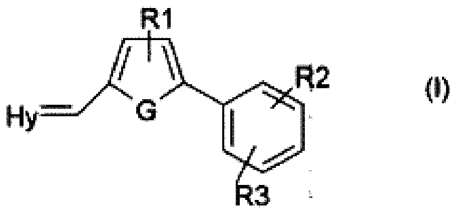 in which:
- Hy represents a heterocycle with 4, 5, 6 or 7 atoms optionally comprising at least one carbonyl function and/or one thiocarbonyl function, said heterocycle being optionally substituted with at least one substituent chosen from a halogen, the groups OR, SR, NRR', COR, CSR, NRCONR'R'', C(=NR)R', C(=NR)NR'R'', NRC(=NR')NR''R''', OCOR, COSR, SCOR, CSNRR', NRCSR', NRCSNR'R'', COOR, CONRR', CF₃, CN, NRCOR', SO₂R', SO₂NRR' and NRSO₂R', saturated or unsaturated, linear or branched C₁-C₂₀ alkyl radicals, and saturated or unsaturated rings with from 4 to 7 atoms, optionally containing at least one heteroatom, it being possible for these rings to be separate or fused, it being possible for the alkyl radicals and the rings to also be substituted, where R, R', R'' and R''', which may be identical or different, denote a hydrogen, a linear or branched C₁-C₂₀ alkyl radical or an aryl radical, which is optionally substituted;
- G represents O, S or NH;
- R₁, R₂ and R₃ represent, independently of one another, a hydrogen, a halogen, an OR₀, SR₀, NR₀R₀', COR₀, CSR₀, NR₀CONR₀' R₀'', C (=NR₀) R₀', C (=NR₀) NR₀' R₀'', NR₀C(=NR₀')NR₀''NR₀''', OCOR₀, COSR₀, SCOR₀, CSNR₀R₀', NR₀CSR₀', NR₀CSNR₀'R₀'', COOR₀, CONR₀R₀', CF₃, NO₂, CN, NR₀COR'₀, SO₂R'₀, SO₂NR₀R'₀ or NR₀SO₂R'₀ group, a saturated or unsaturated, linear or branched C₁-C₂₀ alkyl radical, at least one saturated or unsaturated ring with from 4 to 7 atoms, optionally containing at least one heteroatom, it being possible for the rings to be separate or fused, it being possible for the alkyl radicals and the rings to also be substituted, where R₀, R₀', R₀'' and R₀''', which may be identical or different, denote a hydrogen, a linear or branched C₁-C₂₀ alkyl radical or an aryl radical, which is optionally substituted.

2. Use of at least one 15-hydroxyprostaglandin dehydrogenase inhibitor according to Claim 1, **characterized in that** the heterocyclic compound is of formula (IV) below: 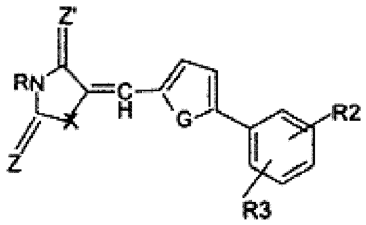 in which Z, Z' and G independently represent O or S; X represents O, NH or S; R represents hydrogen or a saturated linear or branched C₁-C₁₀ alkyl radical; at least one of R₂ and R₃ represents a hydrogen, CN, NO₂, CF₃, a phenyl radical, OR₀ or COOR₀, a saturated linear or branched C₁-C₂₀, and better still C₁-C₁₀, alkyl radical optionally substituted with OR₀, with R₀ being H or a saturated linear or branched C₁-C₂₀, and better still C₁-C₁₀, alkyl radical, on the condition that, when X = S and Z = Z' = G or Z ≠ Z', then R₂ and R₃ are other than COOH.

3. Use according to Claim 2, **characterized in that**, when Z = Z' = G, at least one of R₂ and R₃ represents CF₃ or COOR₀ with R₀ being a saturated linear or branched C₁-C₁₀, and better still C₁-C₅, alkyl radical; or when Z = Z' and are other than G, at least one of R₂ and R₃ represents CF₃ or COOR₀, with R₀ being H.

4. Use according to at least one of Claims 1 to 3, **characterized in that** the salt of the compound of formula (I) is chosen from the sodium or potassium salts, the zinc (Zn²⁺), calcium (Ca²⁺), copper (Cu²⁺), iron (Fe²⁺), strontium (Sr²⁺), magnesium (Mg²⁺), manganese (Mn²⁺) or ammonium salts, the triethanolamine, monoethanolamine, diethanolamine, hexadecylamine, N,N,N',N'-tetrakis(2-hydroxypropyl)ethylenediamine or trishydroxymethylaminomethane salts, the hydroxides, the carbonates, the halides, the sulfates, the phosphates and the nitrates.

5. Use of at least one 15-PGDH inhibitor according to one of the preceding claims, **characterized in that** the inhibitor is a 15-PGDH-specific inhibitor for which the ratio between the 15-PGDH-inhibiting activity and the prostaglandin-F-synthase-inhibiting activity is greater than 1.

6. Use according to any one of Claims 1 to 5, according to which said 15-PGDH inhibitor is contained in a composition.

7. Use of at least one 15-hydroxyprostaglandin dehydrogenase inhibitor according to Claim 6, **characterized in that** the composition is intended for topical administration.

8. Use according to either of Claims 6 and 7, **characterized in that** the composition is intended to be applied to the skin, the hair and/or body hair.

9. Use of at least one 15-hydroxyprostaglandin dehydrogenase inhibitor according to one of Claims 1 to 8, **characterized in that** the 15-hydroxyprostaglandin dehydrogenase inhibitor is encapsulated in a structure chosen from microspheres, nanospheres, oleosomes and nanocapsules.

10. Non-therapeutic use of a cosmetic composition containing at least one 15-hydroxyprostaglandin dehydrogenase inhibitor according to any one of the preceding claims, for treating canities.

11. Use of at least one 15-PGDH inhibitor according to one of Claims 7 to 10, **characterized in that** the inhibitor is an inhibitor specific for 15-PGDH type 1.

12. Use according to at least one of Claims 1 to 11, **characterized in that** the composition also contains at least one agent for promoting the pigmentation of the hair and/or of body hair other than a 15-hydroxyprostaglandin dehydrogenase inhibitor.

13. Use according to at least one of Claims 1 to 12, **characterized in that** the composition also contains at least one penetration-accelerating agent.

14. Use according to one of Claims 1 to 13, **characterized in that** the composition also contains at least one prostaglandin or one prostaglandin derivative.

15. Use according to Claim 14, **characterized in that** the composition also contains at least one compound chosen from prostaglandin PGE1, PGE2, salts thereof, esters thereof, analogues thereof and derivatives thereof, prostaglandin F2 alpha receptor (FP-R) agonists, in particular latanoprost, fluprostenol, cloprostenol, travoprost and bimatoprost, prostaglandin E2 receptor (EP1-R, EP2-R, EP3-R, EP4-R) agonists, such as 17-phenyl-PGE2, viprostol, butaprost, misoprostol, sulprostone, 16,16-dimethyl-PGE2, 11-deoxy-PGE1 or 1-deoxy-PGE1, prostacyclin (IP) receptor agonists and esters thereof, such as cicaprost, iloprost, isocarbacycline or beraprost, prostaglandin D2 receptor agonists and esters thereof, such as BW245C ((4S)-(3-[(3R,S)-3-cyclohexyl-3-isopropyl]-2,5-dioxo)-4-imidazolidineheptanoic acid) or (4R)-(3-[(3R,S)-3-cyclohexyl-3-isopropyl]-2,5-dioxo)-4-imidazolidineheptanoic acid), thromboxane A2 (TP) receptor agonists, such as I-BOP ([1S-[1a,2a(2),3b(1E,3S),4a]]-7-[3-[3-hydroxy-4-(4-iodophenoxy)-1-butenyl]-7-oxabicyclo-[2.2.1]hept-2-yl]-5-heptenoic acid), the precursors of these compounds, the esters thereof and the derivatives thereof.

16. Use according to one of Claims 1 to 15, **characterized in that** the composition comprises a cosmetically acceptable medium constituted of water or a mixture of water and at least one solvent chosen from hydrophilic organic solvents, lipophilic organic solvents, amphiphilic organic solvents and mixtures thereof.

17. Use according to one of Claims 1 to 16, **characterized in that** the 15-hydroxyprostaglandin dehydrogenase inhibitor is present in the composition at a concentration of between 0.001% and 5% w/v.

18. Use according to at least one of Claims 1 to 17, **characterized in that** the compound is chosen from:
